(19) Europäisches Patentamt
European Patent Office
Office européen des brevets

(11) EP 4 032 969 A1

(12) EUROPEAN PATENT APPLICATION
published in accordance with Art. 153(4) EPC

(43) Date of publication:
27.07.2022 Bulletin 2022/30

(21) Application number: 20865544.9

(22) Date of filing: 16.09.2020

(51) International Patent Classification (IPC):
*C12M 1/40* (2006.01) *C12N 15/52* (2006.01)
*C12Q 1/26* (2006.01) *C12N 9/02* (2006.01)

(52) Cooperative Patent Classification (CPC):
C12M 1/40; C12N 15/52; C12Q 1/26

(86) International application number:
PCT/JP2020/035137

(87) International publication number:
WO 2021/054375 (25.03.2021 Gazette 2021/12)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 17.09.2019 JP 2019168805

(71) Applicant: Kikkoman Corporation
Noda-shi, Chiba 278-8601 (JP)

(72) Inventors:
- YAMAMOTO, Kei
Noda-shi, Chiba 278-8601 (JP)
- MASAKARI, Yosuke
Noda-shi, Chiba 278-8601 (JP)
- ARAKI, Yasuko
Noda-shi, Chiba 278-8601 (JP)
- ICHIYANAGI, Atsushi
Noda-shi, Chiba 278-8601 (JP)

(74) Representative: dompatent von Kreisler Selting Werner -
Partnerschaft von Patent- und Rechtsanwälten mbB
Deichmannhaus am Dom
Bahnhofsvorplatz 1
50667 Köln (DE)

# (54) METHOD FOR QUANTIFYING CITRULLINE, OXIDOREDUCTASE FOR QUANTIFICATION, COMPOSITION FOR QUANTIFICATION, KIT FOR QUANTIFICATION, AND METHOD FOR EVALUATING ACTIVITY OF PEPTIDYLARGININE DEIMINASE

(57) A new quantification method for measuring citrulline, which has an association with various diseases and is a biomarker particularly useful for early diagnosis of rheumatoid arthritis, an enzyme for quantification, a composition for quantification, and a kit for quantification are provided. A quantification method of citrulline is provided by adding a citrulline oxidoreductase to a sample. The oxidoreductase is an oxidase, and a concentration of the citrulline may be determined by quantifying hydrogen peroxide produced by addition of the oxidase. A concentration of the citrulline may be determined by reacting a reagent with hydrogen peroxide produced by addition of the oxidase.

EP 4 032 969 A1

# FIG. 3

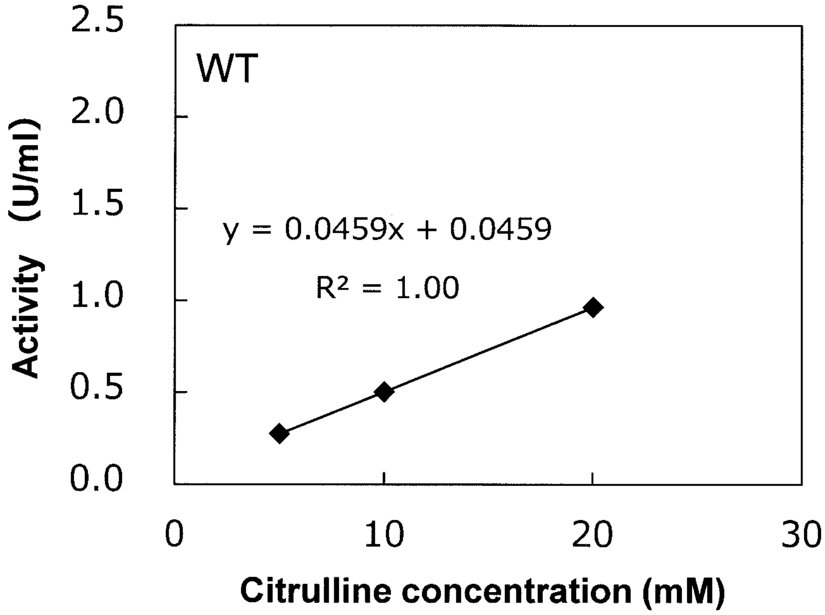
WT
y = 0.0459x + 0.0459
R² = 1.00
Activity (U/ml)
2.5
2.0
1.5
1.0
0.5
0.0
0
10
20
30
Citrulline concentration (mM)

(a)

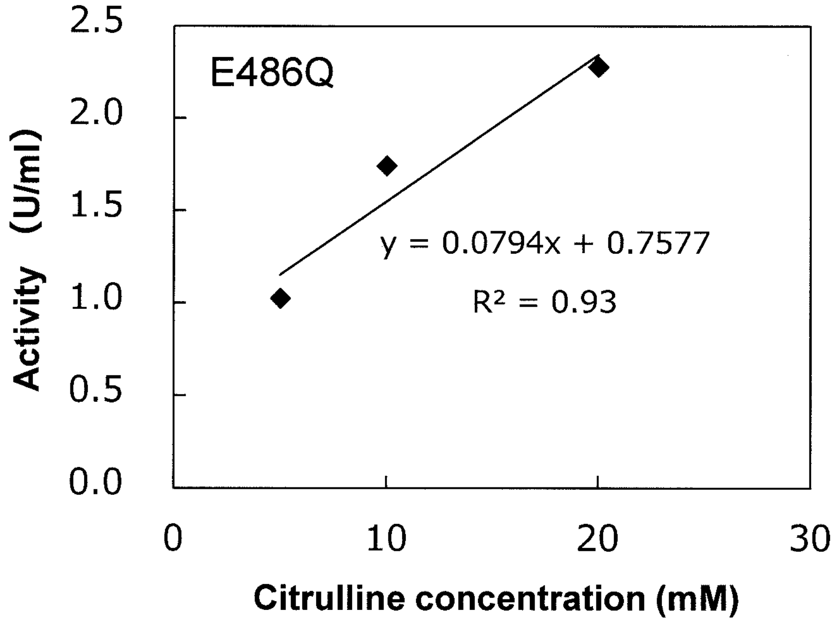
E486Q
y = 0.0794x + 0.7577
R² = 0.93
Activity (U/ml)
2.5
2.0
1.5
1.0
0.5
0.0
0
10
20
30
Citrulline concentration (mM)

(b)

## Description

### TECHNICAL FIELD

The present invention relates to a method for quantifying citrulline, oxidoreductase for quantification, a composition for quantification, a kit for quantification, and a method for evaluating the activity of peptidylarginine deiminase.

### BACKGROUND

It is known that citrulline is not a codon-specified amino acid, and a pathway to be produced by converting arginine residues in a protein into citrulline by peptidylarginine deiminase (PAD) or a pathway produced by converting ornithine into citrulline by ornithine transcarbamoylase in the ornithine cycle are known. Aberrant citrullination of proteins in a living body has been reported to be associated with diseases such as multiple sclerosis, Alzheimer's disease, chronic rheumatoid arthritis, psoriasis, prion disease, liver fibrosis, chronic obstructive pulmonary disease, and cancer.

For example, rheumatoid arthritis is considered to be an autoimmune diseases and causes chronic inflammation in joints, and as it progresses, joints are destroyed, and various dysfunctions occur. Therefore, early detection and early treatment of rheumatoid arthritis are important. However, a rheumatoid factor (RF) which is a traditional test item may show false-positive results even in healthy individuals.

An anti-cyclic citrullinated peptide antibody (anti-CCP antibody) is positive earlier than RF in patients with rheumatoid arthritis and is more specific than RF, making it a useful indicator for early diagnosis of rheumatoid arthritis. However, it was pointed out that in the case of early rheumatoid arthritis, about 30% of false negatives occurred in the test with the anti-CCP antibody, resulting in a delayed definitive diagnosis. In addition, since the test method uses an immunological measurement method, there is a problem that the time required for the measurement is long, and the test cost is high.

Therefore, a method of measuring a citrullinated peptide itself rather than measuring the anti-CCP antibody is desired. Specifically, a method of decomposing a citrullinated peptide with a protease or the like and measuring the liberated citrulline can be considered. For the measurement of citrulline, for example, the use of a measurement system by an enzymatic method is conceivable. For example, Patent Literature 1 describes a method in which citrulline is subjected to argininosuccinate synthetase to produce pyrophosphate, pyrophosphate pyruvate dikinase is applied to the obtained pyrophosphate to produce pyruvic acid, and citrulline is quantified based on the amount of the obtained pyruvic acid.

In addition, five types of PADs from PAD1 to PAD4 and PAD6 are known, and it is considered that PAD2 may be involved in the onset of Alzheimer's disease, and PAD4 may be involved in the onset of rheumatism (non-Patent Literature 1). From the viewpoint of treating these diseases, not only a quantification method of a citrullinated peptide but also an activity evaluation method of PAD which converts an arginine peptide into a citrullinated peptide is desired.

### CITATION LIST

### PATENT LITERATURE

Patent Literature 1: Japanese Patent No. 5303715

### NON-PATENT LITERATURE

Akihito Ishigami, "Citrullinated Molecule and Geriatric Disease", Journal of the Japan geriatrics society, The Japan Geriatrics Society, July 2014, Vol. 51, No. 4, p. 314-320

### SUMMARY OF INVENTION

### TECHNICAL PROBLEM

However, in the method disclosed in Patent Literature 1, a three-step enzymatic reaction in which argininosuccinate synthase, pyrophosphate pyruvate dikinase, and pyruvate oxidase for quantifying pyruvate are acted is necessary, and the operation becomes complicated. The complicated operation causes an error in the measured value, and the inspection accuracy is lowered. Therefore, a method for conveniently quantifying citrulline by directly oxidizing or reducing citrulline is required.

It is an object of the present invention to provide a new quantification method for measuring citrulline, which has an association with various diseases and is a biomarker particularly useful for early diagnosis of rheumatoid arthritis, an enzyme for quantification, a composition for quantification, and a kit for quantification. Another object of the present invention is to provide an activity evaluation method of PAD using a measurement method of citrulline.

## SOLUTION TO PROBLEM

According to one embodiment of the present invention, a quantification method of citrulline is provided including adding a citrulline oxidoreductase to a sample.

The citrulline oxidoreductase is a citrulline oxidase, and a concentration of the citrulline may be determined by quantifying hydrogen peroxide produced by addition of the citrulline oxidase.

The citrulline oxidoreductase is a citrulline oxidase, and a concentration of the citrulline may be determined by reacting a reagent with hydrogen peroxide produced by addition of the citrulline oxidase.

The citrulline oxidoreductase is a citrulline dehydrogenase, and a concentration of the citrulline may be determined by reducing a mediator by addition of the citrulline dehydrogenase.

According to one embodiment of the present invention, a composition for quantification of citrulline is provided including the citrulline oxidoreductase used in the quantification method of citrulline.

The citrulline oxidoreductase is a citrulline oxidase and a reagent reacting with hydrogen peroxide produced by addition of the citrulline oxidase may be included.

According to one embodiment of the present invention, a kit for quantification of citrulline is provided including the citrulline oxidoreductase and a reagent reacting with hydrogen peroxide.

According to one embodiment of the present invention, a composition for quantification of citrulline is provided including a mediator reduced by addition of the citrulline oxidoreductase.

According to one embodiment of the present invention, a kit for quantification of citrulline is provided including a citrulline oxidoreductase and a mediator reduced by addition of the citrulline oxidoreductase.

According to one embodiment of the present invention, a sensor chip is provided including the citrulline oxidoreductase used in the quantification method of citrulline described above.

According to one embodiment of the present invention, a sensor is provided including the sensor chip described above.

According to one embodiment of the present invention, an activity evaluation method of peptidylarginine deiminase including using any of the above quantification methods of citrulline.

According to one embodiment of the present invention, an activity evaluation method of peptidylarginine deiminase is provided including adding a peptide capable of being a substrate of the peptidylarginine deiminase to a sample, adding a protease or peptidase to the sample, and adding a citrulline oxidoreductase to the sample.

The peptidylarginine deiminase may be PAD2 or PAD4.

## ADVANTAGEOUS EFFECTS OF INVENTION

According to the present invention, there is provided a new quantification method for measuring citrulline, which has an association with various diseases and is a biomarker particularly useful for early diagnosis of rheumatoid arthritis, an enzyme for quantification, a composition for quantification, and a kit for quantification. Alternatively, there is provided an activity evaluation method of PAD using a measurement method of citrulline.

## DESCRIPTION OF DRAWINGS

[Fig. 1] (a) is a schematic diagram of a sensor 100 according to one embodiment of the present invention, and (b) is a block diagram of the sensor 100 according to one embodiment of the present invention.

[Fig. 2] (a) is a schematic diagram of a sensor chip 10 according to one embodiment of the present invention, and (b) to (d) are schematic diagrams showing members constituting the sensor chip 10.

[Fig.3] (a) is a diagram showing a correlation of a citrulline concentration (mM) and enzyme activity (U/ml) of a citrulline oxidase (WT) treated with a buffer solution of pH 6.0 according to the Examples of the present invention, and (b) is a diagram showing a correlation of a citrulline concentration (mM) and enzyme activity (U/ml) of a citrulline oxidase mutant E486Q treated with a buffer solution of pH 6.0 according to the Examples of the present invention.

[Fig.4] (a) is a diagram showing a correlation of a citrulline concentration (mM) and enzyme activity (U/ml) measured using a dehydrogenase reaction of a citrulline oxidase (WT) treated with a buffer solution of pH 6.0 according to the Examples of the present invention, and (b) is a diagram showing a correlation of a citrulline concentration (mM) and enzyme activity (U/ml) measured using a dehydrogenase reaction of a citrulline oxidase mutant E486Q treated with a buffer solution of pH 6.0 according to the Examples of the present invention.

[Fig. 5] Fig. 5 is a diagram showing a correlation between a citrulline concentration and a current value at +0.4 V according to the Examples of the present invention.

[Fig. 6] Fig. 6 is a diagram showing a relationship between a citrulline-containing peptide concentration and an amount of change in absorbance per minute according to an embodiment of the present invention.

[Fig. 7] Fig. 7 is a partial view showing aligning citrulline oxidoreductases with a sequence identity of a full-length amino acid sequence of 67% or more.

[Fig. 8] Fig. 8 is a partial view showing aligning a citrulline oxidoreductase with sequence identity of a full-length amino acid sequence of 67% or more.

## DESCRIPTION OF EMBODIMENTS

Hereinafter, a new quantification method for measuring citrulline, which is a biomarker according to the present invention, an enzyme for quantification, a composition for quantification, and a kit for quantification will be described. However, a new quantification method for measuring citrulline, which is a biomarker according to the present invention, an enzyme for quantification, a composition for quantification, and a kit for quantification are not to be construed as being limited to the contents described in the embodiments and examples described below.

In one embodiment, the oxidoreductase used in the present invention is dehydrogenase that acts on the substrate citrulline. An enzyme capable of directly oxidizing or reducing citrulline has not been identified by the time of filing the present application. As a result of the examination by the inventors, a citrulline oxidoreductase derived from the genus Pseudomonas was found for the first time. In this specification, although a citrulline oxidoreductase derived from Pseudomonas and a mutant thereof will be shown and described as an example of a citrulline oxidoreductase, the present invention is not limited thereto, and may include those having a certain level of reactivity with citrulline.

For example, among oxidoreductases belonging to EC No. 1.4 or EC No. 1.5, an enzyme which recognizes citrulline as a substrate and has citrulline oxidoreductase activity can be used as a citrulline oxidoreductase. That is, it is possible to use an oxidase belonging to EC No. 1.4 or EC No. 1.5 which recognizes citrulline as a substrate and has citrulline oxidase activity, or dehydrogenase belonging to EC No. 1.4 or EC No. 1.5 which recognizes citrulline as a substrate and has citrulline dehydrogenase activity.

In one embodiment, the citrulline oxidoreductase may be an oxidoreductase produced by microorganisms existing in nature or an oxidoreductase produced by transformed microorganisms. From the viewpoint of efficient mass expression of enzymes, enzymes can be efficiently expressed in large quantities by using transformed microorganisms.

In one embodiment, a citrulline oxidoreductase may be a multimer or a monomer. For example, in the case where only a certain subunit (monomer) among several subunits constituting an oxidoreductase, which is a multimer, catalyzes a dehydrogenation reaction that takes hydrogen from a substrate and passes it to a hydrogen acceptor, then the oxidoreductase used in the present invention may be a multimer or the subunit (monomer). It may be composed of a partial structure of enzymes as long as it has citrulline oxidoreductase activity.

As described above, the inventors have found for the first time a citrulline oxidoreductase derived from Pseudomonas. In one embodiment, a citrulline oxidoreductase derived from a Pseudomonas sp. BYC41-1 strain can be exemplified as the citrulline oxidoreductase of the present invention but also a citrulline oxidoreductase derived from microorganisms classified into Pseudomonas. In one embodiment, it may be a citrulline oxidoreductase derived from Pseudomonas japonica, Pseudomonas putida, or Pseudomonas mosselii. With respect to the amino acid sequence of the citrulline oxidoreductase described in SEQ ID NO: 1, a citrulline oxidoreductase having high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, e.g., 99% or more), and a citrulline oxidoreductase having an amino acid sequence in which one to several amino acids have been modified or mutated, or deleted, substituted, added, and/or inserted in the amino acid sequence of SEQ ID NO: 1 may be exemplified. Furthermore, it is also possible to screen a citrulline oxidoreductase by culturing microorganisms of Pseudomonas under a predetermined condition (for example, see Journal of the Japanese Society for Bacteriology, 18 (1), 1963), mixing an oxidase or a dehydrogenase reaction reagent containing citrulline (described in detail later) with an extract obtained by crushing bacterial cells and confirming the presence or absence of reactivity with the reagent.

In one embodiment, the present invention provides DNA encoding of a citrulline oxidoreductase. In one embodiment, the present invention provides DNA encoding of the amino acid sequence shown in SEQ ID NO: 1 or DNA having the base sequence shown in SEQ ID NO: 2. In one embodiment, the present invention provides DNA having 60%, 61 %, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 90%, 90%, 91%, 92%, 93%, 94%, 95%, 96%, 97%, 98%, or 99% or more sequence identity with the base sequence shown in SEQ ID NO: 2 and encoding of a protein having citrulline oxidoreductase activity.

In one embodiment, when arginine is contained in a solution to be measured containing citrulline, a citrulline oxidoreductase preferably has high substrate specificity for citrulline and low substrate specificity for arginine. In other words, the ratio (Cit/Arg) of reactivity to citrulline relative to reactivity to arginine is preferably high. For example, it is preferable that Cit/Arg is 0.1% or more, preferably 1% or more, more preferably 3% or more, 5% or more, 10% or more, 20% or more, 30% or more, 40% or more, 50% or more, 60% or more, 70% or more, 80% or more, or 90% or more. More preferably, Cit/Arg is preferably 100% or more. Alternatively, it is preferred not to react with arginine.

In one embodiment, although the citrulline oxidoreductase of the present invention may be a citrulline oxidoreductase derived from a Pseudomonas sp. BYC41-1 strain or a citrulline oxidoreductase produced by E. coli transformed with a plasmid containing a citrulline oxidoreductase gene derived from a Pseudomonas sp. BYC41-1 strain, the citrulline oxidoreductase can be efficiently expressed in large quantities by using E. coli transformed with a plasmid containing a citrulline oxidoreductase gene derived from a Pseudomonas sp. BYC41-1 strain.

In one embodiment, a reaction condition of a citrulline oxidoreductase may be any condition as long as it is a condition for acting on citrulline and efficiently catalyzing an oxidation reaction or a reduction reaction. Generally, an enzyme has an optimum temperature and optimum pH that exhibit the highest activity. Therefore, it is suitable that the reaction condition is near the optimum temperature and the optimum pH. In one embodiment, the reaction condition of the citrulline oxidoreductase is comprehensively examined from a suitable condition for a component such as a composition other than an enzyme, for example, a coloring reagent, a mediator, a stabilizer of an enzyme, or a stabilizer of a measurement sample, compatibility with a measurement device, and the like, and a method of quantifying citrulline under conditions other than an optimum condition of an enzyme alone is also included in the measurement method of the present invention. Specifically, the reaction time of a citrulline oxidoreductase may be a certain period of time, e.g., 5 seconds or more, 10 seconds or more, or 20 seconds or more, less than 180 minutes or less than 150 minutes, e.g., 0.5 minutes to 120 minutes, preferably 0.5 minutes to 60 minutes, more preferably 1 minutes to 30 minutes, after mixing a citrulline oxidoreductase and a citrulline-containing sample. The working temperature of the citrulline oxidoreductase depends on the optimum temperature of the enzyme to be used, and is, for example, 20°C to 45°C, and the temperature used for a normal enzymatic reaction can be appropriately selected.

In one embodiment, a citrulline oxidoreductase derived from a Pseudomonas japonica strain can be exemplified as the citrulline oxidoreductase of the present invention. With respect to the amino acid sequence of the citrulline oxidoreductase described in SEQ ID NO: 31, a citrulline oxidoreductase having high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, e.g., 99% or more), and a citrulline oxidoreductase having an amino acid sequence in which one to several amino acids have been modified or mutated, or deleted, substituted, added, and/or inserted in the amino acid sequence of SEQ ID NO: 31 may be exemplified.

In one embodiment, the present invention provides DNA encoding of the amino acid sequence shown in SEQ ID NO: 31 or DNA having the base sequence shown in SEQ ID NO: 32. In one embodiment, the present invention provides DNA having 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 90%, 90%, 91%, 92%, 93%, 94%, 95%, 96%, 97%, 98%, or 99% or more sequence identity with the base sequence shown in SEQ ID NO: 32 and encoding of a protein having citrulline dehydrogenase activity.

In one embodiment, although the citrulline oxidoreductase of the present invention may be a citrulline oxidoreductase derived from a Pseudomonas japonica strain, or a citrulline oxidoreductase produced by E. coli transformed with a plasmid containing a citrulline oxidoreductase gene derived from a Pseudomonas japonica strain, the citrulline dehydrogenase can be efficiently expressed in large quantities by using E. coli transformed with a plasmid containing a citrulline dehydrogenase gene derived from a Pseudomonas japonica strain.

In one embodiment, a citrulline oxidoreductase derived from a Pseudomonas sp. WCHPs060044 strain can be exemplified as the citrulline oxidoreductase of the present invention. With respect to the amino acid sequence of the citrulline oxidoreductase described in SEQ ID NO: 60, a citrulline oxidoreductases having high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, e.g., 99% or more), and a citrulline oxidoreductase having an amino acid sequence in which one to several amino acids have been modified or mutated, or deleted, substituted, added, and/or inserted in the amino acid sequence of SEQ ID NO: 60 may be exemplified.

In one embodiment, the present invention provides DNA encoding of the amino acid sequence shown in SEQ ID NO: 60 or DNA having the base sequence shown in SEQ ID NO: 61. In one embodiment, the present invention provides DNA having 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 90%, 90%, 91%, 92%, 93%, 94%, 95%, 96%, 97%, 98%, or 99% or more sequence identity with the base sequence shown in SEQ ID NO: 61 and encoding of a protein having citrulline oxidoreductase activity.

In one embodiment, the citrulline oxidoreductase of the present invention may be a citrulline oxidoreductase derived from a Pseudomonas sp. WCHPs060044 strain, or a citrulline oxidoreductase produced by E. coli transformed with a plasmid containing a citrulline oxidoreductase gene derived from a Pseudomonas sp. WCHPs060044 strain, and the citrulline oxidoreductase can be efficiently expressed in large quantities by using E. coli transformed with a plasmid containing a citrulline oxidoreductase gene derived from a Pseudomonas sp. WCHPs060044 strain.

In one embodiment, AncARODn2, which is one of the amino acid sequences deduced on the basis of a Pseudomonas sp. TPU 7192 strain described in S. Nakano et. al., Appl. Environ. Microbiol. 2019 85 (12) e00459-19 can be exemplified as the citrulline oxidoreductase of the present invention. With respect to the amino acid sequence of the citrulline oxidoreductase described in SEQ ID NO: 81, a citrulline oxidoreductase having high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more,93% or more, 94% or more, 95% or more, 96% or more, 97% or more, 98% or more, e.g., 99% or more), and a citrulline oxidoreductase having an amino acid sequence in which one to several amino acids have been modified or mutated, or deleted, substituted, added, and/or inserted in the amino acid sequence of SEQ ID NO: 81 may be exemplified.

In one embodiment, the present invention provides DNA encoding of the amino acid sequence shown in SEQ ID NO: 81 or DNA having the base sequence shown in SEQ ID NO: 82. In one embodiment, the present invention provides DNA having 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 90%, 90%, 91%, 92%, 93%, 94%, 95%, 96%, 97%, 98%, or 99% or more sequence identity with the base sequence shown in SEQ ID NO: 61 and encoding of a protein having citrulline oxidoreductase activity.

In one embodiment, although the citrulline oxidoreductase of the present invention may be derived from computational science, for example, it may be the citrulline oxidoreductase described in SEQ ID NO: 81, or a citrulline oxidoreductase produced by E. coli transformed with a plasmid containing a citrulline oxidoreductase gene described in SEQ ID NO: 82, the citrulline dehydrogenase can be efficiently expressed in large quantities by using E. coli transformed with a plasmid containing a citrulline oxidoreductase gene described in SEQ ID NO: 82.

Suitable examples of microorganisms from which the citrulline oxidoreductase of the present invention is derived include microorganisms classified in the phylum Proteobacteria, preferably the class Gamma Proteobacteria, more preferably Pseudomonadales or Oceanospirillales, and even more preferably Pseudomonodaceae, Alteromonadaceae, or Oceanospirillaceae. Specifically, citrulline oxidoreductases derived from Pseudomonas, Oceanobacter, or Pseudoalteromonas are exemplified.

[Vector]

As a vector that can be used in the present invention, any vector known to those skilled in the art, such as, for example, a bacteriophage, cosmid, and the like, can be used without being limited to the above plasmid. Specifically, for example, pBluescriptII SK+ (manufactured by STRATAGENE), pET-22b(+) (manufactured by Merck), or the like is preferable.

[Mutation treatment of citrulline oxidoreductase gene]

Mutation treatment of a citrulline oxidoreductase gene can be performed by any known method, depending on the intended mutant form. That is, a method of contacting and acting a citrulline oxidoreductase gene or a recombinant DNA incorporating the gene with an agent serving as a mutagen, an ultraviolet irradiation method, a genetic engineering method, and a method of utilizing a protein engineering method, or the like can be widely used.

Hydroxylamine, N-methyl-N'-nitro-N-nitrosoguanidine, nitrous acid, sulfurous acid, hydrazine, formic acid, or 5-bromouracil can be exemplified as agents serving as a mutagen used in the above mutation treatment.

The conditions of the above-mentioned contact and action can be a condition depending on a type of the agent, and are not particularly limited as long as they can actually induce a desired mutation in the citrulline oxidoreductase gene. Typically, the desired mutation can be induced by contacting and acting for 10 minutes or more, preferably 10 to 180 minutes, at the agent concentration of 0.5 M to 12 M, at a reaction temperature of 20°C to 80°C. Ultraviolet irradiation can also be performed according to the conventional method as described above (CHEMISTRY TODAY, p. 24 to 30, June 1989).

Generally, a method known as Site-Specific Mutagenesis can be used as the method of utilizing a protein engineering method. For example, Kramer method (Nucleic Acids Res., 12, 9441 (1984): Methods Enzymol., 154, 350 (1987): Gene, 37, 73 (1985)), Eckstein method (Nucleic Acids Res., 13, 8749 (1985): Nucleic Acids Res., 13, 8765 (1985): Nucleic Acids Res, 14, 9679 (1986)), Kunkel method (Proc. Natl. Acid. Sci. U.S.A., 82, 488 (1985): Methods Enzymol., 154, 367 (1987)), and the like can be exemplified. Specific methods for converting sequences in DNA, for example, the use of commercially available kits (Transformer Mutagenesis Kit; Clonetech, EXOIII/Mung Bean Deletion Kit; manufactured by Stratagene, Quick Change Site Directed Mutagenesis Kit; manufactured by Stratagene, and the like) can be exemplified.

A method known as the common PCR method (Polymerase Chain Reaction) can also be used (Technique, 1, 11 (1989)). In addition to the above gene modification method, a desired modified citrulline oxidoreductase gene can be directly synthesized by an organic synthesis method or an enzyme synthesis method.

Determination or confirmation of the DNA sequence of the citrulline oxidoreductase gene obtained by the above methods can be performed by using, for example, a multicapillary DNA analysis system CEQ 2000 (manufactured by Beckman Coulter) or the like.

[Transformation and transduction]

The citrulline oxidoreductase gene obtained as described above can be incorporated into a vector such as a bacteriophage, cosmid, or a plasmid used for transformation of a prokaryotic cell or a eukaryotic cell by a conventional method, and a host corresponding to each vector can be transformed or transduced by a conventional method. For example, the obtained recombinant DNA can be used to transform or transduce any host, for example, microorganisms belonging to the Escherichia, in particular, E. coli K-12 strains, preferably E. coli JM109 strains, E. coli DH5$\alpha$ strains (both produced by Takara Bio Inc.), E. coli B strains, preferably E. coli BL21 strains (produced by Nippon Gene Co., Ltd.) or the like to obtain each strain.

For example, an example of a eukaryotic host cell includes yeast. For example, yeast belonging to Zygosaccharomyces, Saccharomyces, Pichia, Candida can be exemplified as microorganisms classified as yeast. The insertion gene may include a marker gene to allow the selection of transformed cells. The marker gene includes, for example, genes that complement auxotrophy of the host, such as URA3, TRP1. The insertion gene preferably contains a promoter or other control sequence capable of expressing the gene of the present invention in a host cell (e.g., secretion signal sequence, enhancer sequence, terminator sequence, polyadenylation sequence, and the like). Specific examples of the promoter include a GAL1 promoter, a ADH1 promoter, and the like. Although a known method, for example, a method using lithium acetate (Methods Mol. Cell. Biol., 5, 255-269 (1995)), electroporation (J Microbiol Methods 55 (2003) 481-484), or the like, can be suitably used as a transformation method to yeast, the present invention is not limited thereto, and transformation can be performed using various optional methods including a spheroplast method, a glass beads method, and the like.

For example, other examples of a eukaryotic host cell include filamentous fungi such as Aspergillus and Trichoderma. A method of producing a transformant of the filamentous fungi is not particularly limited, and for example, it includes a method of inserting into the host filamentous fungi in a manner in which a gene encoding of a citrulline oxidoreductase is expressed according to a conventional method. Specifically, a DNA construct in which a gene encoding of a citrulline oxidoreductase is inserted between an expression-inducing promoter and terminator is prepared, the host filamentous fungi is transformed with a DNA construct containing the gene encoding of a citrulline oxidoreductase, and then a transformant overexpressing the gene encoding of a citrulline oxidoreductase is obtained. In this specification, a DNA fragment consisting of an expression-inducing promoter - gene encoding of a citrulline oxidoreductase - terminator produced for transforming host filamentous fungi, and a recombinant vector containing the DNA fragment are collectively referred to as a DNA construct.

The method of inserting into the host filamentous fungi in a manner in which a gene encoding of a citrulline oxidoreductase is expressed is not particularly limited, and for example, a method of directly inserting into a chromosome of the host organism by utilizing homologous recombination; a method of introducing into the host filamentous fungi by ligating the gene into a plasmid vector, and the like can be exemplified.

In the method utilizing homologous recombination, a DNA construct can be ligated between sequences homologous to the upstream region and downstream region of the recombination site on a chromosome and inserted into a genome of host filamentous fungi. Transformants by self-cloning can be obtained by overexpressing in the host filamentous fungi under the control of the high expression promoter of the host filamentous fungi itself. The high expression promoter is not particularly limited, and examples thereof include a promoter region of a TEF1 gene (tef1), which is a translation elongation factor, a promoter region of an $\alpha$-amylase gene (amy), and a promoter region of an alkaline protease gene (alp).

In the method utilizing a vector, a DNA construct can be incorporated into a plasmid vector used in the transformation of filamentous fungi in a conventional method, and the corresponding host filamentous fungi can be transformed by a conventional method.

Such suitable vector-host systems are not particularly limited as long as they are capable of producing a citrulline oxidoreductase in the host filamentous fungi, for example, pUC19 and filamentous fungal systems, and pSTA14 (Mol.

Gen. Genet. 218, 99-104, 1989) and filamentous fungal systems and the like can be exemplified.

Although the DNA construct is preferably used by introducing it into the chromosome of the host filamentous fungi, it can also be used by incorporating the DNA construct into an autonomously replicated vector without introducing it into the chromosome (Ozeki et al. Biosci. Biotechnol. Biochem 59, 1133 (1995)).

The DNA construct may include a marker gene to allow the selection of transformed cells. The marker gene is not particularly limited, and for example, genes that complement auxotrophy of the host such as pyrG, niaD, adeA; and a drug resistance gene against a drug such as pyritiamine, hygromycin B, or oligomycin can be exemplified. The DNA construct preferably contains promoters, terminators, or other regulatory sequences (e.g., enhancers, polyadenylation sequences, etc.) that allow overexpression of the gene encoding of a citrulline oxidoreductase in the host cell. The promoter is not particularly limited, and examples thereof include a suitable expression-induced promoter and a constitutive promoter, such as a tef1 promoter, an alp-promoter, amy-promoter, and the like. The terminator is also not particularly limited, and examples thereof include an alp terminator, an amy terminator, and a tef1 terminator.

In a DNA construct, the expression control sequence of the gene encoding of a citrulline oxidoreductase is not necessarily required in the case where the DNA fragment containing a gene encoding of a citrulline oxidoreductase to be inserted contains a sequence having the expression control function. In the case where transformation is performed by a co-transformation method, the DNA construct may not have a marker gene.

One embodiment of the DNA construct is a DNA construct in which, for example, a tef1 gene promoter, a gene encoding of a citrulline oxidoreductase, an alp gene terminator, and a pyrG marker gene are linked to an In-Fusion Cloning Site located at a multicloning site of pUC19.

As a method for transforming into filamentous fungi, a method known to those skilled in the art can be appropriately selected, and for example, a protoplast PEG method using polyethylene glycol and calcium chloride (see, for example, Mol. Gne. Genet. 218, 99-104, 1989, Japanese laid-open patent publication No. 2007-222055, and the like) can be used after preparing a protoplast of a host filamentous fungi. An appropriate medium is used for the regeneration of transformation filamentous fungi depending on the host filamentous fungi and the transformation marker gene to be used. For example, in the case where Aspergillus sojae is used as the host filamentous fungi and pyrG gene is used as the transformation marker gene, the regeneration of the transformation filamentous fungi can be performed, for example, in a Czapek-Dox minimal medium containing 0.5% agar and 1.2 M sorbitol (produced by Difco).

[Identity or similarity of amino acid sequence]

The identity or similarity of the amino acid sequence can be calculated by a program such as maximum matching or search homology of GENETYX Ver. 11 (manufactured by Genetyx Corporation) or a program such as maximum matching or multiple alignment of DNASIS Pro (manufactured by Hitachi Solutions Ltd.). In order to calculate amino acid sequence identity, the positions of amino acids that are identical in the two or more citrulline oxidoreductases can be examined when two or more citrulline oxidoreductases are aligned. Based on this information, the same region in the amino acid sequence can be determined.

Positions of amino acids that are similar in two or more citrulline oxidoreductases can also be examined. For example, CLUSTALW can be used to align a plurality of amino acid sequences, and in this case, Blosum62 is used as an algorithm, and amino acids that are judged to be similar when the plurality of amino acid sequences is aligned are sometimes referred to as similar amino acids. In the mutant of the invention, the amino acid substitution may occur by substitution between such similar amino acids. By such an alignment, for a plurality of amino acid sequences, it is possible to investigate the region having the same amino acid sequence and the position occupied by similar amino acids. Based on this information, the homology region (conservation region) in the amino acid sequence can be determined.

In this specification, "homology region" refers to a region in which, when two or more citrulline oxidoreductases are aligned, the amino acid at the corresponding position of a reference citrulline oxidoreductase and a comparison citrulline oxidoreductase is the same or is composed of similar amino acids, and is composed of 3 or more, 4 or more, 5 or more, 6 or more, 7 or more, 8 or more, 9 or more or 10 or more consecutive amino acids. For example, in Fig. 7 and Fig. 8, a citrulline oxidoreductase having a sequence identity of 67% or more in the full-length amino acid sequence was aligned. Of these, 10th to 18th are composed of the same amino acids based on the citrulline oxidoreductase shown in SEQ ID NO: 1 and therefore correspond to the homology region. Similarly, based on the citrulline oxidoreductase shown in SEQ ID NO: 1, 20th to 26th, 34th to 40th, 43rd to 50th, 53rd to 67th, 96th to 99th, 113th to 119th, 125th to 127th, 134th to 136th, 142nd to 144th, 147th to 149th, 151st to 155th, 160th to 162nd, 183rd to 186th, 192nd to 196th, 198th to 214th, 216th to 225th, 237th to 239th, 241st to 245th, 312th to 314th, 316th to 323rd, 339th to 341st, 345th to 347th, 350th to 353rd, 355th to 358th, 361st to 363rd, 365th to 375th, 377th to 383rd, 393rd to 407th, 409th to 413th, 467th to 472nd, 481st to 489th, 500th to 504th, 511th to 518th, 520th to 527th and 530th to 532nd may correspond to the homology region.

In an embodiment, based on the citrulline oxidoreductase shown in SEQ ID NO: 1, the homology region of a citrulline oxidoreductase is a region composed of the amino acid sequence of 10th to 18th, 20th to 26th, 33rd to 35th, 37th to 40th, 43rd to 48th, 53rd to 62nd, 64th to 67th, 96th to 99th, 113th to 119th, 125th to 127th, 134th to 136th, 140th to 144th, 146th to 149th, 151st to 155th, 160th to 162nd, 170th to 186th, 192nd to 196th, 198th to 214th, 216th to 227th, 231st to 235th, 237th to 239th, 241st to 245th, 311th to 314th, 316th to 323rd, 330th to 334th, 339th to 341st, 345th to 347th, 352nd to 360th, 361st to 375th, 377th to 383rd, 393rd to 407th, 409th to 413th, 467th to 472nd, 481st to 490th, 500th to 504th, 510th to 518th, 520th to 527th, and 529th to 534th.

The citrulline oxidoreductase of the present invention has a full-length amino acid sequence identity of 50% or more, 55% or more, 60% or more, 65% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, e.g., 99% or more when aligned with the citrulline oxidoreductase having an amino acid sequence shown in SEQ ID NO: 1, and has citrulline oxidoreductase activity. Furthermore, the amino acid sequence in the homology region of the citrulline oxidoreductase of the present invention has a sequence identity of 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, e.g., 99% or more with the amino acid sequence in the homology region in SEQ ID NO: 1.

Similarly, based on the citrulline oxidoreductase shown in SEQ ID NO: 81, 8th to 16th are composed of the same amino acids and therefore correspond to the homology region. Similarly, 18th to 24th, 32nd to 38th, 41st to 48th, 51st to 65th, 88th to 91st, 115th to 121st, 127th to 129th, 136th to 138th, 144th to 146th, 149th to 151st, 153rd to 157th, 162nd to 164th, 185th to 188th, 194th to 198th, 200th to 216th, 218th to 227th, 239th to 241st, 243rd to 247th, 308th to 310th, 312th to 319th, 335th to 337th, 341st to 343rd, 346th to 349th, 351st to 354th, 357th to 359th, 361st to 371st, 373rd to 379th, 389th to 403rd, 405th to 409th, 463rd to 468th, 477th to 485th, 496th to 500th, 507th to 514th, 516th to 523rd, and 526th to 528th may correspond to the homology region.

The citrulline oxidoreductase of the present invention has a full-length amino acid sequence identity of 50% or more, 55% or more, 65% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, and 87% or more, 88% or more, 89% or more, 90% or more, 91% or more, 92% or more, 93% or more, 94% or more, 95% or more, 96% or more, 97% or more, 98% or more, e.g., 99% or more when aligned with the citrulline oxidoreductase having an amino acid sequence shown in SEQ ID NO: 81, and has citrulline oxidoreductase activity. Furthermore, the amino acid sequence in the homology region of the citrulline oxidoreductase of the present invention has a sequence identity of 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, e.g., 99% or more with the amino acid sequence in the homology region in SEQ ID NO: 81.

[Corresponding position]

A position corresponding to a position of 486th in the amino acid sequence shown in SEQ ID NO: 1 refers to the position in the amino acid sequence of a citrulline oxidoreductase derived from other species corresponding to the position of 486th in the amino acid sequence of SEQ ID NO: 1 when aligned with the amino acid sequence of SEQ ID NO: 1.

As a method of identifying a "corresponding position", for example, a known algorithm such as a Lipman-Pearson method is first used to compare amino acid sequences, and a multiple alignment is performed to give maximum identity to a conserved amino acid residue present in the amino acid sequence of each citrulline oxidoreductase. By aligning the amino acid sequences of the citrulline oxidoreductases in this manner, it is possible to determine positions of the homologous amino acid residues in the sequence in each citrulline oxidoreductase sequence regardless of insertions or deletions in the amino acid sequence. Then, in some cases, secondary structures such as $\alpha$ helices, $\beta$ sheets, and coils can be predicted using known secondary structure prediction algorithms or the like.

For example, with respect to the amino acid sequence shown in SEQ ID NO: 1 or an amino acid sequence of a suitable citrulline oxidoreductase, its secondary structure can be predicted by using the secondary structure prediction algorithm. Jpred 3 (Cole C et al. The Jpred 3 secondary structure prediction server. Nucleic Acids Res. 2008,: W197-201) and Jpred4 (Drozdetskiy A et al. (2015) JPred4: a protein secondary structure prediction server, Nucleic Acids Res., doi:10.1093/nar/gkv332) implementing a JNet algorithm can be exemplified as secondary structural prediction tools.

[Enzyme preparation method]

Hereinafter, a method for preparing a citrulline oxidoreductase according to the present invention will be described.

[Construction of expression plasmid]

A plasmid for expressing a citrulline oxidoreductase according to the present invention is obtained by a commonly used method. For example, DNA is extracted from a microorganism producing a citrulline oxidoreductase according to the present invention to create a DNA library. From the created DNA library, a DNA fragment encoding of the citrulline oxidoreductase according to the present invention is identified and isolated. Complementary primers using the isolated DNA fragment as a template are used to amplify the DNA fragment by polymerase chain reaction (PCR) to clone a gene encoding of the citrulline oxidoreductase according to the present invention. The amplified DNA fragment is ligated into a vector to obtain a plasmid having a DNA fragment encoding of the citrulline oxidoreductase according to the present invention.

Alternatively, the DNA fragment encoding of the citrulline oxidoreductase according to the present invention is chemically synthesized, and the DNA fragment is ligated into a vector to obtain a plasmid having DNA encoding of the citrulline oxidoreductase according to the present invention.

A strain of E. coli or the like is transformed with the obtained plasmid to obtain a strain of E. coli or the like having DNA encoding of the citrulline oxidoreductase according to the present invention.

[Recombinant expression of enzyme].

The strain of E. coli or the like having DNA encoding of the citrulline oxidoreductase according to the present invention is cultured in a culture medium. When culturing a microbial host cell, it may be carried out by aeration stirring deep culture, shaking culture, static culture, or the like, at a culture temperature of 10°C to 42°C, preferably at a culture temperature of about 25°C for several hours to several days, more preferably at a culture temperature of about 25°C for 1 to 7 days. Any synthetic medium or a natural medium can be used as long as it contains a normal medium for culturing filamentous fungi, that is, a carbon source, a nitrogen source, an inorganic substance, or other nutrients in an appropriate ratio. For example, one or more of inorganic salt such as sodium chloride, monopotassium phosphate, dipotassium phosphate, magnesium sulfate, magnesium chloride, ferric chloride, ferric sulfate, or manganese sulfate or the like is added to one or more nitrogen sources such as yeast extract, tryptone, peptone, meat extract, corn steep liquor or a leaching solution of soybean or wheat bran, and further, a sugar raw material, a vitamin, or the like is appropriately added according to necessity and used as the medium for culturing the microbial host cell.

Culture conditions of filamentous fungi commonly known by those skilled in the art may be adopted as culture conditions, and for example, an initial pH of the medium may be adjusted to 5 to 10, and the culture temperature may be appropriately set to 20°C to 40°C, the culture time may be set for several hours to several days, preferably for 1 to 7 days, more preferably for 2 to 5 days, and the like. The culture means is not particularly limited, and aeration stirring deep culture, shaking culture, static culture, and the like can be adopted, and it is preferable to culture under conditions such that dissolved oxygen becomes sufficient. For example, examples of a medium and a culture condition when culturing an Aspergillus microorganism include a shaking culture at 30°C at 160 rpm for 3 to 5 days using a DPY medium as described in the Examples described later.

After completion of the culture, the citrulline oxidoreductase of the present invention is collected from the culture. An ordinary known enzyme collection means may be used for this. For example, the culture medium supernatant fraction is collected, or the enzyme can be extracted by ultrasonically destroying, grinding, etc. the cell body by a conventional method, or using a lytic enzyme such as lysozyme or yatalase, the enzyme is discharged to the outside of the cell body by shaking or leaving in the presence of toluene or the like to bacteriolyse. Then, the solution is filtered, centrifuged, or the like to remove a solid portion, and if necessary, a nucleic acid is removed with streptomycin sulfate, protamine sulfate, or manganese sulfate or the like, and then ammonium sulfate, alcohol, acetone, and the like are added to fractionate it, and a precipitate is collected to obtain a crude enzyme of the citrulline oxidoreductase of the present invention.

[Purification of enzyme]

The method for purifying an enzyme may be any method as long as it is capable of purifying an enzyme from a crude enzyme solution. For example, a purified citrulline oxidoreductase enzyme preparation of the present invention can be obtained by appropriately selecting or combining a gel filtration method using Sephadex, Ultrogel or Biogel, or the like; an adsorption elution method using an ion exchanger; an electrophoresis method using a polyacrylamide gel or the like; an adsorption elution method using hydroxyapatite; a sedimentation method such as a sucrose density gradient centrifugation method; an affinity chromatography method; a fractionation method using a molecular sieve membrane or a hollow fiber membrane, or the like.

[Enzyme activity measurement]

A method for measuring the activity of an enzyme may be any method as long as it is a method for directly or indirectly measuring a product of a redox reaction catalyzed by an enzyme. For example, a reduced product is produced by catalyzing a redox reaction by an enzyme, and a current value generated by passing electrons from the reduced product to an electrode is measured, so that enzyme activity can be measured. Suitably, the enzyme activity can be measured by reacting a reduced product by a redox reaction catalyzed by an enzyme with a reagent containing an absorbing substance reacting with the reduced product (hereinafter, an "absorbing reagent") and performing an absorbance measurement.

[Composition containing citrulline oxidoreductase and kit for quantification of citrulline]

A quantification method of citrulline utilizing the citrulline oxidoreductase according to the present invention may be carried out by providing a composition containing a citrulline oxidoreductase and a product reaction reagent or may be carried out by combining a citrulline oxidoreductase with a commercially available product reaction reagent.

Since the quantification method of citrulline, the citrulline oxidoreductase for quantification, the composition for quantification, and the kit for quantification according to the present invention contain a citrulline oxidoreductase, it is possible to provide a new quantification method, an enzyme for quantification, a composition for quantification, and a kit for quantification, which quantify the concentration of a citrullinated peptide, which is a biomarker of diseases associated with abnormal citrullination of proteins, such as, multiple sclerosis, Alzheimer's disease, chronic rheumatoid arthritis, psoriasis, prion disease, liver fibrosis, chronic obstructive pulmonary disease, cancer, or the like, or the citrulline concentration released from the citrullinated peptide.

[Composition containing citrulline oxidase and kit for quantification of citrulline]

In one embodiment, citrulline may be quantified utilizing a citrulline oxidase according to the present invention. The quantification method of citrulline utilizing a citrulline oxidase according to the present invention may be carried out by providing a composition containing a citrulline oxidase and a product reaction reagent or may be carried out by combining a citrulline oxidase with a commercially available product reaction reagent. For example, it may be provided as a composition for quantification of a citrulline containing a citrulline oxidase or a composition for quantification of a citrulline further containing a reagent reacting with hydrogen peroxide produced by addition of the citrulline oxidase. It may be provided as a kit for quantification of a citrulline containing a citrulline oxidase and a reagent reacting with hydrogen peroxide produced by addition of the citrulline oxidase.

[Citrulline measurement sensor]

In one embodiment, a citrulline measurement sensor using the citrulline oxidase of the present invention is provided. Fig. 1 (a) is a schematic diagram of a sensor 100 according to one embodiment of the present invention. The sensor 100 is a citrulline measuring device using a citrulline oxidase and includes a sensor chip 10 containing a citrulline oxidase and a measurement unit 30. The measurement unit 30 may include, for example, a switch 31 serving as an input unit and a display 33 serving as a display unit. The switch 31 may be used, for example, to control ON/OFF of a power supply of the measurement unit 30, or to control the start and interruption of citrulline in the sensor 100. The display 33 may display, for example, a measured value of citrulline and may include a touch panel as an input unit for controlling the measurement unit 30.

Fig. 1 (b) is a block diagram of the sensor 100 according to one embodiment of the present invention. The sensor 100 may include, for example, a control unit 110, a display unit 120, an input unit 130, a memory unit 140, a communication unit 150, and a power supply 160 in the measurement unit 30, which may be electrically connected to each other by a wiring 190. Further, a terminal of the sensor chip 10 to be described later and a terminal of the measurement unit 30 are electrically connected, and a current generated by the sensor chip 10 is detected by the control unit 110. The control unit 110 is a control device configured to control the sensor 100 and is composed of, for example, a known central processing unit (CPU) and an operation program configured to control the sensor 100. The control unit 110 includes a central processing unit and an operating system (OS) and may include an application program or module for performing a citrulline measurement.

The display unit 120 may include, for example, the known display 33, and may display measured values of citrulline, states of the measurement unit 30, and operation requests to a measurer. The input unit 130 is an input device for the measurer to operate the sensor 100, and may be for example, a touch panel arranged on the switch 31 or the display 33. A plurality of switches 31 may be arranged in the measurement unit 30.

The memory unit 140 is composed of a main memory device (memory) and an auxiliary memory device (hard disks) may be arranged externally. The main memory device (memory) may be composed of a read-only memory (ROM) and/or a random-access memory (RAM). An operation program, operating system, application program, or module is stored in the memory unit 140, and executed by a central processing unit to constitute the control unit 110. The measured values and the current values can be stored in the memory unit 140.

The communication unit 150 is a known communication device that connects the sensor 100 or the measurement unit 30 to external devices (computers, printers, or networks). The communication unit 150 and the external devices are connected by wired or wireless communication. The power supply 160 is a known power supply device that supplies power to the sensor 100 or the measurement unit 30.

[Sensor chip]

Fig. 2 (a) is a schematic diagram of the sensor chip 10 according to one embodiment of the present invention, and Fig. 2 (b) to Fig. 2 (d) are schematic diagrams showing members composing the sensor chip 10. The sensor chip 10 includes two or more electrodes arranged on a base material 11. The base material 11 is made of an insulating material. In Fig. 2 (a) and Fig. 2 (b), a working electrode 1, a counter electrode 3, and a reference electrode 5 are arranged on the base material 11 as an example. Each electrode is electrically connected to a wiring portion 7, respectively, and the wiring portion 7 is electrically connected to a terminal 9 located on the opposite side of a wiring direction from each electrode. The working electrode 1, the counter electrode 3, and the reference electrode 5 are arranged apart from each other. The working electrode 1, the counter electrode 3, and the reference electrode 5 are preferably formed integrally with the wiring portion 7 and the terminal 9. The counter electrode 3 and the reference electrode 5 may be integrated.

As shown in Fig. 2 (a) and Fig. 2 (c), a spacer 13 is arranged on an end portion of the base material 11 parallel to the wiring portion 7, and a cover 15 is arranged that covers the working electrode 1, the counter electrode 3, the reference electrode 5, and the spacer 13. The spacer 13 and the cover 15 are made of an insulating material. The spacer 13 preferably has a thickness substantially equal to that of the working electrode 1, the counter electrode 3, and the reference electrode 5, and is in close contact with the working electrode 1, the counter electrode 3, and the reference electrode 5. The spacer 13 and the cover 15 may be integrally configured. The cover 15 is a protective layer for preventing the wiring portion 7 from deteriorating due to exposure to the outside air or short circuit due to bleeding of the measurement sample.

As shown in Fig. 2 (a) and Fig. 2 (d), a reaction layer 19 is arranged on the working electrode 1, the counter electrode 3, and the reference electrode 5. The reaction layer 19 provides a field of reaction of citrulline with a citrulline oxidase. In one embodiment, the citrulline oxidase of the present invention may be applied, adsorbed, or immobilized on these electrodes. Preferably, the citrulline oxidase of the present invention is applied, adsorbed, or immobilized on the working electrode. In another embodiment, a mediator together with a citrulline oxidase may also be applied, adsorbed, or immobilized on the electrode. A carbon electrode, a metal electrode such as platinum, gold, silver, nickel, or palladium can be used as the electrode. In the case of a carbon electrode, pyrolytic graphite carbon (PG), glassy carbon (GC), carbon paste, and plastic foamed carbon (PFC) can be exemplified as materials. A measurement system may be a two-electrode system or a three-electrode system and, for example, an enzyme may be immobilized on the working electrode. A standard hydrogen electrode, a reversible hydrogen electrode, a silver-silver chloride electrode (Ag/AgCl), a palladium-hydrogen electrode, and a saturated calomel electrode can be exemplified as the reference electrode, and the Ag/AgCl is preferably used from the viewpoint of stability and reproducibility.

The enzyme can be immobilized on the electrode by crosslinking, coating with a dialysis membrane, encapsulation in a polymer matrix, by use of a photocrosslinkable polymer, by use of an electrically conductive polymer, or by use of an oxidation/reduction polymer, and the like. An enzyme may be immobilized in a polymer with the mediator or adsorbed and immobilized on the electrode, and these techniques may be combined.

The citrulline oxidase of the present invention can be applied to various electrochemical measurement methods by using a potentiostat, a galvanostat, or the like. Various techniques such as amperometry, potentiometry, and coulometry can be exemplified as electrochemical measurements. For example, by an amperometry method, a citrulline concentration in a sample can be calculated by measuring a current value generated by applying from +600 to +1000 mV (vs. Ag/AgCl) from the power supply 160 to hydrogen peroxide produced when citrulline oxidase reacts with citrulline by a hydrogen peroxide electrode by the control unit 110. For example, a calibration curve can be created by measuring current values for known citrulline concentrations (5, 10, 20, 30, 40, 50 mM) and plotting against the citrulline concentration. The citrulline concentration can be obtained from the calibration curve by measuring the current value of unknown citrulline. For example, a platinum electrode can be used as the hydrogen peroxide electrode. The amount of hydrogen peroxide can be quantified by measuring the reduction current generated by applying -400 mV to +100 mV (vs. Ag/AgCl) using an electrode immobilized with a reductase such as peroxidase or catalase, instead of the hydrogen peroxide electrode, and the value of citrulline can also be measured.

In addition, a printing electrode can also be used to reduce the amount of a solution required for measurement.

In this case, the electrode is preferably formed on the base material 11 made of an insulating substrate. Specifically, it is desirable that the electrode is formed on the base material 11 by a photolithography technique or a printing technique such as screen printing, gravure printing, or flexographic printing. Although silicon, glass, ceramic, polyvinyl chloride, polyethylene, polypropylene, and polyester can be exemplified as a material of the insulating substrate, a material having strong resistance to various solvents and chemicals is preferably used.

As described above, the quantification method of citrulline according to the present invention, the citrulline oxidase for quantification, the composition for quantification, and the kit for quantification can provide a new quantification method, an enzyme for quantification, a composition for quantification, and a kit for quantification, which quantify the citrulline concentration, which is a biomarker of diseases associated with abnormal citrullination of proteins, such as, multiple sclerosis, Alzheimer's disease, chronic rheumatoid arthritis, psoriasis, prion disease, liver fibrosis, chronic obstructive pulmonary disease, cancer, and the like by containing a citrulline oxidase.

[Quantification method of citrulline using citrulline oxidase reaction]

The citrulline oxidase used in the present invention is an oxidase that acts on citrulline as a substrate. However, a citrulline oxidase has not been identified by the time of filing the present application.

In one embodiment, the citrulline oxidase may be selected from the citrulline oxidoreductase described above or a mutant having high citrulline oxidase activity among the citrulline oxidoreductase described above. In one embodiment, the citrulline oxidase is a citrulline oxidase derived from Pseudomonas. With respect to the amino acid sequence described in SEQ ID NO: 1, a citrulline oxidase having high sequence identity (for example, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, e.g., 99% or more), and a citrulline oxidase having an amino acid sequence in which one to several amino acids have been modified or mutated, or deleted, substituted, added, and/or inserted in the amino acid sequence of SEQ ID NO: 1 may be exemplified.

In one embodiment, although the citrulline oxidase may be a citrulline oxidase derived from a Pseudomonas sp. BYC41-1 strain or a citrulline oxidase produced by E. coli transformed with a plasmid containing a citrulline oxidase gene derived from a Pseudomonas sp. BYC41-1 strain, the citrulline oxidase can be efficiently expressed in large quantities by using E. coli transformed with a plasmid containing a citrulline oxidase gene derived from a Pseudomonas sp. BYC41-1 strain.

In one embodiment, the citrulline oxidase may be a citrulline oxidase derived from a Pseudomonas japonica strain or a citrulline oxidase produced by E. coli transformed with a plasmid containing the citrulline oxidase gene derived from a Pseudomonas japonica strain, and the citrulline oxidase can be efficiently expressed in large quantities by using E. coli transformed with a plasmid containing a citrulline oxidase gene derived from a Pseudomonas japonica strain.

In one embodiment, although the citrulline oxidase may be a citrulline oxidase derived from a Pseudomonas sp. WCHPs060044 strain or a citrulline oxidase produced by E. coli transformed with a plasmid containing a citrulline oxidase gene derived from a Pseudomonas sp. WCHPs060044 strain, the citrulline oxidase can be efficiently expressed in large quantities by using E. coli transformed with a plasmid containing a citrulline oxidase gene derived from a Pseudomonas sp. WCHPs060044 strain.

In one embodiment, although the citrulline oxidase may be the citrulline oxidase described in SEQ ID NO: 81 or the citrulline oxidase produced by E. coli transformed with a plasmid containing the citrulline oxidase gene described in SEQ ID NO: 82, the citrulline oxidase can be efficiently expressed in large quantities by using E. coli transformed with a plasmid containing a citrulline oxidase gene described in SEQ ID NO: 82.

Suitable examples of microorganisms from which the citrulline oxidase of the present invention is derived include microorganisms classified in the phylum Proteobacteria, preferably the class Gamma Proteobacteria, more preferably Pseudomonas or Oceanospirillales, and even more preferably Pseudomonadales, Alteromonadaceae, or Oceanospirillaceae. Specifically, citrulline oxidases derived from Pseudomonas, Oceanobacter, or Pseudarlteromonas are exemplified.

In one embodiment, the reaction condition of the citrulline oxidase may be any condition as long as it is a condition for acting on citrulline and efficiently catalyzing an oxidation reaction. Generally, an enzyme has an optimum temperature and optimum pH that exhibit the highest activity. Therefore, it is suitable that the reaction condition is near the optimum temperature and the optimum pH.

In one embodiment, as the reaction process of a citrulline oxidase, various chemicals may be involved when the citrulline oxidase of the present invention acts on citrulline. For example, when the citrulline oxidase acts on citrulline, oxygen may be involved as an electron acceptor in a redox reaction.

As citrullinated proteins, myelin basic protein, filaggrin, histone protein, fibrin, vimentin, fibrinogen, and the like are known. In one embodiment, when quantification of citrulline in blood as an analyte is performed, the sample may be optionally selected from whole blood, plasma, and serum depending on the citrullinated protein to be measured. Citrulline or a composition for quantification of citrulline containing a citrulline oxidase may be directly mixed with a sample, and a sample may be pretreated before mixing with the citrulline oxidase or the composition for quantification of citrulline containing the citrulline oxidase. For example, the citrullinated protein may be degraded with a protease and/or peptidase to liberate the citrulline and then mixed with a citrulline oxidase or a composition for quantification of citrulline containing a citrulline oxidase.

A range of quantification of citrulline is not particularly limited, and may be, for example, 0.001 mM to 1000 mM, 0.01 mM to 500 mM, 0.01 mM to 300 mM, 0.01 mM to 100 mM, 0.01 mM to 50 mM, 0.01 mM to 30 mM, 0.01 mM to 20 mM, and the like.

[Enzyme preparation method]

The citrulline oxidase according to the present invention can be prepared by a preparation method similar to that of the citrulline oxidoreductase described above.

[Construction of expression plasmid]

For example, similar to the method for preparing a citrulline oxidoreductase described above, a plasm id for expression is constructed, or a DNA fragment encoding of the citrulline oxidase according to the present invention is chemically synthesized, and the DNA fragment is ligated into a vector to obtain a plasmid having a DNA fragment encoding of the citrulline oxidase according to the present invention. A strain of E. coli or the like is transformed with the obtained plasmid to obtain a strain of E. coli or the like having DNA encoding of the citrulline oxidase according to the present invention.

[Recombinant expression and purification of enzyme]

Expression and purification of a citrulline oxidase may be performed by the same method as in the expression and purification of the oxidoreductase described above, and a detailed description thereof will be omitted.

[Enzyme activity measurement]

A method for measuring the activity of an enzyme may be any method as long as it is a method for directly or indirectly measuring a product of a reaction catalyzed by an enzyme. For example, if a product by a reaction catalyzed by an enzyme is reacted with a reagent reacting with the product (hereinafter, a "product reaction reagent") and an absorbing substance produced by the reaction is measured, the enzyme activity can be measured by performing an absorbance measurement.

[Composition containing citrulline dehydrogenase and kit for quantification of citrulline]

In one embodiment, citrulline may be quantified utilizing a citrulline dehydrogenase according to the present invention. A quantification method of citrulline utilizing a citrulline dehydrogenase according to the present invention may be carried out by providing a composition containing a citrulline dehydrogenase and a product reaction reagent, or may be carried out by combining a citrulline dehydrogenase with a commercially available product reaction reagent. For example, it may be provided as a composition for quantification of citrulline containing a citrulline dehydrogenase, or a composition for quantification of citrulline further including a mediator reduced by addition of the citrulline dehydrogenase and a reagent reacting with the reduced mediator. It may be provided as a kit for quantification of citrulline including a citrulline dehydrogenase, a mediator reduced by addition of the citrulline dehydrogenase, and a reagent reacting with the reduced mediator.

The mediator (also referred to as an artificial electron mediator, an artificial electron acceptor, or an electron mediator) used in the measurement method or the kit for quantification of the present invention is not particularly limited as long as it can receive an electron from a citrulline dehydrogenase. Quinones, phenazines, viologens, cytochromes, phenoxazines, phenothiazines, phenylenediamines, ferricyanides, e.g., potassium ferricyanide, ferredoxins, ferrocene, ruthenium complexes, osmium complexes and derivatives thereof can be exemplified as the mediator, and PMS and methoxy PMS can be exemplified as the phenazine compounds, but are not limited thereto.

[Citrulline measurement sensor]

In one embodiment, a citrulline measurement sensor using a citrulline dehydrogenase of the present invention is provided. The citrulline measurement sensor using a citrulline dehydrogenase may have the same configuration as the basic configuration of the sensor 100 described above except that a citrulline dehydrogenase is used as an enzyme, and a detailed description thereof will be omitted.

[Sensor chip]

A sensor chip using a citrulline dehydrogenase may have the same configuration as the basic configuration of the sensor chip 10 described above except that a citrulline dehydrogenase is used as an enzyme. A carbon electrode, a gold electrode, and a platinum electrode and the like are used as the electrode, and the citrulline dehydrogenase of the present invention can be applied or immobilized on this electrode. Examples of the immobilization method include a method using a crosslinking reagent, a method of encapsulating in a polymer matrix, a method of coating with a dialysis membrane, and the like, and may be immobilized in a polymer such as a photocrosslinkable polymer, a conductive polymer, or a redox polymer, or adsorbed and immobilized on an electrode, and these may be combined. Typically, the citrulline dehydrogenase of the present invention can be immobilized on a carbon electrode using glutaraldehyde and then treated with a reagent having an amine group to block the glutaraldehyde.

The citrulline dehydrogenase of the present invention can be applied to various electrochemical measurement methods by using a potentiostat, a galvanostat, or the like. Electrochemical measurement methods include various techniques such as amperometry, voltammetry, potentiometry, and coulometry. For example, a citrulline concentration in the sample can be calculated by measuring a current at which citrulline is reduced by an amperometry method by the control unit 110. The applied voltage may be, for example, -1000 mV to +1000 mV (vs. Ag/AgCl), depending on the condition and the setting of the device.

Measurement of a citrulline concentration can be performed as follows. A buffer solution is put in a constant temperature cell and maintained at a constant temperature. An electrode immobilized with a citrulline dehydrogenase of the present invention is used as a working electrode, and a counter electrode (e.g., a platinum electrode) and a reference electrode (e.g., an Ag/AgCl electrode) are used. After the control unit 110 applies a constant voltage to the carbon electrode from the power supply 160 and the current becomes steady, a sample containing citrulline is added to measure the increase in the current. A citrulline concentration in the sample can be calculated according to the calibration curve made by the standard concentration of citrulline solution.

As a specific example, a 0.2 U to 150 U of the citrulline dehydrogenase of the present invention, more preferably a 0.5 U to 100 U of the citrulline dehydrogenase is immobilized on a glassy carbon (GC) electrode, and a response current value to the citrulline concentration is measured. 10.0 ml of 100 mM potassium phosphate buffer (pH 6.0) containing 300 mM of potassium ferricyanide is added into an electrolytic cell. The GC electrode is connected to a potentiostat BAS100B/W (manufactured by BAS), and the solution is stirred at 37°C, and +500 mV is applied to a silver chloride reference electrode. A 1 M citrulline solution is added to these systems to be a final concentration of 5, 10, 20, 30, 40, 50 mM, and a steady-state current value is measured for each addition. This current value is plotted against known citrulline concentrations (5, 10, 20, 30, 40, 50 mM) to generate a calibration curve. The citrulline concentration can be obtained from the calibration curve by measuring the current value of an unknown citrulline. Thus, quantification of citrulline is enabled with an enzyme-immobilized electrode using the citrulline dehydrogenase of the present invention.

In addition, a printing electrode can be used for the electrochemical measurements. Thus, it is possible to reduce the amount of a solution required for measurement. In this case, the electrode is preferably formed on the base material 11 made of an insulating substrate. More specifically, it is desirable that the electrode is formed on the base material 11 by a photolithography technique or a printing technique such as screen printing, gravure printing, or flexographic printing. Although silicon, glass, ceramic, polyvinyl chloride, polyethylene, polypropylene, and polyester can be exemplified as a material of the insulating substrate, more preferably, a material having strong resistance to various solvents and chemicals is preferably used.

As described above, the quantification method of citrulline according to the present invention, the citrulline dehydrogenase for quantification, the composition for quantification, and the kit for quantification can provide a new quantification method of citrulline concentration, an enzyme for quantification, a composition for quantification, and a kit for quantification, which quantify the citrulline concentration, which is a biomarker of diseases associated with abnormal citrullination of proteins, such as, multiple sclerosis, Alzheimer's disease, chronic rheumatoid arthritis, psoriasis, prion disease, liver fibrosis, chronic obstructive pulmonary disease, cancer, and the like by containing a citrulline dehydrogenase.

[Quantification method of citrulline using citrulline dehydrogenase reaction]

The citrulline dehydrogenase used in the present invention is a dehydrogenase that acts on a citrulline as a substrate. However, a citrulline dehydrogenase has not been identified by the time of filing the present application.

In one embodiment, the citrulline dehydrogenase may be selected from the citrulline oxidoreductase described above or a mutant having high citrulline dehydrogenase activity among the citrulline oxidoreductase described above. In one embodiment, the citrulline dehydrogenase is a citrulline dehydrogenase derived from Pseudomonas. With respect to the amino acid sequence described in SEQ ID NO: 1, a citrulline dehydrogenase having high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, e.g., 99% or more), and a citrulline dehydrogenase having an amino acid sequence in which one to several amino acids have been modified or mutated, or deleted, substituted, added, and/or inserted in the amino acid sequence of SEQ ID NO: 1 may be exemplified.

In one embodiment, although the citrulline dehydrogenase may be a citrulline dehydrogenase derived from a Pseudomonas sp. BYC41-1 strain, or a citrulline dehydrogenase produced by E. coli transformed with a plasmid containing a citrulline dehydrogenase gene derived from a Pseudomonas sp. BYC41-1 strain, the citrulline dehydrogenase can be efficiently expressed in large quantities by using E. coli transformed with a plasmid containing a citrulline dehydrogenase gene derived from a Pseudomonas sp. BYC41-1 strain.

In an example, although the citrulline dehydrogenase may be a citrulline dehydrogenase derived from a Pseudomonas japonica strain or a citrulline dehydrogenase produced by E. coli plasmid containing a citrulline dehydrogenase gene derived from a Pseudomonas japonica strain, the citrulline dehydrogenase can be efficiently expressed in large quantities by using E. coli transformed with a plasmid containing a citrulline dehydrogenase gene derived from a Pseudomonas japonica strain.

In one embodiment, although the citrulline dehydrogenase may be a citrulline dehydrogenase derived from a Pseudomonas sp. WCHPs060044 strain or a citrulline dehydrogenase produced by E. coli plasmid containing a citrulline dehydrogenase gene derived from a Pseudomonas sp. WCHPs060044 strain, the citrulline dehydrogenase can be efficiently expressed in large quantities by using E. coli transformed with a plasmid containing a citrulline dehydrogenase gene derived from a Pseudomonas sp. WCHPs060044 strain.

In one embodiment, although the citrulline dehydrogenase may be derived from computational science, for example, it may be the citrulline dehydrogenase shown in SEQ ID NO: 81, or a citrulline dehydrogenase produced by E. coli transformed with a plasmid comprising the citrulline dehydrogenase gene shown in SEQ ID NO: 82, the citrulline dehydrogenase can be efficiently expressed in large quantities by using E. coli transformed with a plasmid containing the citrulline dehydrogenase gene shown in SEQ ID NO: 82.

Suitable examples of microorganisms from which the citrulline dehydrogenase of the present invention is derived include microorganisms classified in the phylum Proteobacteria, preferably the class Gamma Proteobacteria, more preferably Pseudomonas or Oceanospirillales, and even more preferably Pseudomonas, Alteromonadaceae or Oceanospirillaceae. Specifically, citrulline dehydrogenases derived from Pseudomonas, Oceanobacter ,or Pseudoalteromonas are exemplified.

In one embodiment, the reaction condition of the citrulline dehydrogenase may be any condition as long as it is a condition for acting on citrulline and efficiently catalyzing an oxidation reaction. Generally, an enzyme has an optimum temperature and optimum pH that exhibit the highest activity. Therefore, it is suitable that the reaction condition is near the optimum temperature and the optimum pH.

In one embodiment, when quantification of citrulline using blood as an analyte is performed, the sample may be optionally selected from whole blood, plasma, or serum depending on the citrullinated protein to be measured. A citrulline dehydrogenase or a composition for quantification of citrulline containing a citrulline dehydrogenase may be mixed directly with a sample, and the sample may be pretreated before mixing with the citrulline dehydrogenase or the composition for quantification of citrulline containing the citrulline dehydrogenase. For example, the citrullinated protein may be degraded with a protease to liberate the citrulline and then mixed with the citrulline dehydrogenase or the composition for quantification of citrulline containing the citrulline dehydrogenase.

A range of quantification of citrulline is not particularly limited, and may be, for example, 0.001 mM to 1000 mM, 0.01 mM to 500 mM, 0.01 mM to 300 mM, 0.01 mM to 100 mM, 0.01 mM to 50 mM, 0.01 mM to 30 mM, 0.01 mM to 20 mM, and the like.

[Enzyme preparation method]

The citrulline dehydrogenase according to the present invention can be prepared by a preparation method similar to that of the citrulline oxidoreductase described above.

[Construction of expression plasmid]

For example, similar to the method for preparing the citrulline oxidoreductase described above, a plasmid for expression is constructed, or the DNA fragment encoding of the citrulline oxidase according to the present invention is chemically synthesized, and the DNA fragment is ligated into a vector to obtain a plasmid having a DNA fragment encoding of the citrulline dehydrogenase according to the present invention . A strain of E. coli or the like is transformed with the obtained plasmid to obtain a strain of E. coli or the like having DNA encoding of the citrulline dehydrogenase according to the present invention.

[Recombinant expression and purification of enzyme]

Expression and purification of a citrulline dehydrogenase may be performed by the same method as in the expression and purification of the oxidoreductase described above, and a detailed description thereof will be omitted.

[Enzyme activity measurement]

A method for measuring the activity of an enzyme may be any method as long as it directly or indirectly measures a product of a reaction catalyzed by an enzyme. For example, if a product by a reaction catalyzed by an enzyme and a reagent reacting with the product (hereinafter, a "product reaction reagent") are reacted and an absorbing substance generated by the reaction is measured, the enzyme activity can be measured by performing an absorbance measurement.

[PAD activity measurement]

It is possible to examine the application of the PAD activity measurement to early diagnosis in specific cases such as Alzheimer's disease and rheumatoid arthritis. To evaluate the therapeutic effect of these specific cases, it is possible to examine the application of the PAD activity measurement. Furthermore, it is possible to examine the application of the PAD activity measurement to the search for inhibitors of PAD typified by PAD2 and PAD4, and to the search for factors related to the expression control of PAD and inhibitors thereof.

At present, although it is possible to quantify the amount of PAD contained in blood by using an antibody, it is not sufficient as a method for evaluating PAD contributing to citrullination because the amount of PAD contained in blood is quantified by including the inactivated PAD. The activity of PAD can be assessed by reacting a peptide that can be a substrate for PAD (see, e.g., C. Assohou-Luty et. al., "The human peptidylarginine deiminases type 2 and type 4 have distinct substrate specificities," Biochi. Biophys. Acta 1844 (2014) 829-836) with PAD contained in blood for a period of time and quantifying the produced citrullinated peptide. In one embodiment, the activity of PAD can be assessed by quantifying the citrullinated peptide using the quantification method using the citrulline oxidoreductase, citrulline oxidase, or citrulline dehydrogenase according to the present invention described above. The sample for measuring the activity of PAD can be optionally selected from whole blood, plasma, or serum.

Specifically, a PAD-containing sample and an arginine peptide according to the type of PAD for which activity is to be assessed can be for a period of time, e.g., 5 seconds or more, 10 seconds or more, or 20 seconds or more, 180 minutes or less or 150 minutes or less, e.g., 0.5 to 120 minutes, preferably 0.5 to 60 minutes, more preferably 1 to 30 minutes. The working temperature of the PAD depends on the optimum temperature of the enzyme to be used, and is, for example, 20°C to 45°C, and the temperature used for a normal enzymatic reaction can be appropriately selected. Optionally, any means can be used to stop the reaction.

The citrullinated peptide in the reaction solution is quantified by a method described later to measure the citrullinated amount of per minute, and the number of micromoles in which arginine is converted into citrulline per minute can be defined as an active unit (U) in the enzyme solution and calculated. In one embodiment of the present invention as described above, although it is possible to calculate the activity of PAD, it is not possible to calculate the activity of PAD even though the amount of PAD can be measured by an antibody method which is a conventional method.

To quantify the citrullinated peptide, the citrullinated peptide is treated with a protease or peptidase. The reaction time of the citrullinated peptide with the protease or peptidase may be preferably within 1 day, more preferably within 14 hours, within 5 hours, within 1 hour, even more preferably within 30 minutes, within 10 minutes, within 5 minutes. The protease or peptidase is not particularly limited as long as it releases citrulline from the citrullinated peptide, and one type of protease or peptidase may act or a combination of a plurality of types of proteases and/or peptidases may act. The working temperature of the protease or peptidase depends on the optimum temperature of the enzyme to be used, and is, for example, 20°C to 95°C, and the temperature used for a normal enzymatic reaction can be appropriately selected. Optionally, any means can be used to stop the reaction.

The citrulline oxidoreductase, citrulline oxidase, or citrulline dehydrogenase according to the present invention is caused to act on the liberated citrulline, and the citrulline is quantified based on the quantification method of citrulline described above. Since the content of arginine residues contained in the peptide which can be a substrate of PAD used for measurement is known, the amount of the peptide citrullinated by PAD in the sample can be calculated. The activity of PAD in the sample can be assessed from the amount of the citrullinated peptide.

Although a high-performance chromatographic method, an ELISA method using an antibody, or the like can be used as a quantification method of a citrullinated peptide, in view of the long time required for measurement, it is clear that it is preferable to use an enzymatic method to which a quantification method of citrulline according to the present invention described above is applied. The enzymatic method is also preferred from the viewpoint of sensitivity of detection.

[Examples]

Specific examples and test results of the quantification method, the citrulline oxidoreductase for quantification, the composition for quantification, and the kit for quantification described above will be described in more detail.

[Preparation of recombinant plasmid pET-22b(+)-CitOX]

A citrulline oxidoreductase gene (hereinafter also referred to as CitOX, CitOX(WT)) derived from Pseudomonas sp. BYC41-1 strain having the base sequence of SEQ ID NO: 2 including the restriction enzyme sites NdeI and BamHI at both ends was synthesized entirely, and first, the CitOX(WT) gene was inserted between the restriction enzyme sites NdeI and BamHI of pET-22b(+), and this was used to transform E. coli JM109.

E. coli JM109 (pET-22b(+)-CitOX(WT) strain with a recombinant plasmid was inoculated into 2.5 ml of LB-amp medium [1% (W/V) bactotryptone, 0.5% (W/V) peptone, 0.5% (W/V) NaCl, 50 μg/ml Ampicillin], and cultured by shaking at 37°C for 24 hours to obtain a culture.

The culture was centrifuged at 7,000 rpm for 5 minutes to harvest and obtain bacterial cells. Then, a recombinant plasmid pET-22b(+)-CitOX(WT) was extracted from the bacterial cells using ISOSPIN Plasmid (produced by Nippon Gene Co., Ltd.) and purified to obtain 2.5 μg of DNA of the recombinant plasmid pET-22b(+)-CitOX(WT).

[Preparation of plasmid for mutant E62Q]

The fragment of the vector was prepared by PCR using pET-22b(+)-CitOX(WT) as a template and E62Q-Fw (SEQ ID NO: 4) and E62X-Rv (SEQ ID NO: 3) as the primers. Specifically, 5 μl of a 10 × KOD-Plus-buffer solution, 5μl of a dNTPs mixture solution prepared so that each dNTP is 2 mM, 2 μl of a 25 mM $MgSO_4$ solution, 50 ng of a DNA construct obtained by linking a CitOX(WT) gene serving as a template, 15 pmol of each of the above synthetic oligonucleotides, and 1 Unit of KOD-Plus- were added, and the total amount was set to 50 μl by sterilized water. The prepared reaction solution was incubated using a thermal cycler (manufactured by Eppendorf Co., Ltd.) for 2 minutes at 94°C, and 30 cycles of "94°C, 15 seconds"-"50°C, 30 seconds"-"68°C, 8 minutes".

A portion of the reaction solution was electrophoresed on a 1.0% agarose gel to confirm that approximately 8,000 bp of DNA was specifically amplified. The obtained DNA was treated with a restriction enzyme DpnI (produced by NEW ENGLAND BIOLABS Corporation), and the remaining template DNA was cut, and then the E. coli JM109 was transformed and developed on a LB-amp agar medium.

A plasmid (pET-22b(+)-CitOX(E62Q)) for expression of CitOX(E62Q) was obtained by culturing the E. coli JM109 in the same method as described above and extracting the recombinant plasmid.

[Preparation of plasmid for mutant D74N]

A plasmid (pET-22b(+)-CitOX(D74N)) for expression of CitOX(D74N) was obtained by the same method as in the preparation method of a plasmid for a mutant E62Q, except that D74N-Fw (SEQ ID NO: 6) and D74X-Rv (SEQ ID NO: 5) were used as the primers.

[Preparation of plasmid for mutant D74R]

A plasmid (pET-22b(+)-CitOX(D74R)) for expression of CitOX(D74R) was obtained by the same method as in the preparation method of a plasmid for a mutant E62Q, except that D74R-Fw (SEQ ID NO: 7) and D74X-Rv (SEQ ID NO : 5) were used as the primers.

[Preparation of plasmid for mutant E92Q]

A plasmid (pET-22b(+)-CitOX(E92Q)) for expression of CitOX(E92Q) was obtained by the same method as in the preparation method of a plasmid for a mutant E62Q, except that E92Q-Fw (SEQ ID NO: 9) and E92X-Rv (SEQ ID NO: 8) were used as the primers.

[Preparation of plasmid for mutant E92R]

A plasmid (pET-22b(+)-CitOX(E92R)) for expression of CitOX(E92R) was obtained by the same method as in the preparation method of a plasmid for a mutant E62Q, except that E92R-Fw (SEQ ID NO: 10) and E92X-Rv (SEQ ID NO: 8) were used as the primers.

[Preparation of plasmid for mutant E208R]

A plasmid (pET-22b(+)-CitOX(E208R)) for expression of CitOX(E208R) was obtained by the same method as in the preparation method of a plasmid for a mutant E62Q, except that E208R-Fw (SEQ ID NO: 12) and E208X-Rv (SEQ ID NO: 11) were used as the primers.

[Preparation of plasmid for mutant E224Q]

A plasmid (pET-22b(+)-CitOX(E224Q)) for expression of CitOX(E224Q) was obtained by the same method as in the preparation method of a plasmid for a mutant E62Q, except that E224Q-Fw (SEQ ID NO: 14) and E224X-Rv (SEQ ID NO: 13) were used as the primers.

[Preparation of plasmid for mutant E224R]

A plasmid (pET-22b(+)-CitOX(E224R)) for expression of CitOX(E224R) was obtained by the same method as in the preparation method of a plasmid for a mutant E62Q, except that E224R-Fw (SEQ ID NO: 15) and E224X-Rv (SEQ ID NO: 13) were used as the primers.

[Preparation of plasmid for mutant D402N]

A plasmid (pET-22b(+)-CitOX(D402N)) for expression of CitOX(D402N) was obtained by the same method as in the preparation method of a plasmid for a mutant E62Q, except that D402N-Fw (SEQ ID NO: 17) and D402X-Rv (SEQ ID NO: 16) were used as the primers.

[Preparation of plasmid for mutant D402Q]

A plasmid (pET-22b(+)-CitOX(D402Q)) for expression of CitOX(D402Q) was obtained by the same method as in the preparation method of a plasmid for a mutant E62Q, except that D402Q-Fw (SEQ ID NO: 18) and D402X-Rv (SEQ ID NO: 16) were used as the primers.

[Preparation of plasmid for mutant D402R]

A plasmid (pET-22b(+)-CitOX(D402R)) for expression of CitOX(D402R) was obtained by the same method as in the preparation method of a plasmid for a mutant E62Q, except that D402R-Fw (SEQ ID NO: 19) and D402X-Rv (SEQ ID NO: 16) were used as the primers.

[Preparation of plasmid for mutant E486M]

A plasmid (pET-22b(+)-CitOX(E486M)) for expression of CitOX(E486M) was obtained by the same method as in the preparation method of a plasmid for mutant E62Q, except that E486M-Fw (SEQ ID NO: 21) and E486X-Rv (SEQ ID NO: 20) were used as the primers.

[Preparation of plasmid for mutant E486Q]

A plasmid (pET-22b(+)-CitOX(E486Q)) for expression of CitOX(E486Q) was obtained by the same method as in the preparation method of a plasmid for mutant E62Q, except that E486Q-Fw (SEQ ID NO: 22) and E486X-Rv (SEQ ID NO: 20) were used as the primers.

[Preparation of plasmids for mutant E486H]

A plasmid (pET-22b(+)-CitOX(E486H)) for expression of CitOX(E486H) was obtained by the same method as in the preparation method of a plasmid for mutant E62Q, except that E486H-Fw (SEQ ID NO: 23) and E486X-Rv (SEQ ID NO: 20) were used as the primers.

[Preparation of plasmid for mutant D402Q/E486Q]

A plasmid (pET-22b(+)-CitOX(D402Q/E486Q)) for expression of a double mutant, CitOX(D402Q/E486Q) was obtained by the same method as in the preparation method of a plasmid for a mutant E62Q, except that D402Q-Fw (SEQ ID NO: 18) and D402X-Rv (SEQ ID NO: 16) were used as the primers, and pET-22b(+)-CitOX(E486Q) was used as the template.

[Preparation of plasmid for mutant D402R/E486Q]

A plasmid (pET-22b(+)-CitOX(D402R/E486Q)) for expression of a double mutant, CitOX(D402R/E486Q) was obtained by the same method as in the preparation method of a plasmid for a mutant E62Q, except that D402R-Fw (SEQ ID NO: 19) and D402X-Rv (SEQ ID NO: 16) were used as the primers and pET-22b(+)-CitOX(E486Q) was used as the template.

[Preparation of plasmid for mutant D402H]

A plasmid (pET-22b(+)-CitOX(D402H)) for expression of CitOX(D402H) was obtained by the same method as in the preparation method of a plasmid for a mutant E62Q, except that D402H-Fw (SEQ ID NO: 24) and D402X-Rv (SEQ ID NO: 16) were used as the primers.

[Preparation of plasmid for mutant D476R]

A plasmid (pET-22b(+)-CitOX(D476R)) for expression of CitOX(D476R) was obtained by the same method as in the preparation method of a plasmid for a mutant E62Q, except that D476R-Fw (SEQ ID NO: 26) and D476X-Rv (SEQ ID NO: 25) were used as the primers.

[Preparation of plasmids for mutant E514Q]

A plasmid (pET-22b(+)-CitOX(E514Q)) for expression of CitOX(E514Q) was obtained by the same method as in the preparation method of a plasmid for a mutant E62Q, except that E514Q-Fw (SEQ ID NO: 28) and E514X-Rv (SEQ ID NO: 27) were used as the primers.

[Preparation of plasmid for mutant E524Q]

A plasmid (pET-22b(+)-CitOX(E524Q)) for expression of CitOX(E524Q) was obtained by the same method as in the preparation method of a plasmid for a mutant E62Q, except that E524Q-Fw (SEQ ID NO: 30) and E524X-Rv (SEQ ID NO: 29) were used as the primers.

[Preparation of plasmid for mutant D74R/E486Q]

A plasmid (pET-22b(+)-CitOX(D74R/E486Q)) for expression of a double mutant, CitOX(D74R/E486Q) was obtained by the same method as in the preparation method of a plasmid for a mutant E62Q, except that D74R-Fw (SEQ ID NO: 7) and D74X-Rv (SEQ ID NO: 5) were used as the primers and pET-22b(+)-CitOX(E486Q) was used as the template.

[Preparation of recombinant plasmid pET-22b(+)-PjCitOX]

A citrulline oxidoreductase gene (hereinafter also referred to as PjCitOX, PjCitOX(WT)) derived from a Pseudomonas japonica strain having the base sequence of SEQ ID NO: 32 including the restriction enzyme sites Ndel and BamHI at both ends was synthesized entirely, and first, the PjCitOX(WT) gene was inserted between the restriction enzyme sites Ndel and BamHI of pET-22b(+), and this was used to transform E. coli JM109.

E. coli JM109 (pET-22b(+)-PjCitOX(WT) strain with a recombinant plasmid was inoculated into 2.5 ml of LB-amp medium [1% (W/V) bactotryptone, 0.5% (W/V) peptone, 0.5% (W/V) NaCl, 50 μg/ml Ampicillin], and cultured by shaking at 37°C for 24 hours to obtain a culture.

The culture was centrifuged at 7,000 rpm for 5 minutes to harvest and obtain bacterial cells. Then, a recombinant plasmid pET-22b(+)-PjCitOX(WT) was extracted from the bacterial cells using ISOSPIN Plasmid (manufactured by Nippon Gene Co., Ltd.) and purified to obtain 2.5 μg of DNA of the recombinant plasmid pET-22b(+)-PjCitOX(WT).

[Preparation of plasmid for mutant PjCitOX(D8Q)]

A plasmid (pET-22b(+)-PjCitOX(D8Q)) for expression of PjCitOX(D8Q) was obtained by the same method as in the preparation method of a plasmid for a mutant E62Q, except that pET-22b(+)-PjCitOX(WT) was used as the template of the fragment of the vector and D8Q-Fw (SEQ ID NO: 34) and D8X-Rv (SEQ ID NO: 33) were used as the primers.

[Preparation of plasmid for mutant PjCitOX(D8S)]

A plasmid (pET-22b(+)-PjCitOX(D8S))) for expression of PjCitOX(D8S) was obtained by the same method as in the preparation method of a plasmid for a mutant PjCitOX(D8Q), except that D8S-Fw (SEQ ID NO: 35) and D8X-Rv (SEQ ID NO: 33) were used as the primers.

[Preparation of plasmid for mutant PjCitOX(E39N)]

A plasmid (pET-22b(+)-PjCitOX(E39N)) for expression of PjCitOX(E39N) was obtained by the same method as in the preparation method of a plasmid for a mutant PjCitOX(D8Q), except that E39N-Fw (SEQ ID NO: 37) and E39X-Rv (SEQ ID NO: 36) were used as the primers.

[Preparation of plasmid for mutant PjCitOX(E39S)]

A plasmid (pET-22b(+)-PjCitOX(E39S)) for expression of PjCitOX(E39S) was obtained by the same method as in the preparation method of a plasmid for a mutant PjCitOX(D8Q), except that E39S-Fw (SEQ ID NO: 38) and E39X-Rv (SEQ ID NO: 36) were used as the primers.

[Preparation of plasmid for mutant PjCitOX(E62Q)]

A plasmid (pET-22b(+)-PjCitOX(E62Q)) for expression of PjCitOX(E62Q) was obtained by the same method as in the preparation method of a plasmid for a mutant PjCitOX(D8Q), except that E62Q-Fw (SEQ ID NO: 40) and E62X-Rv (SEQ ID NO: 39) were used as the primers.

[Preparation of plasmid for mutant PjCitOX(E148H)]

A plasmid (pET-22b(+)-PjCitOX(E148H)) for expression of PjCitOX(E148H) was obtained by the same method as in the preparation method of a plasmid for a mutant PjCitOX(D8Q), except that E148H-Fw (SEQ ID NO: 42) and E148X-Rv (SEQ ID NO: 41) were used as the primers.

[Preparation of plasmid for mutant PjCitOX(E148Q)]

A plasmid (pET-22b(+)-PjCitOX(E148Q)) for expression of PjCitOX(E148Q) was obtained by the same method as in the preparation method of a plasmid for a mutant PjCitOX(D8Q), except that E148Q-Fw (SEQ ID NO: 43) and E148X-Rv (SEQ ID NO: 41) were used as the primers.

[Preparation of plasmid for mutant PjCitOX(E148R)]

A plasmid (pET-22b(+)-PjCitOX(E148R)) for expression of PjCitOX(E148R) was obtained by the same method as in the preparation method of a plasmid for a mutant PjCitOX(D8Q), except that E148R-Fw (SEQ ID NO: 44) and E148X-Rv (SEQ ID NO: 41) were used as the primers.

[Preparation of plasmid for mutant PjCitOX(E148N)]

A plasmid (pET-22b(+)-PjCitOX(E148N)) for expression of PjCitOX(E148N) was obtained by the same method as in the preparation method of a plasmid for a mutant PjCitOX(D8Q), except that E148N-Fw (SEQ ID NO: 45) and E148X-Rv (SEQ ID NO: 41) were used as the primers.

[Preparation of plasmid for mutant PjCitOX(E148S)]

A plasmid (pET-22b(+)-PjCitOX(E148S)) for expression of PjCitOX(E148S) was obtained by the same method as in the preparation method of a plasmid for a mutant PjCitOX(D8Q), except that E148S-Fw (SEQ ID NO: 46) and E148X-Rv (SEQ ID NO: 41) were used as the primers.

[Preparation of plasmid for mutant PjCitOX(E353N)]

A plasmid (pET-22b(+)-PjCitOX(E353N)) for expression of PjCitOX(E353N) was obtained by the same method as in the preparation method of a plasmid for a mutant PjCitOX(D8Q), except that E353N-Fw (SEQ ID NO: 48) and E353X-Rv (SEQ ID NO: 47) were used as the primers.

[Preparation of plasmid for mutant PjCitOX(E353Q)]

A plasmid (pET-22b(+)-PjCitOX(E353Q)) for expression of PjCitOX(E353Q) was obtained by the same method as in the preparation method of a plasmid for a mutant PjCitOX(D8Q), except that E353Q-Fw (SEQ ID NO: 49) and E353X-Rv (SEQ ID NO: 47) were used as the primers.

[Preparation of plasmid for mutant PjCitOX(E382S)]

A plasmid (pET-22b(+)-PjCitOX(E382S)) for expression of PjCitOX(E382S) was obtained by the same method as in the preparation method of a plasmid for a mutant PjCitOX(D8Q), except that E382S-Fw (SEQ ID NO: 51) and E382X-Rv (SEQ ID NO: 50) were used as the primers.

[Preparation of plasmid for mutant PjCitOX(E382H)]

A plasmid (pET-22b(+)-PjCitOX(E382H)) for expression of PjCitOX(E382H) was obtained by the same method as in the preparation method of a plasmid for a mutant PjCitOX(D8Q), except that E382H-Fw (SEQ ID NO: 52) and E382X-Rv (SEQ ID NO: 50) were used as the primers.

[Preparation of plasmid for mutant PjCitOX(D402R)]

A plasmid (pET-22b(+)-PjCitOX(D402R)) for expression of PjCitOX(D402R) was obtained by the same method as in the preparation method of a plasmid for a mutant PjCitOX(D8Q), except that D402R-Fw (SEQ ID NO: 54) and D402X-Rv (SEQ ID NO: 53) were used as the primers.

[Preparation of plasmid for mutant PjCitOX(D402N)]

A plasmid (pET-22b(+)-PjCitOX(D402N)) for expression of PjCitOX(D402N) was obtained by the same method as in the preparation method of a plasmid for a mutant PjCitOX(D8Q), except that D402N-Fw (SEQ ID NO: 55) and D402X-Rv (SEQ ID NO: 53) were used as the primers.

[Preparation of plasmid for mutant PjCitOX(D402Q)]

A plasmid (pET-22b(+)-PjCitOX(D402Q)) for expression of PjCitOX(D402Q) was obtained by the same method as in the preparation method of a plasmid for a mutant PjCitOX(D8Q), except that D402Q-Fw (SEQ ID NO: 56) and D402X-Rv (SEQ ID NO: 53) were used as primers.

[Preparation of plasmid for mutant PjCitOX(E486Q)]

A plasmid (pET-22b(+)-PjCitOX(E486Q)) for expression of PjCitOX(E486Q) was obtained by the same method as in the preparation method of a plasmid for a mutant PjCitOX(D8Q), except that E486Q-Fw (SEQ ID NO: 58) and E486X-Rv (SEQ ID NO: 57) were used as the primers.

[Preparation of plasmid for mutant PjCitOX(E486H)]

A plasmid (pET-22b(+)-PjCitOX(E486H)) for expression of PjCitOX(E486H) was obtained by the same method as in the preparation method of a plasmid for mutant PjCitOX(D8Q), except that E486H-Fw (SEQ ID NO: 59) and E486X-Rv (SEQ ID NO: 57) were used as the primers.

[Preparation of plasmids for mutant PjCitOX(E148Q/E486Q)]

A plasmid (pET-22b(+)-PjCitOX(E148Q/E486Q)) for expression of PjCitOX(E148Q/E486Q), which is a double mutant, was obtained by the same method as in the preparation method of a plasmid for a mutant PjCitOX(D8Q), except that E148Q-Fw (SEQ ID NO: 43) and E148X-Rv (SEQ ID NO: 41) were used as the primers and pET-22b(+)-PjCitOX(E486Q) was used as the template.

[Preparation of recombinant plasmid pET-22b(+)-PWCitOX]

A citrulline oxidoreductase gene (hereinafter also referred to as PWCitOX, PWCitOX(WT)) derived from a Pseudomonas sp. WCHPs060044 strain having the base sequence of SEQ ID NO: 61 including the restriction enzyme sites NdeI and BamHI at both ends was synthesized entirely, and first, a PWCitOX(WT) gene was inserted between the restriction enzyme sites NdeI and BamHI of pET-22b(+), and this was used to transform E. coli JM109.

E. coli JM109 (pET-22b(+)-PWCitOX(WT) strain with a recombinant plasmid was inoculated into 2.5 ml of LB-amp medium [1% (W/V) bactotryptone, 0.5% (W/V) peptone, 0.5% (W/V) NaCl, 50 μg/ml Ampicillin], and cultured by shaking at 37°C for 24 hours to obtain a culture.

The culture was centrifuged at 7,000 rpm for 5 minutes to harvest and obtain bacterial cells. Then, a recombinant plasmid pET-22b(+)-PWCitOX(WT) was extracted from the bacterial cells using ISOSPIN Plasmid (manufactured by Nippon Gene Co., Ltd.) and purified to obtain 2.5 μg of recombinant plasmid pET-22b(+)-PWCitOX(WT).

[Preparation of plasmid for mutant PWCitOX(E39Q)]

A plasmid (pET-22b(+)-PWCitOX(E39Q)) for expression of PWCitOX(E39Q) was obtained by the same method as the preparation method of a plasmid for a mutant E62Q, except that pET-22b(+)-PWCitOX(WT) was used as the template of the fragment of the vector and E39Q-Fw (SEQ ID NO: 63) and E39X-Rv (SEQ ID NO: 62) were used as the primers.

[Preparation of plasmid for mutant PWCitOX(D74R)]

A plasmid (pET-22b(+)-PWCitOX(D74R)) for expression of PWCitOX(D74R) was obtained by the same method as in the preparation method of a plasmid for a mutant PWCitOX(E39Q) except that D74R-Fw (SEQ ID NO: 65) and D74X-Rv (SEQ ID NO: 64) were used as the primers.

[Preparation of plasmid for mutant PWCitOX(D74N)]

A plasmid (pET-22b(+)-PWCitOX(D74N)) for expression of PWCitOX(D74N) was obtained by the same method as in the preparation method of a plasmid for a mutant PWCitOX(E39Q) except that D74N-Fw (SEQ ID NO: 66) and D74X-Rv (SEQ ID NO: 64) were used as the primers.

[Preparation of plasmid for mutant PWCitOX(E224R)]

A plasmid (pET-22b(+)-PWCitOX(E224R) for expression of PWCitOX(E224R) was obtained by the same method as in the preparation method of a plasmid for a mutant PWCitOX(E39Q) except that E224R-Fw (SEQ ID NO: 68) and E224X-Rv (SEQ ID NO: 67) were used as the primers.

[Preparation of plasmid for mutant PWCitOX(E354Q)]

A plasmid (pET-22b(+)-PWCitOX(E354Q)) for expression of PWCitOX(E354Q) was obtained by the same method as in the preparation method of a plasmid for a mutant PWCitOX(E39Q) except that E354Q-Fw (SEQ ID NO: 70) and E354X-Rv (SEQ ID NO: 69) were used as the primers.

[Preparation of plasmid for mutant PWCitOX(E383Q)]

A plasmid (pET-22b(+)-PWCitOX(E383Q)) for expression of PWCitOX(E383Q) was obtained by the same method as in the preparation method of a plasmid for a mutant PWCitOX(E39Q) except that E383Q-Fw (SEQ ID NO: 72) and E383X-Rv (SEQ ID NO: 71) were used as the primers.

[Preparation of plasmid for mutant PWCitOX(D403R)]

A plasmid (pET-22b(+)-PWCitOX(D403R)) for expression of PWCitOX(D403R) was obtained by the same method as in the preparation method of a plasmid for a mutant PWCitOX(E39Q) except that D403R-Fw (SEQ ID NO: 74) and D403X-Rv (SEQ ID NO: 73) were used as the primers.

[Preparation of plasmid for mutant PWCitOX(D403N)]

A plasmid (pET-22b(+)-PWCitOX(D403N)) for expression of PWCitOX(D403N) was obtained by the same method as in the preparation method of a plasmid for a mutant PWCitOX(E39Q) except that D403N-Fw (SEQ ID NO: 75) and D403X-Rv (SEQ ID NO: 73) were used as the primers.

[Preparation of plasmid for mutant PWCitOX(E487H)]

A plasmid (pET-22b(+)-PWCitOX(E487H)) for expression of PWCitOX(E487H) was obtained by the same method as in the preparation method of a plasmid for a mutant PWCitOX(E39Q) except that E487H-Fw (SEQ ID NO: 77) and E487X-Rv (SEQ ID NO: 76) were used as the primers.

[Preparation of plasmid for mutant PWCitOX(E487Q]

A plasmid (pET-22b(+)-PWCitOX(E487Q)) for expression of PWCitOX(E487Q) was obtained by the same method as in the preparation method of a plasmid for a mutant PWCitOX(E39Q) except that E487Q-Fw (SEQ ID NO: 78) and E487X-Rv (SEQ ID NO: 76) were used as the primers.

[Preparation of plasmid for mutant PWCitOX(E532Q)]

A plasmid (pET-22b(+)-PWCitOX(E532Q)) for expression of PWCitOX(E532Q) was obtained by the same method as in the preparation method of a plasmid for a mutant PWCitOX(E39Q) except that E532Q-Fw (SEQ ID NO: 80) and E532X-Fw (SEQ ID NO: 79) were used as the primers.

[Preparation of recombinant plasmid pET-22b(+)-Pn2CitOX]

The citrulline dehydrogenase gene described in SEQ ID NO: 82 which encodes AncARODn2 (amino acid sequence deduced on the basis of a Pseudomonas sp. TPU 7192 strain described in S. Nakano et. al., Appl. Environ. Microbiol. 2019 85(12) e00459-19) was used as a citrulline dehydrogenase gene derived from computational science. The citrulline oxidoreductase gene (hereinafter, also referred to as Pn2CitOX gene and Pn2CitOX(WT) gene) having the base sequence of SEQ ID NO: 82 including the restriction enzyme sites Ndel and BamHI at both ends was synthesized entirely, and first, a Pn2CitOX(WT) gene was inserted between the restriction enzyme sites Ndel and BamHI of pET-22b(+), and this was used to transform E. coli JM109.

E. coli JM109 (pET-22b(+)-Pn2CitOX(WT) strain with a recombinant plasmid was inoculated into 2.5 ml of LB-amp medium [1% (W/V) bactotryptone, 0.5% (W/V) peptone, 0.5% (W/V) NaCl, 50$\mu$g/ml Ampicillin], and cultured by shaking at 37°C for 24 hours to obtain a culture.

The culture was centrifuged at 7,000 rpm for 5 minutes to harvest and obtain bacterial cells. Then, a recombinant plasmid pET-22b(+)-Pn2CitOX(WT) was extracted from the bacterial cells using ISOSPIN Plasmid (manufactured by Nippon Gene Co., Ltd.) and purified to obtain 2.5 $\mu$g of DNA of the recombinant plasmid pET-22b(+)-Pn2CitOX(WT).

[Production of citrulline oxidoreductase]

The recombinant plasmid of each mutant obtained by the above procedures was used to transform the E. coli BL21(DE3) strain. Each E. coli BL21(DE3) strain was cultured in 2.5 ml ZYP-5052 medium (0.5% glycerol, 0.05% glucose, 0.2% lactose, 50mM $(NH_4)_2SO_4$, 50 mM $KH_2PO_4$, 50 mM $Na_2HPO_4$, 1 mM $MgSO_4$) at 30°C for 24 hours.

3 ml of the culture solution was collected and centrifuged at 12,000 rpm for 5 minutes to harvest and obtain bacterial cells. Each bacterial cell was washed with 0.01 M potassium phosphate buffer solution at pH 7. 0, ultrasonically destroyed, and centrifuged at 15,000 rpm for 10 minutes to obtain a crude enzyme solution containing a citrulline oxidoreductase having the amino acid sequence of each mutant.

Similarly, a strain of E. coli BL21(DE3) transformed with the pET-22b(+) vector only was cultured and subjected to ultrasonic crushing treatment to prepare 1.5ml of a crude enzyme solution.

[Confirmation of expression of citrulline oxidoreductase]

The expression level of a citrulline oxidoreductase was confirmed by polyacrylamide gel electrophoresis (SDS-PAGE). 10% to 20% gradient polyacrylamide gel, and CLEARLY Stained Protein Ladder (produced by Takara Bio Inc.) as a marker were used, and the amounts of the citrulline oxidoreductase contained in each crude enzyme solution were confirmed. The results showed no significant difference in expression levels between the citrulline oxidoreductase (WT) and almost all mutants. In addition, in the citrulline oxidoreductase (WT) and all mutants, the activity to citrulline was confirmed when the measurement was carried out according to the measurement method shown below.

[Citrulline oxidase activity measurement]

The citrulline oxidase activity was measured for the crude enzyme solution obtained by the method described above. After 725 µl of reagent with the composition shown in Table 1 was incubated at 37°C for 5 minutes, 25µl of crude enzyme solution was added and mixed, and $A_{555}$ amount of change ($\Delta A_S$) per minute at 37°C was measured using a spectrophotometer (U-3900, manufactured by Hitachi High-Tech Science Corporation).

Next, 25 µl of potassium phosphate buffer solution of pH 7. 0 containing 0.1% BSA was added instead of the crude enzyme solution and mixed, and $A_{555}$ amount of change ($\Delta A_0$) per minute at 37°C was measured. 4-aminoantipyrine (4-AA) produced by FUJIFILM Wako Pure Chemical Corporation, citrulline produced by Sigma-Aldrich, N-ethyl-N-(2-hydroxy-3-sulfopropyl)-3-methylaniline (TOOS) produced by DOJINDO LABORATORIES, and horseradish peroxidase (POD) produced by TOYOBO CO., LTD. were used.

[Table 1]

| | |
|---|---|
| 0.74 mM 4-AA | |
| 7.5 U/ml POD | 450 µl |
| 150 mM potassium phosphate buffer solution pH 7. 0 | |
| 15 mM TOOS | 25 µl |
| $H_2O$ (ion-exchanged water) | 225 µl |
| 150 mM arginine or citrulline solution | 25 µl |
| | 725 µl |

The citrulline oxidase activity was calculated based on the following formula.

$$\text{Oxidase activity (U/ml)} = (\Delta A_S - \Delta A_0) \times 750 \times df / (39.2 \times 0.5 \times 25) = 1.53 \times (\Delta A_S - \Delta A_0) \times df$$

39.2: Millimolar extinction coefficient ($mM^{-1}cm^{-1}$) of 4-AA-TOOS condensation dyes for light with a wavelength of 555 nm
df: Dilution rate of crude enzyme solution

[Measurement results of citrulline oxidase activity]

The reactivity to arginine was also confirmed as a criterion for the reactivity improvement to citrulline. Table 2 shows the measurement results of citrulline oxidase activity of CitOX(WT), mutants E62Q, D74N, D74R, E92Q, E92R, E208N, E208R, E224Q, E224R, D402N, D402Q, D402R, E486M, E486Q, double mutant D402Q/E486Q, and double mutant D402R/E486Q.

[Table 2]

| Presence or absence of mutation | Cit/Arg (%) |
|---|---|
| WT | 0.36 |
| E62Q | 15.5 |
| D74N | 0.58 |

(continued)

| Presence or absence of mutation | Cit/Arg (%) |
|---|---|
| D74R | 6.83 |
| E92Q | 2.31 |
| E92R | 10.7 |
| E208R | 4.35 |
| E224Q | 0.68 |
| E224R | 0.95 |
| D402N | 4.92 |
| D402Q | 6.23 |
| D402R | 11.5 |
| E486M | 0.48 |
| E486Q | 1.25 |
| E486H | 21.1 |
| D402Q/E486Q | 11.7 |
| D402R/E486Q | 95.2 |

The results in Table 2 indicate that the percentage of reactivity to citrulline relative to reactivity to arginine is increased in all mutants, and the reactivity to citrulline is greatly improved. Further, it is possible to infer the content of arginine and citrulline contained in the sample by calculating from a reaction ratio of arginine and citrulline. When the activity was measured in the same manner using the ultrasonic crushed supernatant solution obtained by culturing the E. coli BL21(DE3) strain transformed with the pET-22b(+) vector only, the activity against citrulline was not observed at all.

[Evaluation of pH dependence of citrulline oxidase activity]

To evaluate the pH-dependence of citrulline oxidase activity, the citrulline oxidase activity was measured using a potassium phosphate buffer solution of pH 6.0 instead of 150 mM potassium phosphate buffer solution pH 7.0 in the measurement method described above. Table 3 shows measurement results of citrulline oxidase activity of CitOX(WT), double mutant D402Q/E486Q, and double mutant D402R/E486Q treated with the buffer solution of pH 6.0.

[Table 3]

| Presence or absence of mutation | Cit/Arg (%) |
|---|---|
| WT | 0.17 |
| D402Q/E486Q | 13 |
| D402R/E486Q | 113 |

From the results shown in Table 3, it was shown that the reactivity to citrulline was improved when treated with a buffer solution of pH 6.0 than when treated with a buffer solution of pH 7.0.
For other citrulline oxidases prepared above, citrulline oxidase activity in pH 6.0 was measured by the same method as described above. The measurement results for each mutant are shown in Table 4.

[Table 4]

| Presence or absence of mutation | Cit/Arg (%) |
|---|---|
| D402H | 23.1 |
| D476R | 200 |
| E514Q | 16.7 |

(continued)

| Presence or absence of mutation | Cit/Arg (%) |
|---|---|
| E524Q | 0.78 |
| D74R/E486Q | 10.9 |
| D74R | 5.75 |
| E92R | 10.7 |

The measurement results of the citrulline oxidase activity for PjCitOX(WT) and the mutants thereof are shown in Table 5.

[Table 5]

| Presence or absence of mutation | Cit/Arg (%) |
|---|---|
| WT | 1.31 |
| D8Q | 1.96 |
| D8S | 2.37 |
| E39N | 7.55 |
| E39S | 1.96 |
| E62Q | 13.30 |
| E148H | 3.85 |
| E148Q | 2.78 |
| E148R | 2.74 |
| E148N | 2.34 |
| E148S | 1.57 |
| E353N | 1.90 |
| E353Q | 1.81 |
| E382S | 1.95 |
| E382H | 1.92 |
| D402R | 15.38 |
| D402N | 11.43 |
| D402Q | 2.60 |
| E486Q | 2.22 |
| E486H | 4.36 |
| E148Q/E486Q | 3.91 |

The measurement results of citrulline oxidase activity for PWCitOX(WT) and the mutants thereof are shown in Table 6. PWCitOX(E354Q), PWCitOX(E383Q), PWCitOX(E354Q), PWCitOX(D403R), PWCitOX(D403N), PWCitOX(E487H), and PWCitOX(E487Q) are mutants of amino acid residues of PWCitOX corresponding to amino acid residues in 353rd, 353rd, 402nd, and 486th of PjCitOX.

[Table 6]

| Presence or absence of mutation | Cit/Arg (%) |
|---|---|
| WT | 1.04 |
| E39Q | 1.68 |

(continued)

| Presence or absence of mutation | Cit/Arg (%) |
|---|---|
| D74R | 1.91 |
| D74N | 1.32 |
| E224R | 1.37 |
| E354Q | 1.21 |
| E383Q | 1.31 |
| D403R | 10.00 |
| D403N | 2.04 |
| E487H | 5.51 |
| E487Q | 2.00 |
| E532Q | 2.31 |

A measurement result of citrulline oxidase activity for Pn2CitOX(WTs) is shown in Table 7.

[Table 7]

| Presence or absence of mutation | Cit/Arg (%) |
|---|---|
| WT | 0.35 |

[Purification of citrulline oxidase]

Anion-exchange chromatography was performed on 1.5 ml of a Q-Sepharose resin equilibrated by 10 mM potassium phosphate buffer solution (pH 7.5) using a column. The above cell-free extract solution dialyzed with 10 mM potassium phosphate buffer solution (pH 7.5) was adsorbed. After washing the column with 7.5 ml of 10 mM potassium phosphate buffer solution, NaCl levels were gradually increased in a stepwise manner using 10 mM potassium phosphate buffer solution (pH 7.5) and 10 mM potassium phosphate buffer solution (pH 7.5) containing 1000 mM NaCl to elute the enzyme. Fractions for which activity was observed were collected.

Subsequently, gel-filtration chromatography with a liquid chromatography system (AKTA avant25, column: HiLoad 26/60 superdex200 equilibrated with 10 mM potassium phosphate buffer solution containing 150 mM NaCl) was performed. The fractions obtained above were injected and the enzymes were fractionated with 10 mM potassium phosphate buffer solution containing 150 mM NaCl. The activities of each of the resulting fractions were measured, and the fractions for which activity was observed were collected.

The degree of purification was confirmed by SDS-PAGE, and if a contaminant protein was observed, anion-exchange chromatography was performed again. Each fraction was confirmed by SDS-PAGE, and fractions that were single-band and found to be active were collected and concentrated by ultrafiltration. When Cit/Arg was measured using the obtained citrulline oxidase preparation, it was equivalent to the result obtained using the crude enzyme solution.

[Quantification of citrulline by citrulline oxidase]

Of the activity measurement methods described above, a solution containing each concentration of citrulline solution (5, 10, 20, or 50 mM) was prepared. Subsequently, 25 μl of a solution containing citrulline oxidase preparation was added and mixed, and $A_{555}$ change for 5 minutes at 37°C was measured using the spectrophotometer (U-3900). The correlation between the enzyme activity (U/ml) and the citrulline concentration was evaluated with the enzyme activity (U/ml) calculated from $A_{555}$ change as the vertical axis and the citrulline concentration as the horizontal axis.

Fig. 3 is a diagram showing the correlation of a citrulline concentration and enzyme activity (U/ml). Fig. 3 (a) shows a measurement result of CitOX(WT) treated with a buffer solution of pH 6.0, and Fig. 3 (b) shows a measurement result of CitOX(E486Q) treated with a buffer solution of pH 6.0. In CitOX(WT), since the coefficient of determination ($R^2$), which is an index of the correlation between the citrulline concentration and the enzyme activity (U/ml), was 1.00 in the range of 5 mM to 20 mM, the result showed that there was a correlation between the citrulline concentration (μM) and the enzyme activity (U/ml). Also, in CitOX mutant E486Q, since the coefficient of determination ($R^2$), which is an index of the correlation between the citrulline concentration and the enzyme activity (U/ml), was 0.93 in the range of 5 mM to 20 mM, the result showed that there was a correlation between the citrulline concentration (μM) and the enzyme activity (U/ml). Therefore, it has been shown that CitOX(WT) and CitOX(E486Q) can be used to quantify citrulline.

[Quantification of citrulline by citrulline dehydrogenase]

The citrulline dehydrogenase activity was evaluated for CitOX(WT) and CitOX mutant E486Q treated with a buffer solution of pH 6.0. The citrulline dehydrogenase activity was measured using 2,6-Dichlorophenolindophenol (DCIP). Specifically, the activity of citrulline dehydrogenase can be measured according to the following procedure. 2.05 ml of 100 mM phosphate buffer solution (pH 6.0), 0.6 ml of citrulline solution, and 0.15 ml of 2 mM DCIP solution, are mixed, and incubated at 37°C for 5 minutes. Then 0.1 ml of 15 mM PMS solution and 0.1 ml of enzyme sample solution are added to start the reaction. The absorbance at the start of the reaction and over time is measured, and the amount of decrease per minute (ΔA600) of the absorbance at 600 nm accompanying the progress of the enzymatic reaction is determined using the spectrophotometer (U-3900, manufactured by Hitachi High-Tech Science Corporation), and the citrulline dehydrogenase activity is calculated according to the following formula. In this case, for the citrulline dehydrogenase activity, the amount of enzyme that reduces 1 μmol of DCIP per minute in the presence of citrulline at a concentration of 20 mM at 37°C is defined as 1U. A citrulline solution (5, 10, or 20 mM) or ion-exchanged water was used as the substrate.

$$\text{Dehydrogenase activity (U/ml)}$$
$$= (\Delta A_S - \Delta A_0) \times 3.0 \times df / (14.18 \times 0.1 \times 1.0)$$
$$= 2.12 \times (\Delta A_S - \Delta A_0) \times df$$

14.18: Millimole absorption coefficient ($mM^{-1}cm^{-1}$) of DCIP dye for light with a wavelength of 600 nm
df: Dilution rate of enzyme solution

Fig. 4 is a diagram showing the correlation of a citrulline concentration and enzyme activity (U/ml) according to an example of the present invention. Fig. 4 (a) shows a measurement result of CitOX(WT) treated with a buffer solution of pH 6.0, and Fig. 4 (b) shows a measurement result of CitOX(E486Q) treated with a buffer solution of pH 6.0. In CitOX(WT), since the coefficient of determination ($R^2$), which is an index of the correlation between the citrulline concentration and the enzyme activity (U/ml), was 0.99 in the range of 5 mM to 20 mM, the result showed that there was a correlation between the citrulline concentration (mM) and the enzyme activity (U/ml). In CitOX(E486Q), since the coefficient of determination ($R^2$), which is an index of the correlation between the citrulline concentration and the enzyme activity (U/ml), was 0.98 in the range of 5 mM to 20 mM, the result showed that there was a correlation between the citrulline concentration (mM) and the enzyme activity (U/ml). Therefore, it was shown that citrulline can be quantified even by using the dehydrogenase reaction.

[Citrulline quantification by citrulline dehydrogenase in electrochemical measurement]

15 μl of 100 mM phosphate buffer solution (pH 6.0) containing 1M sodium chloride and 200 mM potassium ferricyanide, and 4 μl of CitOX(E486Q) solution were applied and mixed on SCREEN-PRINTED ELECTRODES (manufactured by DropSens, Product Number DRP-C110). Then it was connected to an ALS electrochemical analyzer 814D using a dedicated connector (DRP-CAC). Cyclic voltammetry measurements were performed at sweeping speeds at 20 mV/s in a range from 0 to +600 mV (Ag/AgCl). Subsequently, 1 μl of citrulline solution at each concentration was added, and the current value at +0.4 V was recorded and plotted for 0 to 26 mM citrulline addition. As a result, it was found that as the citrulline concentration increases, the current value also increases (see Fig. 5). Since the coefficient of determination ($R^2$), which is an index of the correlation between the citrulline concentration and the current value (μA) at +0.4 V, was 0.98 in the range of 0 mM to 26 mM, the result showed that there was a correlation between the citrulline concentration (mM) and the current value (μA) at +0.4 V. Therefore, it was shown that citrulline can be quantified even by the electrochemical measurement.

[Quantification of citrulline-containing peptide by citrulline dehydrogenase]

It was verified whether the citrulline-containing peptide could be quantified by citrulline dehydrogenase when all the peptides (FFDSHKWHRDFFYSD) that serve as the substrates of PAD4 were citrullinated by PAD4. Specifically, FFDSHKWH(Cit)DFFYSD (PEPTIDE INSTITUTE, INC.) having a final concentration of 6.7 mM as a citrulline-containing peptide, Pfu Aminopeptidase I (Takara Bio Inc.) as peptidase, and $CoCl_2$ having a final concentration of 20 μM were

mixed and reacted at 75°C for 14 hours. The solution after the peptidase reaction was used as a substrate solution, and CitOX(E62Q) treated with a buffer solution of pH 6.0 was used to evaluate the citrulline dehydrogenase activity. Specifically, the activity was measured according to the procedure of activity measurement of citrulline dehydrogenase described above. However, the wavelength of the measured absorbance was 520 nm, and the millimole absorption coefficient ($mM^{-1}cm^{-1}$) of DCIP for light with a wavelength of 520 nm was calculated as 6.8.

Fig. 6 is a diagram showing a relationship between a citrulline-containing peptide concentration and an amount of change in absorbance per minute according to an example of the present invention. Fig. 6 shows an activity measurement results of CitOX(E62Q) treated with the buffer solution of pH 6.0. In a range of 0.01 mM to 0.22 mM, since the coefficient of determination ($R^2$), which is an index of the correlation between the citrulline-containing peptide concentration and the amount of change in absorbance, was 1.00 in the range of 0.01 mM to 22 mM, the result showed that there was a correlation between the citrulline-containing peptide concentration (mM) and the amount of change in absorbance. Therefore, it was shown that a citrulline-containing peptide can be quantified by using the citrulline dehydrogenase according to the present invention.

[Assessment of PAD4 activity using precitrullinated peptide (arginine peptide) and citrulline dehydrogenase]

It was also clarified that the citrullination rate can be calculated by reacting PAD4 contained in blood with the peptide (FFDSHKWHRDFFYSD) and quantifying the produced FFDSHKWH(Cit)DFFYSD. Therefore, the activity of PAD4 in blood samples can also be evaluated.

As described above, a quantification method of citrulline according to the present invention in which citrulline oxidase or citrulline dehydrogenase is added to a sample containing citrulline, citrulline oxidase or citrulline dehydrogenase for quantification of citrulline added to a sample containing citrulline, a composition for quantification of citrulline containing citrulline oxidase or citrulline dehydrogenase added to a sample containing citrulline, and a kit for quantification of citrulline containing citrulline oxidase or citrulline dehydrogenase added to a sample containing citrulline can provide a new quantification method of citrulline concentration, an enzyme for quantification, a composition for quantification, and a kit for quantification, which quantify the citrulline concentration, which is a biomarker of diseases associated with abnormal citrullination of proteins, such as, multiple sclerosis, Alzheimer's disease, chronic rheumatoid arthritis, psoriasis, prion disease, liver fibrosis, chronic obstructive pulmonary disease, cancer. Further, it is possible to provide an activity evaluation method of PAD.

## Claims

1. A quantitation method of citrulline comprising: adding a citrulline oxidoreductase to a sample.

2. The quantitation method of citrulline according to claim 1, wherein

   the citrulline oxidoreductase is a citrulline oxidase, and
   a concentration of the citrulline is determined by quantifying hydrogen peroxide produced by addition of the citrulline oxidase.

3. The quantitation method of citrulline according to claim 1, wherein

   the citrulline oxidoreductase is a citrulline dehydrogenase, and
   a concentration of the citrulline is determined by reducing a mediator by addition of the citrulline dehydrogenase.

4. A composition for quantification of citrulline comprising:
   the citrulline oxidoreductase used in the quantitation method of citrulline according to any one of claims 1 to 3.

5. A kit for quantification of citrulline comprising:

   the citrulline oxidoreductase used in the quantitation method of citrulline according to any one of claims 1 to 3; and
   a reagent reacting with hydrogen peroxide.

**6.** A kit for quantification of citrulline comprising:

the citrulline oxidoreductase used in the quantitation method of citrulline according to any one of claims 1 to 3; and
a mediator reduced by addition of the citrulline dehydrogenase.

**7.** A sensor chip comprising:
the citrulline oxidoreductase used in the quantitation method of citrulline according to any one of claims 1 to 3.

**8.** A sensor comprising:
the sensor chip according to claim 7.

**9.** An activity evaluation method of peptidylarginine deiminase comprising: using the quantitation method of citrulline according to any one of claims 1 to 3.

**10.** An activity evaluation method of peptidylarginine deiminase comprising:

adding a peptide able to be a substrate of the peptidylarginine deiminase to a sample;
adding a protease or peptidase to the sample; and
adding a citrulline oxidoreductase to the sample.

FIG. 1

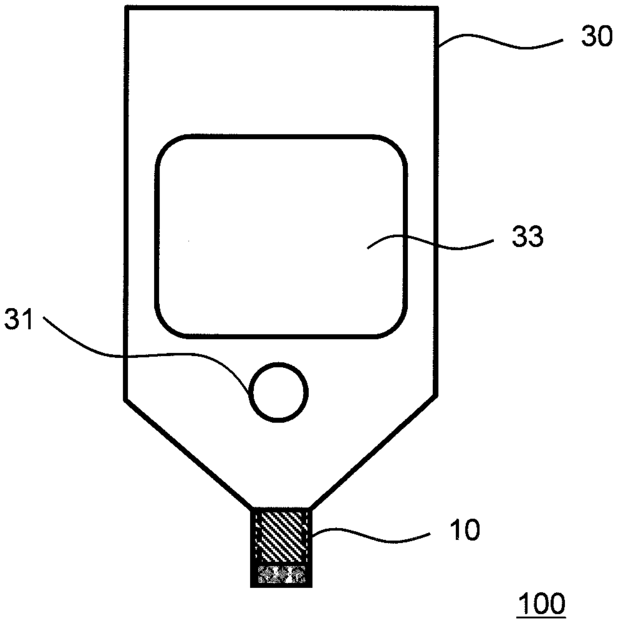
30
33
31
10
100

(a)

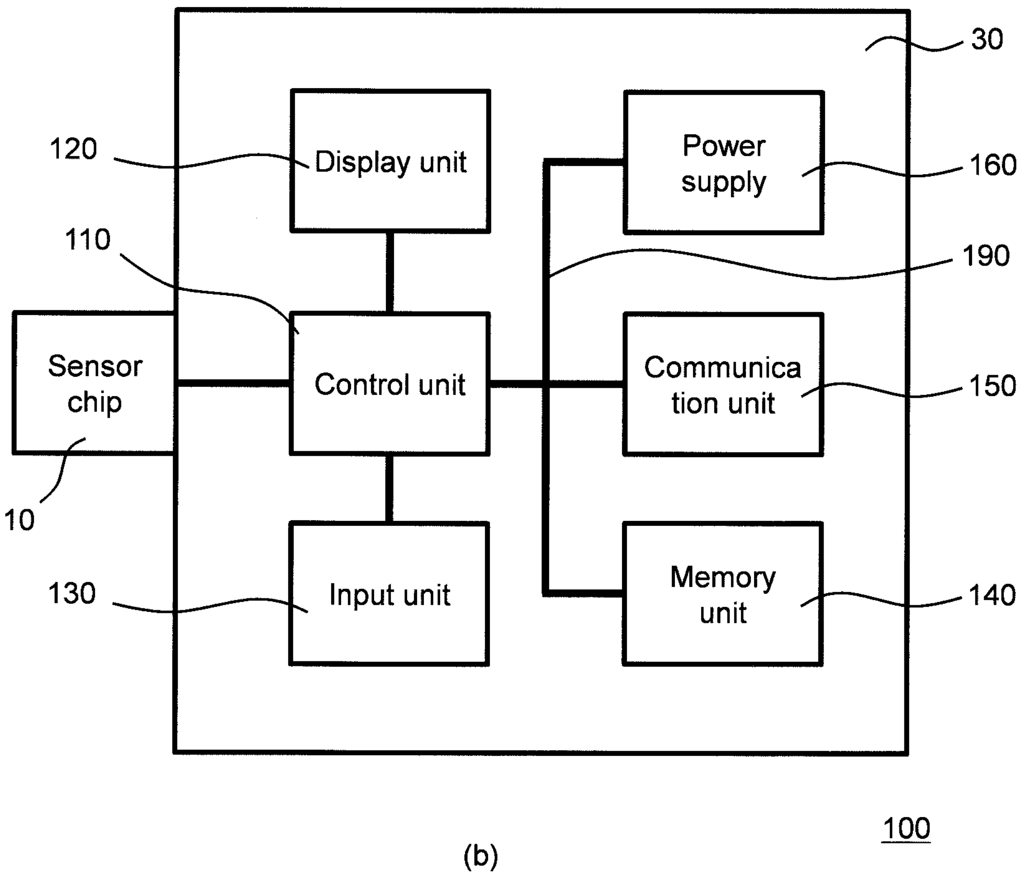
30
120
Display unit
Power supply
160
110
190
Sensor chip
Control unit
Communication unit
150
10
130
Input unit
Memory unit
140
100

(b)

FIG. 2

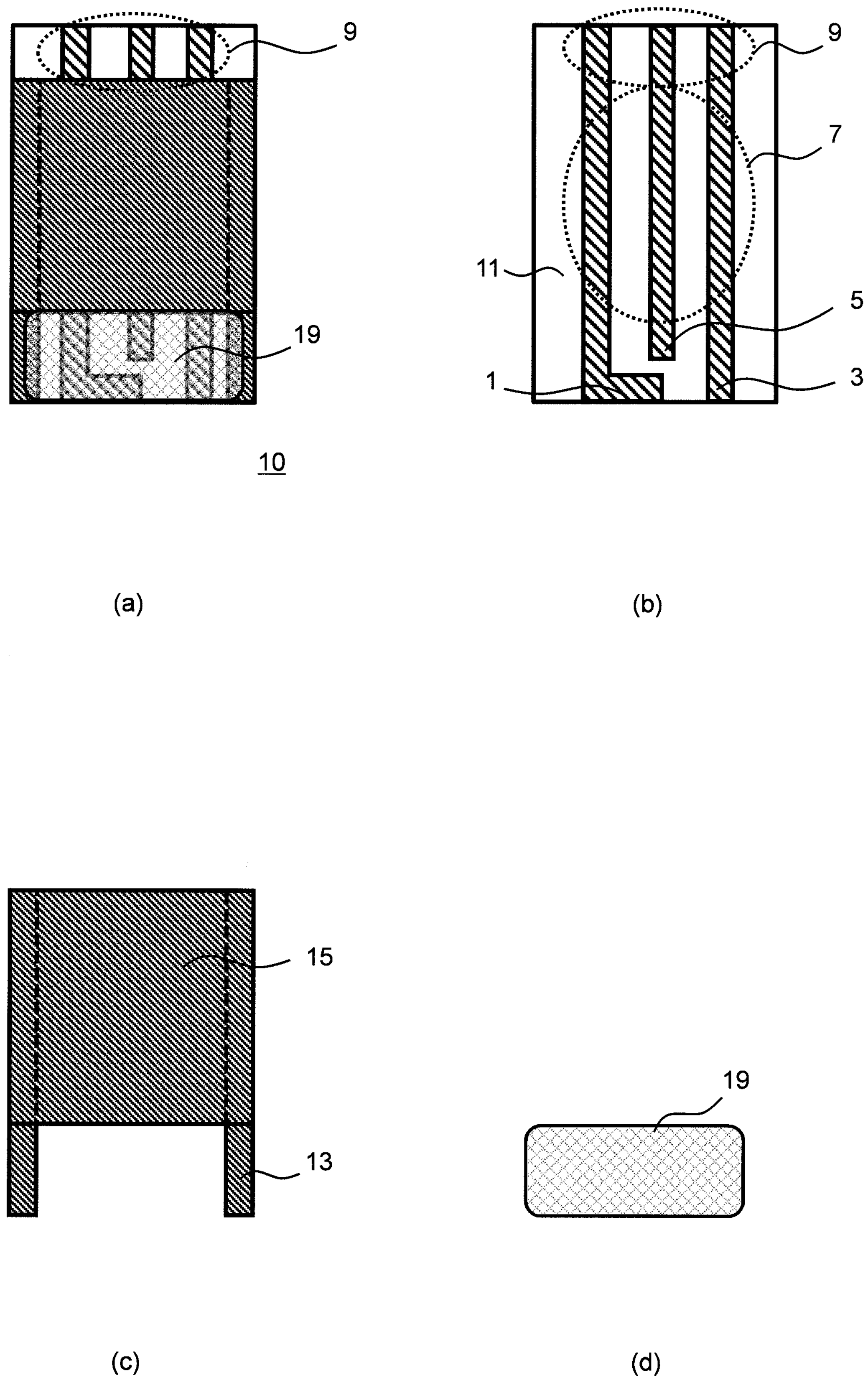

FIG. 3

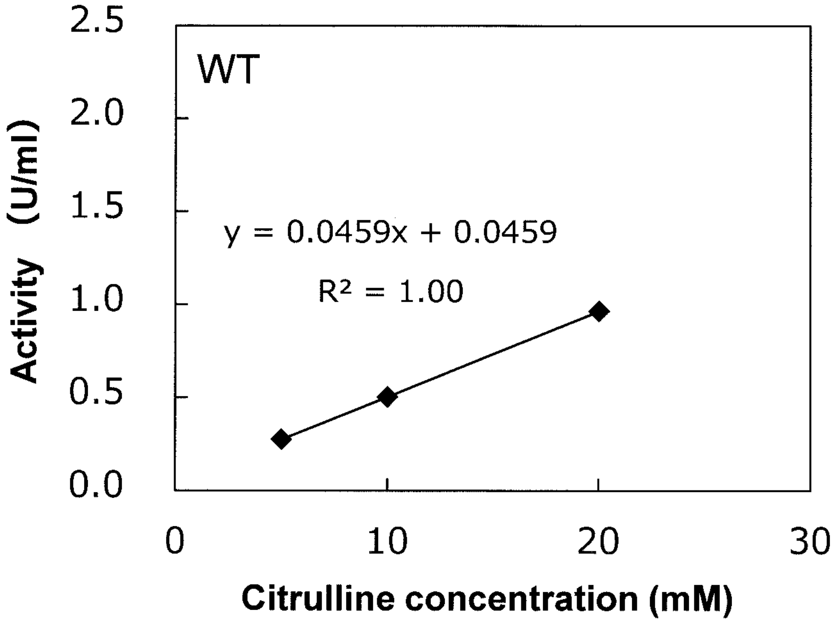
WT
y = 0.0459x + 0.0459
$R^2$ = 1.00
Activity (U/ml)
2.5
2.0
1.5
1.0
0.5
0.0
0
10
20
30
Citrulline concentration (mM)

(a)

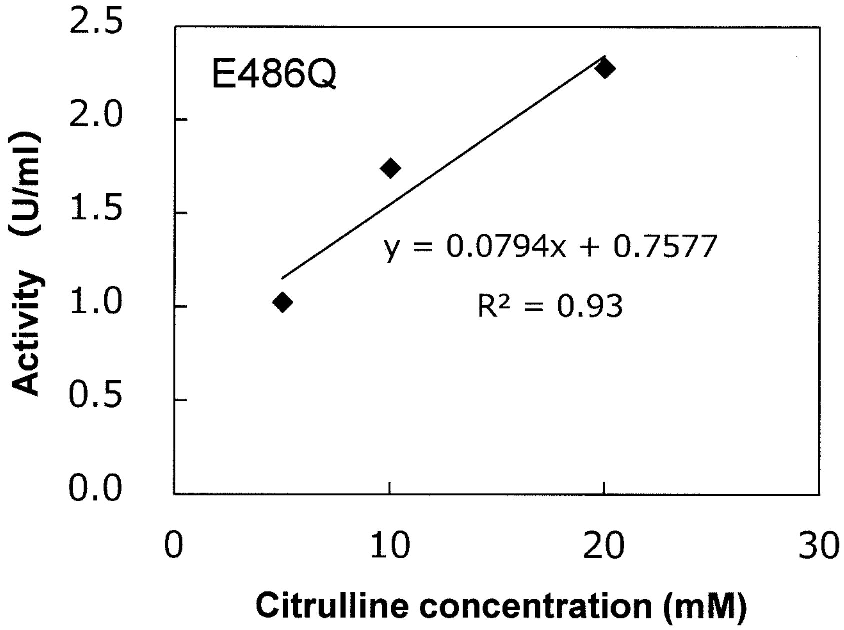
E486Q
y = 0.0794x + 0.7577
$R^2$ = 0.93
Activity (U/ml)
2.5
2.0
1.5
1.0
0.5
0.0
0
10
20
30
Citrulline concentration (mM)

(b)

FIG. 4

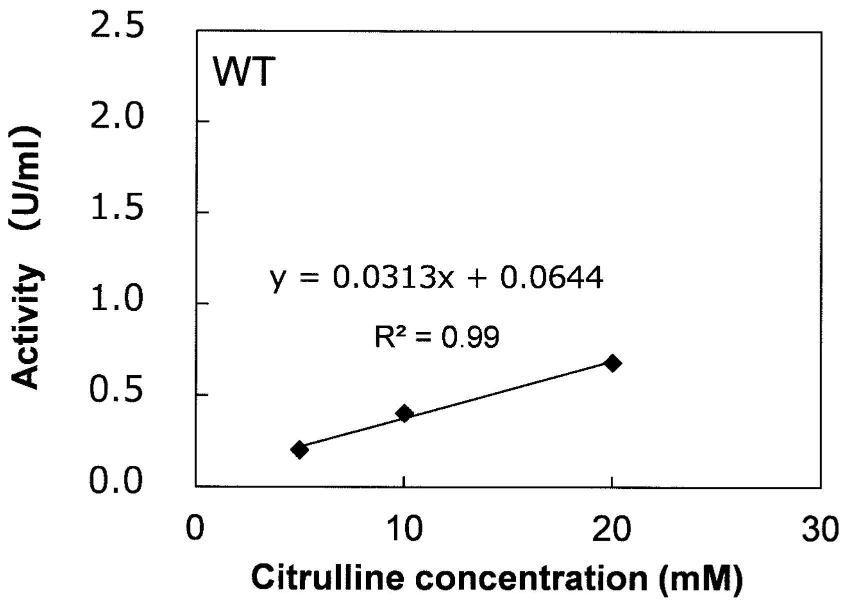
WT
y = 0.0313x + 0.0644
R² = 0.99
Activity (U/ml)
2.5
2.0
1.5
1.0
0.5
0.0
0
10
20
30
Citrulline concentration (mM)

(a)

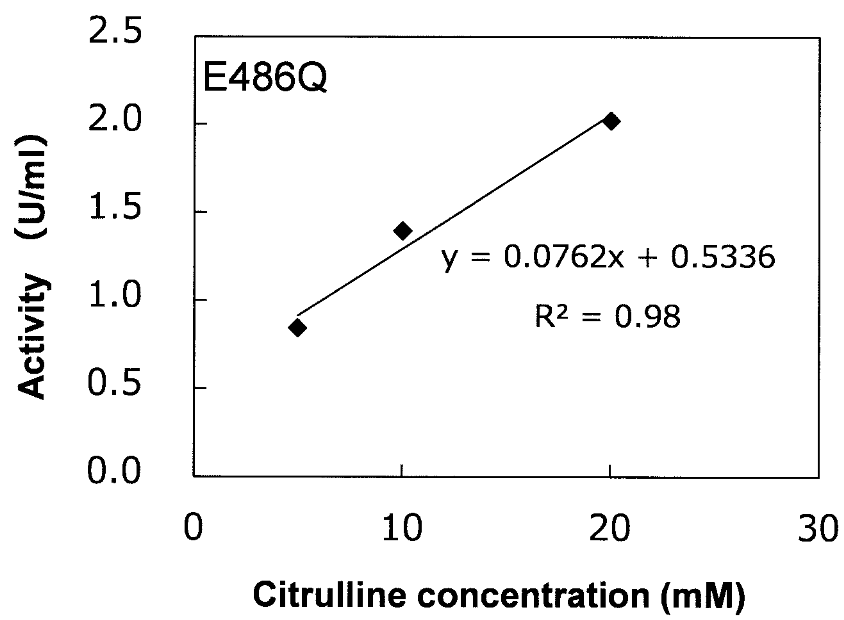
E486Q
y = 0.0762x + 0.5336
R² = 0.98
Activity (U/ml)
2.5
2.0
1.5
1.0
0.5
0.0
0
10
20
30
Citrulline concentration (mM)

(b)

FIG. 5

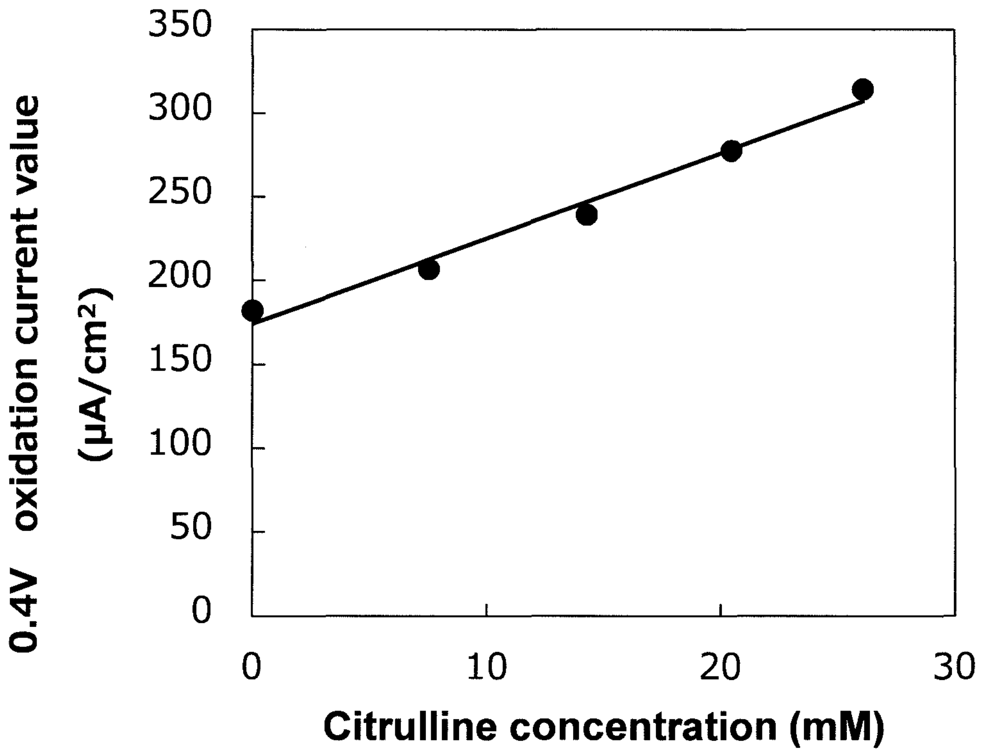
350
300
250
200
150
100
50
0
0.4V oxidation current value
(μA/cm²)
0
10
20
30
Citrulline concentration (mM)

FIG. 6

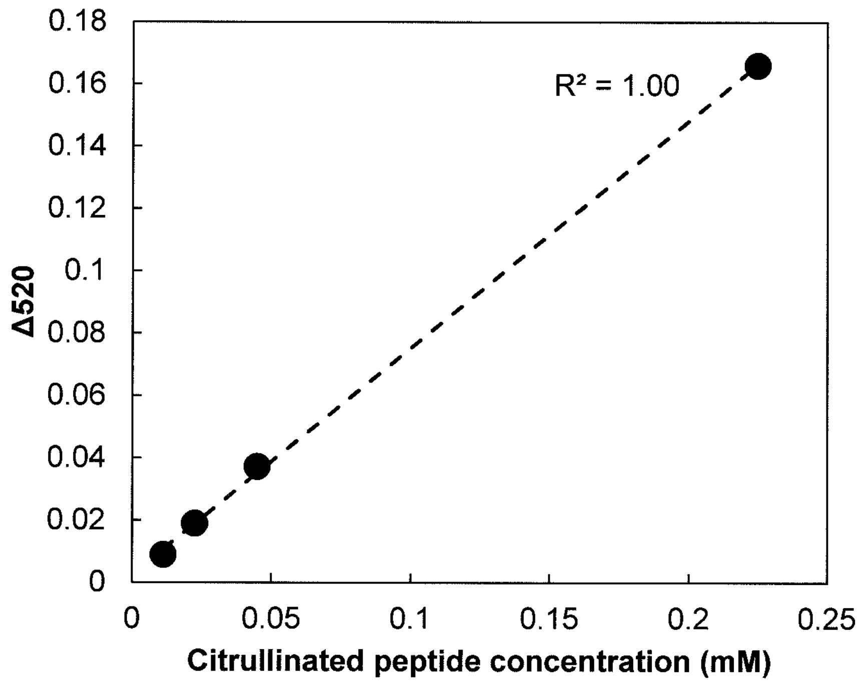
0.18
0.16
0.14
0.12
0.1
0.08
0.06
0.04
0.02
0
Δ520
R² = 1.00
0
0.05
0.1
0.15
0.2
0.25
Citrullinated peptide concentration (mM)

# FIG. 7

```
                           10        20        30        40        50        60        70
CitOX     1    MSQTQPLDVAIIGGVSGTYSAWRLQEAQGDHQRIQLFEYSDRIGGRLFSINLPGLPNVVAEVGGMRWMP   70
PjCitOX   1    MSQTQPLDVAIIGGVSGTYSAWRLQEAQGDRQRIQLFEYSDRIGGRLFSINLPGLPNVVAEVGGMRWMP   70
PWCitOX   1    MSQTQPLDVAIIGGVSGTYSAWRLQEAQGDHQRIQLFEYGDRIGGRLFSITLPGLPNVVAEVGGMRWMP   70
Pn2CitOX  1    --MPTELDIAIIGGVSGVYSAWRLQQHRGDEQRIALFEYSNRIGGRLYSRKLPGLPNVVAELGGMRYIP   68

                           80        90        100       110       120       130       140
CitOX     71   ATKDNTGGHVMVDKLVGELKLESKNFPMGSNLPD----KDP------VGAKDNLFYLRGERFRLRDFTEA  130
PjCitOX   71   ATDDGTGGHVMVDKLVGELKLPTKDFPMGSNLPE----KDP------VGAKDNLFYLRGERFRLRDFTEA  130
PWCitOX   71   ATEDDTGGHVMVDKLVDYLELPKKDFPMGNEQPE----GDP------VGAKNNLFYLRGQRFRFRDFTES  130
Pn2CitOX  69   ED------HLMVNSLVNELKLPTKDFPMGSSLPLDPKSKDPSPKDVKAGSENNLFYLRGQYFRYRDFAEC  132

                           150       160       170       180       190       200       210
CitOX     131  PDKIPYKLAWSERGYGPEDLQVKVMHNIYPGFDKLSLAEQMQVKVFGKEIWRYGFWDLLYRVLSNEGYQF  200
PjCitOX   131  PEKIPYKLAWSERGFGPEDLQVKVMNSIYPGFDQLSLAEQMQVKVFGKEIWRYGFWDLLYRVLSNEGYQF  200
PWCitOX   131  PDKIPYNLAWSERGFGPEDLQVKVMNSIYPGFDQLSLADQMQVKVFGKEIWRYGFWDLLYRVLSNEGYQF  200
Pn2CitOX  133  PDRIPYNLSWSERGYGPEDMQVKVMNLICPGFADMSLCEQMQVKVFGKEIWRYGFWNLLERVLSNEAYQF  202

                           220       230       240       250       260       270       280
CitOX     201  MKDAGGYEANVANASAVTQLPATEYSDKTVFLTLKKGFQALPLTLAKRFAEVPGGLIAGEQRIRMNRRLA  270
PjCitOX   201  MKDAGGYEANVANASAVTQLPATEYSDNTKFLTLKTGFQTLPLTLAKRFVDVAGGLVPAEQRVQMNRRLV  270
PWCitOX   201  MKDAGGYEANVANASAVTQLPATEYSDDTKFLTLKTGFQTLPLTLNERFKAVPGGLIPSDQRVQLNRRLA  270
Pn2CitOX  203  MKDAGGYEANVANANAVTQLPATEYSDDTEFLTLKDGFQALPLTLCEQFEELPG-------ALHMNQRLA  265

                           290       300       310       320       330       340       350
CitOX     271  SVQFSDDTEYPYRLHFQATRTVD-GKTSDVPGA-EEIIHARQVILALPRRSLELIQSPLFDDPWLKENID  338
PjCitOX   271  SVQFSDDTEYPYRLHFQATVTVD-GNTTDVAGP-EEIIHARQVILAMPRRSLELIQSPLFDDPWLKQNID  338
PWCitOX   271  SLQYSDDTEYPYRLHFQPTQTID-GTTTDIVGAPEVIVHARQVILAMPRRSLELIESPLFQDPWLKENLG  339
Pn2CitOX  266  EIRIDDDSDYRYTLIFQPTSTDDSGKTTDKDDA-AVTVRAKKVILAMPRRSLELIKSPFFDDPWLKENIP  334
```

# FIG. 8

```
                               360       370       380       390       400       410       420
CitOX      339  SVLVQSAFKLFLAYEQPWWRSQGLVAGRSVTDLPIRQCYYMGTECEQDGGEKTLNSLLMASYNDIGTVPF  408
PjCitOX    339  SVLVQSAFKLFLAYEQPWWRSQGLVAGRSVTDLPIRQCYYMGTECEQDGGEKTLNSLLMASYNDIGTVPF  408
PWCitOX    340  SVLVQSAFKLFLAYEQPWWRSLGLVAGRSVTDLPIRQCYYMGTECEQEGGQPSLNSLLMASYNDIGTVPF  409
Pn2CitOX   335  SVLIQKAFKMFMAYEQPWWRSLGLVAGRSVTDLPIRQTYYMGTECDQSGGEPTTNSLLMASYNDIGTVPY  404

                               430       440       450       460       470       480       490
CitOX      409  WKGLEDGAPFEGYQPKSLQGRIDANEVVPKMQYQISEEMVRIAQRQVTSLHDQIELPAPYSAVYHAWDAD  478
PjCitOX    409  WKGLEGGLPFGGYQPKSLQGRIDANEVVPRMQYQISDEMVQIAQRQVTSLHDQIELPAPYSAVYHAWDAD  478
PWCitOX    410  WKGLEGGLPFQGYQPKSLLGEVDANEIVPKTQNQVSEEMVRIAQRQVTSLHDQVELPAPYSAVYHAWDAD  479
Pn2CitOX   405  WKGLEAGEPFQGYTPASLSEGCAEEQIVPKHQFQITEDMVQAAHRQVEALHNQKQLPQPYSAVYQEWGDD  474

                               500       510       520       530       540       550       560
CitOX      479  PFGGGWHEWKANYRLDLIIQRMRHPVQEQEVYIVGEAYSYGQGWVEGALTTAESTLQDFFGLPRPAWLPE  548
PjCitOX    479  PFGGGWHEWKANYRLDLIIQRMRHPVQEQEVYIVGEAYSYGQGWVEGALTTAESTLQDFFGLPRPEWLPE  548
PWCitOX    480  PYGGGWHEWKANFRLDLIIQNMRHPVQEQSVYIVGEAYSYGQGWVEGALTTAESTLQDFFGLARPSWLPA  549
Pn2CitOX   475  PYGGGWHEWKANYRLDEIMCRMRHPVEDEEIYIVGEAYSYEQGWVEGALNTAESTLEEFFGLPTPSWLSK  544

                               570       580       590       600
CitOX      549  AYQLLPAPAP-VDIDNPPALACTDCKKTLTEVTEFAYTGIKA  589
PjCitOX    549  AYQLLPTPAP-IDIENPPSLACTDCKKTLADVTEFAYTGIKA  589
PWCitOX    550  AYQLLPEPGP-TDIADPAPLACKDCAGTLEAVTEFAYTGIKP  590
Pn2CitOX   545  DHNFLPVACNGTSSDNSATSACNASSETLNEVTEFAYEGINH  586
```

**INTERNATIONAL SEARCH REPORT**

International application No.
PCT/JP2020/035137

A. CLASSIFICATION OF SUBJECT MATTER
C12M 1/40(2006.01)i; C12N 15/52(2006.01)i; C12Q 1/26(2006.01)i; C12N 9/02(2006.01)i
FI: C12Q1/26 ZNA; C12N9/02; C12M1/40 B; C12N15/52 Z
According to International Patent Classification (IPC) or to both national classification and IPC

B. FIELDS SEARCHED
Minimum documentation searched (classification system followed by classification symbols)
C12M1/40; C12N15/52; C12Q1/26; C12N9/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
WPI; JSTPlus/JMEDPlus/JST7580 (JDreamIII); CAplus/MEDLINE/EMBASE/BIOSIS/WPIDS (STN); GenBank/EMBL/DDBJ/ GeneSeq; UniProt/GeneSeq

C. DOCUMENTS CONSIDERED TO BE RELEVANT

| Category* | Citation of document, with indication, where appropriate, of the relevant passages | Relevant to claim No. |
|---|---|---|
| Y | KOGA, R. et al., "Mouse D-Amino-Acid Oxidase: Distribution and Physiological Substrates", Frontiers in Molecular Biosciences, (2017), vol. 4, article 82, pp. 1-10 abstract | 1-10 |
| Y | JP 2013-162752 A (TOYAMA-KEN et al.) 22 August 2013 (2013-08-22) claims, paragraph [0003] | 1-10 |
| Y | JP 2018-524575 A (CEDARS-SINAI MEDICAL CENTER) 30 August 2018 (2018-08-30) claims, paragraph [0003] | 1-10 |
| Y | JP 2006-133177 A (INST OF PHYSICAL & CHEMICAL RES) 25 May 2006 (2006-05-25) claims, paragraphs [0001]-[0008] | 1-10 |
| A | WO 2014/129529 A1 (TOYAMA-KEN) 28 August 2014 (2014-08-28) claims, examples, SEQ ID NO: 2 | 1-10 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 November 2020 (09.11.2020) | 17 November 2020 (17.11.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/035137

| Patent Documents referred in the Report | Publication Date | Patent Family | Publication Date |
|---|---|---|---|
| JP 2013-162752 A | 22 Aug. 2013 | US 2014/0:357524 A1 claims, paragraph [0007] | |
| JP 2018-524575 A | 30 Aug. 2018 | US 2018/0299467 A1 claims, paragraph [0003] EP 3311176 A2 | |
| JP 2006-133177 A | 25 May 2006 | (Family: none) | |
| WO 2014/129529 A1 | 28 Aug. 2014 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5303715 B
- JP 2007222055 A


### Non-patent literature cited in the description


- **AKIHITO ISHIGAMI.** Citrullinated Molecule and Geriatric Disease. *Journal of the Japan geriatrics society, The Japan Geriatrics Society,* July 2014, vol. 51 (4), 314-320
- *Journal of the Japanese Society for Bacteriology,* 1963, vol. 18 (1
- **S. NAKANO.** *Appl. Environ. Microbiol.,* 2019, vol. 85 (12), e00459-19
- *CHEMISTRY TODAY,* June 1989, 24-30
- *Nucleic Acids Res.,* 1984, vol. 12, 9441
- *Methods Enzymol.,* 1987, vol. 154, 350
- *Gene,* 1985, vol. 37, 73
- *Nucleic Acids Res.,* 1985, vol. 13, 8749
- *Nucleic Acids Res.,* 1985, vol. 13, 8765
- *Nucleic Acids Res,* 1986, vol. 14, 9679
- *Proc. Natl. Acid. Sci. U.S.A.,* 1985, vol. 82, 488
- *Methods Enzymol.,* 1987, vol. 154, 367
- *Technique,* 1989, vol. 1, 11
- *Methods Mol. Cell. Biol.,* 1995, vol. 5, 255-269
- *J Microbiol Methods,* 2003, vol. 55, 481-484
- *Mol. Gen. Genet.,* 1989, vol. 218, 99-104
- **OZEKI et al.** *Biosci. Biotechnol. Biochem,* 1995, vol. 59, 1133
- *Mol. Gne. Genet.,* 1989, vol. 218, 99-104
- **COLE C et al.** The Jpred 3 secondary structure prediction server. *Nucleic Acids Res.,* 2008, W197-201
- **DROZDETSKIY A et al.** JPred4: a protein secondary structure prediction server. *Nucleic Acids Res.,* 2015
- **C. ASSOHOU-LUTY.** The human peptidylarginine deiminases type 2 and type 4 have distinct substrate specificities. *Biochi. Biophys. Acta,* 2014, vol. 1844, 829-836

SEQUENCE LISTING

<110> KIKKOMAN CORPORATION

<120> METHOD FOR QUANTITATIVE DETERMINATION OF CITRULLINE OXIREDUCTASE, CITRULLINE OXIREDUCTASE FOR QUANTITATIVE DETERMINATION, COMPOSITION FOR QUANTIFICATION, KIT FOR QUANTIFICATION, AND ACTIVITY EVALUATION METHOD OF PEPTIDYLARGININE DEIMINASE

<130> KM19I001

<160> 82

<170> PatentIn version 3.5

<210> 1
<211> 589
<212> PRT
<213> Pseudomonas sp.BYC41-1

<400> 1

```
Met Ser Gln Thr Gln Pro Leu Asp Val Ala Ile Ile Gly Gly Gly Val
1               5                   10                  15

Ser Gly Thr Tyr Ser Ala Trp Arg Leu Gln Glu Ala Gln Gly Asp His
            20                  25                  30

Gln Arg Ile Gln Leu Phe Glu Tyr Ser Asp Arg Ile Gly Gly Arg Leu
        35                  40                  45

Phe Ser Ile Asn Leu Pro Gly Leu Pro Asn Val Val Ala Glu Val Gly
    50                  55                  60

Gly Met Arg Trp Met Pro Ala Thr Lys Asp Asn Thr Gly Gly His Val
65                  70                  75                  80

Met Val Asp Lys Leu Val Gly Glu Leu Lys Leu Glu Ser Lys Asn Phe
                85                  90                  95

Pro Met Gly Ser Asn Leu Pro Asp Lys Asp Pro Val Gly Ala Lys Asp
            100                 105                 110

Asn Leu Phe Tyr Leu Arg Gly Glu Arg Phe Arg Leu Arg Asp Phe Thr
        115                 120                 125

Glu Ala Pro Asp Lys Ile Pro Tyr Lys Leu Ala Trp Ser Glu Arg Gly
    130                 135                 140

Tyr Gly Pro Glu Asp Leu Gln Val Lys Val Met His Asn Ile Tyr Pro
145                 150                 155                 160

Gly Phe Asp Lys Leu Ser Leu Ala Glu Gln Met Gln Val Lys Val Phe
```

165 170 175

Gly Lys Glu Ile Trp Arg Tyr Gly Phe Trp Asp Leu Leu Tyr Arg Val
180 185 190

Leu Ser Asn Glu Gly Tyr Gln Phe Met Lys Asp Ala Gly Gly Tyr Glu
195 200 205

Ala Asn Val Ala Asn Ala Ser Ala Val Thr Gln Leu Pro Ala Thr Glu
210 215 220

Tyr Ser Asp Lys Thr Val Phe Leu Thr Leu Lys Lys Gly Phe Gln Ala
225 230 235 240

Leu Pro Leu Thr Leu Ala Lys Arg Phe Ala Glu Val Pro Gly Gly Leu
245 250 255

Ile Ala Gly Glu Gln Arg Ile Arg Met Asn Arg Arg Leu Ala Ser Val
260 265 270

Gln Phe Ser Asp Asp Thr Glu Tyr Pro Tyr Arg Leu His Phe Gln Ala
275 280 285

Thr Arg Thr Val Asp Gly Lys Thr Ser Asp Val Pro Gly Ala Glu Glu
290 295 300

Ile Ile His Ala Arg Gln Val Ile Leu Ala Leu Pro Arg Arg Ser Leu
305 310 315 320

Glu Leu Ile Gln Ser Pro Leu Phe Asp Asp Pro Trp Leu Lys Glu Asn
325 330 335

Ile Asp Ser Val Leu Val Gln Ser Ala Phe Lys Leu Phe Leu Ala Tyr
340 345 350

Glu Gln Pro Trp Trp Arg Ser Gln Gly Leu Val Ala Gly Arg Ser Val
355 360 365

Thr Asp Leu Pro Ile Arg Gln Cys Tyr Tyr Met Gly Thr Glu Cys Glu
370 375 380

Gln Asp Gly Gly Glu Lys Thr Leu Asn Ser Leu Leu Met Ala Ser Tyr
385 390 395 400

Asn Asp Ile Gly Thr Val Pro Phe Trp Lys Gly Leu Glu Asp Gly Ala
405 410 415

```
Pro Phe Glu Gly Tyr Gln Pro Lys Ser Leu Gln Gly Arg Ile Asp Ala
            420                 425                 430

Asn Glu Val Val Pro Lys Met Gln Tyr Gln Ile Ser Glu Glu Met Val
        435                 440                 445

Arg Ile Ala Gln Arg Gln Val Thr Ser Leu His Asp Gln Ile Glu Leu
    450                 455                 460

Pro Ala Pro Tyr Ser Ala Val Tyr His Ala Trp Asp Ala Asp Pro Phe
465                 470                 475                 480

Gly Gly Gly Trp His Glu Trp Lys Ala Asn Tyr Arg Leu Asp Leu Ile
                485                 490                 495

Ile Gln Arg Met Arg His Pro Val Gln Glu Gln Glu Val Tyr Ile Val
            500                 505                 510

Gly Glu Ala Tyr Ser Tyr Gly Gln Gly Trp Val Glu Gly Ala Leu Thr
        515                 520                 525

Thr Ala Glu Ser Thr Leu Gln Asp Phe Phe Gly Leu Pro Arg Pro Ala
    530                 535                 540

Trp Leu Pro Glu Ala Tyr Gln Leu Leu Pro Ala Pro Ala Pro Val Asp
545                 550                 555                 560

Ile Asp Asn Pro Pro Ala Leu Ala Cys Thr Asp Cys Lys Lys Thr Leu
                565                 570                 575

Thr Glu Val Thr Glu Phe Ala Tyr Thr Gly Ile Lys Ala
            580                 585


<210>  2
<211>  1770
<212>  DNA
<213>  Pseudomonas sp.BYC41-1

<400>  2
atgtcccaaa cccaaccgct ggatgtcgca attatcggtg gtggtgtctc gggtacctac      60

agtgcctggc gtctgcaaga agcccaaggc gatcatcagc gtattcagct gtttgaatat     120

agcgatcgca ttggcggtcg tctgtttagc atcaacctgc cgggtctgcc gaatgtggtt     180

gcggaagtgg gcggtatgcg ttggatgccg gcaaccaaag ataacaccgg cggtcatgtg     240

atggttgata aactggtggg cgaactgaaa ctggaaagca aaaactttcc gatgggtagc     300

aatctgccgg ataaagatcc ggttggcgcg aaagataacc tgttttatct gcgtggcgaa     360

cgttttcgcc tgcgtgattt taccgaagcg ccggataaaa tcccgtataa actggcctgg     420
```

agcgaacgcg gttatggccc ggaagatctg caggtgaaag ttatgcataa catttatccg 480

ggctttgata aactgagcct ggcggaacag atgcaggtga aagtttttgg taaagaaatc 540

tggcgttatg gcttttggga tctgctgtat cgcgtgctga gcaatgaagg ttatcagttt 600

atgaaagatg ccggcggtta tgaagcgaac gtggcgaatg cgagcgccgt tacccagctg 660

ccggccaccg aatatagcga taaaaccgtt tttctgaccc tgaagaaagg cttccaggca 720

ctgccgctga ccctggcaaa acgttttgcg gaagtgccgg gcggtctgat tgcgggtgaa 780

cagcgcatcc gtatgaaccg tcgcctggcc agcgttcagt ttagcgatga taccgaatat 840

ccgtatcgcc tgcattttca ggcgacccgt accgtggatg gtaaaaccag cgatgttccg 900

ggcgccgaag aaattatcca tgcccgtcaa gtgattctgg cactgccgcg tcgcagcctg 960

gaactgatcc agagcccgct gtttgatgat ccgtggctga aagaaaatat tgatagcgtg 1020

ctggttcaga gcgcgtttaa actgtttctg gcctatgaac agccgtggtg gcgcagccag 1080

ggtctggtgg cgggccgtag cgttaccgat ctgccgatcc gccagtgcta ttatatgggc 1140

accgaatgtg aacaggatgg cggtgaaaaa accctgaaca gcctgctgat ggccagctat 1200

aatgatattg gtaccgtgcc gttttggaaa ggtctggaag atggcgcgcc gtttgaaggt 1260

tatcagccga aaagcctgca gggccgtatc gatgccaacg aagtggttcc gaaaatgcag 1320

tatcagatta gcgaagaaat ggtgcgcatc gcgcagcgtc aggttaccag cctgcatgat 1380

cagattgaac tgccggcccc gtatagcgcg gtttatcatg cctgggatgc ggatccgttt 1440

ggcggtggct ggcatgaatg gaaagcgaat tatcgtctgg atctgattat ccagcgcatg 1500

cgtcatccgg tgcaggaaca ggaagtgtat atcgttggcg aagcctatag ctatggtcag 1560

ggctgggttg aaggtgccct gaccaccgcg gaaagcaccc tgcaggattt ctttggtctg 1620

ccgcgtccgg catggctgcc ggaagcctat cagctgctgc cggcaccggc cccggtggat 1680

attgataacc cgccggccct ggcgtgcacg gactgcaaaa aaacgctgac ggaagttacg 1740

gaattcgcat acacgggcat caaagcctga 1770

<210> 3
<211> 30
<212> DNA
<213> Artificial Sequence

<220>
<223> Artificially Synthesized Primer Sequence E62X-Rv

<400> 3
ggttgccggc atccaacgca taccgcccac 30

<210> 4
<211> 30
<212> DNA

<213> Artificial Sequence

<220>
<223> Artificially Synthesized Primer Sequence E62Q-Fw

<400> 4
aatgtggttg cgcaggtggg cggtatgcgt 30

<210> 5
<211> 30
<212> DNA
<213> Artificial Sequence

<220>
<223> Artificially Synthesized Primer Sequence D74X-Rv

<400> 5
tttatcaacc atcacatgac cgccggtgtt 30

<210> 6
<211> 30
<212> DNA
<213> Artificial Sequence

<220>
<223> Artificially Synthesized Primer Sequence D74N-Fw

<400> 6
ccggcaacca aaaacaacac cggcggtcat 30

<210> 7
<211> 30
<212> DNA
<213> Artificial Sequence

<220>
<223> Artificially Synthesized Primer Sequence D74R-Fw

<400> 7
ccggcaacca aacgcaacac cggcggtcat 30

<210> 8
<211> 30
<212> DNA
<213> Artificial Sequence

<220>
<223> Artificially Synthesized Primer Sequence E92X-Rv

<400> 8
cagattgcta cccatcggaa agtttttgct 30

<210> 9
<211> 30
<212> DNA
<213> Artificial Sequence

<220>

<223> Artificially Synthesized Primer Sequence E92Q-Fw

<400> 9
gaactgaaac tgcagagcaa aaactttccg 30

<210> 10
<211> 30
<212> DNA
<213> Artificial Sequence

<220>
<223> Artificially Synthesized Primer Sequence E92R-Fw

<400> 10
gaactgaaac tgcgcagcaa aaactttccg 30

<210> 11
<211> 30
<212> DNA
<213> Artificial Sequence

<220>
<223> Artificially Synthesized Primer Sequence E208X-Rv

<400> 11
ggtaacggcg ctcgcattcg ccacgttcgc 30

<210> 12
<211> 30
<212> DNA
<213> Artificial Sequence

<220>
<223> Artificially Synthesized Primer Sequence E208R-Fw

<400> 12
gccggcggtt atcgcgcgaa cgtggcgaat 30

<210> 13
<211> 30
<212> DNA
<213> Artificial Sequence

<220>
<223> Artificially Synthesized Primer Sequence E224X-Rv

<400> 13
cagggtcaga aaaacggttt tatcgctata 30

<210> 14
<211> 30
<212> DNA
<213> Artificial Sequence

<220>
<223> Artificially Synthesized Primer Sequence E224Q-Fw

<400> 14

ctgccggcca cccagtatag cgataaaacc 30

<210> 15
<211> 30
<212> DNA
<213> Artificial Sequence

<220>
<223> Artificially Synthesized Primer Sequence E224R-Fw

<400> 15
ctgccggcca cccgctatag cgataaaacc 30

<210> 16
<211> 30
<212> DNA
<213> Artificial Sequence

<220>
<223> Artificially Synthesized Primer Sequence D402X-Rv

<400> 16
cagacctttc caaaacggca cggtaccaat 30

<210> 17
<211> 30
<212> DNA
<213> Artificial Sequence

<220>
<223> Artificially Synthesized Primer Sequence D402N-Fw

<400> 17
gccagctata ataatattgg taccgtgccg 30

<210> 18
<211> 30
<212> DNA
<213> Artificial Sequence

<220>
<223> Artificially Synthesized Primer Sequence D402Q-Fw

<400> 18
gccagctata atcagattgg taccgtgccg 30

<210> 19
<211> 30
<212> DNA
<213> Artificial Sequence

<220>
<223> Artificially Synthesized Primer Sequence D402R-Fw

<400> 19
gccagctata atcgtattgg taccgtgccg 30

<210> 20
<211> 30
<212> DNA
<213> Artificial Sequence

<220>
<223> Artificially Synthesized Primer Sequence E486X-Rv

<400> 20
aatcagatcc agacgataat tcgctttcca 30

<210> 21
<211> 29
<212> DNA
<213> Artificial Sequence

<220>
<223> Artificially Synthesized Primer Sequence E486M-Fw

<400> 21
ggtggctggc atatgtggaa agcgaatta 29

<210> 22
<211> 30
<212> DNA
<213> Artificial Sequence

<220>
<223> Artificially Synthesized Primer Sequence E486Q-Fw

<400> 22
ggtggctggc atcaatggaa agcgaattat 30

<210> 23
<211> 30
<212> DNA
<213> Artificial Sequence

<220>
<223> Artificially Synthesized Primer Sequence E486H-Fw

<400> 23
ggtggctggc atcattggaa agcgaattat 30

<210> 24
<211> 30
<212> DNA
<213> Artificial Sequence

<220>
<223> Artificially Synthesized Primer Sequence D402H-Fw

<400> 24
gccagctata atcatattgg taccgtgccg 30

<210> 25
<211> 30
<212> DNA

<213> Artificial Sequence

<220>
<223> Artificially Synthesized Primer Sequence D476X-Rv

<400> 25
ttcatgccag ccaccgccaa acggatccgc 30

<210> 26
<211> 30
<212> DNA
<213> Artificial Sequence

<220>
<223> Artificially Synthesized Primer Sequence D476R-Fw

<400> 26
tatcatgcct ggcgcgcgga tccgtttggc 30

<210> 27
<211> 30
<212> DNA
<213> Artificial Sequence

<220>
<223> Artificially Synthesized Primer Sequence E514X-Rv

<400> 27
ttcaacccag ccctgaccat agctataggc 30

<210> 28
<211> 30
<212> DNA
<213> Artificial Sequence

<220>
<223> Artificially Synthesized Primer Sequence E514Q-Fw

<400> 28
tatcgttgga caggcctata gctatggtca 30

<210> 29
<211> 30
<212> DNA
<213> Artificial Sequence

<220>
<223> Artificially Synthesized Primer Sequence E524X-Rv

<400> 29
cagggtgctt tccgcggtgg tcagggcacc 30

<210> 30
<211> 30
<212> DNA
<213> Artificial Sequence

<220>

<223>  Artificially Synthesized Primer Sequence E524Q-Fw

<400>  30
cagggctggg ttcagggtgc cctgaccacc                                        30

<210>  31
<211>  589
<212>  PRT
<213>  Pseudomonas japonica

<400>  31

```
Met Ser Gln Thr Gln Pro Leu Asp Val Ala Ile Ile Gly Gly Gly Val
1               5                   10                  15

Ser Gly Thr Tyr Ser Ala Trp Arg Leu Gln Glu Ala Gln Gly Asp Arg
            20                  25                  30

Gln Arg Ile Gln Leu Phe Glu Tyr Ser Asp Arg Ile Gly Gly Arg Leu
        35                  40                  45

Phe Ser Ile Asn Leu Pro Gly Leu Pro Asn Val Val Ala Glu Val Gly
    50                  55                  60

Gly Met Arg Trp Met Pro Ala Thr Asp Asp Gly Thr Gly Gly His Val
65                  70                  75                  80

Met Val Asp Lys Leu Val Gly Glu Leu Lys Leu Pro Thr Lys Asp Phe
                85                  90                  95

Pro Met Gly Ser Asn Leu Pro Glu Lys Asp Pro Val Gly Ala Lys Asp
            100                 105                 110

Asn Leu Phe Tyr Leu Arg Gly Glu Arg Phe Arg Leu Arg Asp Phe Thr
        115                 120                 125

Glu Ala Pro Glu Lys Ile Pro Tyr Lys Leu Ala Trp Ser Glu Arg Gly
    130                 135                 140

Phe Gly Pro Glu Asp Leu Gln Val Lys Val Met Asn Ser Ile Tyr Pro
145                 150                 155                 160

Gly Phe Asp Gln Leu Ser Leu Ala Glu Gln Met Gln Val Lys Val Phe
                165                 170                 175

Gly Lys Glu Ile Trp Arg Tyr Gly Phe Trp Asp Leu Leu Tyr Arg Val
            180                 185                 190

Leu Ser Asn Glu Gly Tyr Gln Phe Met Lys Asp Ala Gly Gly Tyr Glu
        195                 200                 205
```

Ala Asn Val Ala Asn Ala Ser Ala Val Thr Gln Leu Pro Ala Thr Glu
    210                 215                 220

Tyr Ser Asp Asn Thr Lys Phe Leu Thr Leu Lys Thr Gly Phe Gln Thr
225                 230                 235                 240

Leu Pro Leu Thr Leu Ala Lys Arg Phe Val Asp Val Ala Gly Gly Leu
                245                 250                 255

Val Pro Ala Glu Gln Arg Val Gln Met Asn Arg Arg Leu Val Ser Val
            260                 265                 270

Gln Phe Ser Asp Asp Thr Glu Tyr Pro Tyr Arg Leu His Phe Gln Ala
        275                 280                 285

Thr Val Thr Val Asp Gly Asn Thr Thr Asp Val Ala Gly Pro Glu Glu
    290                 295                 300

Ile Ile His Ala Arg Gln Val Ile Leu Ala Met Pro Arg Arg Ser Leu
305                 310                 315                 320

Glu Leu Ile Gln Ser Pro Leu Phe Asp Asp Pro Trp Leu Lys Gln Asn
                325                 330                 335

Ile Asp Ser Val Leu Val Gln Ser Ala Phe Lys Leu Phe Leu Ala Tyr
            340                 345                 350

Glu Gln Pro Trp Trp Arg Ser Gln Gly Leu Val Ala Gly Arg Ser Val
        355                 360                 365

Thr Asp Leu Pro Ile Arg Gln Cys Tyr Tyr Met Gly Thr Glu Cys Glu
    370                 375                 380

Gln Asp Gly Gly Glu Lys Thr Leu Asn Ser Leu Leu Met Ala Ser Tyr
385                 390                 395                 400

Asn Asp Ile Gly Thr Val Pro Phe Trp Lys Gly Leu Glu Gly Gly Leu
                405                 410                 415

Pro Phe Gly Gly Tyr Gln Pro Lys Ser Leu Gln Gly Arg Ile Asp Ala
            420                 425                 430

Asn Glu Val Val Pro Arg Met Gln Tyr Gln Ile Ser Asp Glu Met Val
        435                 440                 445

Gln Ile Ala Gln Arg Gln Val Thr Ser Leu His Asp Gln Ile Glu Leu

450 455 460

Pro Ala Pro Tyr Ser Ala Val Tyr His Ala Trp Asp Ala Asp Pro Phe
465 470 475 480

Gly Gly Gly Trp His Glu Trp Lys Ala Asn Tyr Arg Leu Asp Leu Ile
485 490 495

Ile Gln Arg Met Arg His Pro Val Gln Glu Gln Glu Val Tyr Ile Val
500 505 510

Gly Glu Ala Tyr Ser Tyr Gly Gln Gly Trp Val Glu Gly Ala Leu Thr
515 520 525

Thr Ala Glu Ser Thr Leu Gln Asp Phe Phe Gly Leu Pro Arg Pro Glu
530 535 540

Trp Leu Pro Glu Ala Tyr Gln Leu Leu Pro Thr Pro Ala Pro Ile Asp
545 550 555 560

Ile Glu Asn Pro Pro Ser Leu Ala Cys Thr Asp Cys Lys Lys Thr Leu
565 570 575

Ala Asp Val Thr Glu Phe Ala Tyr Thr Gly Ile Lys Ala
580 585

<210> 32
<211> 1767
<212> DNA
<213> Pseudomonas japonica

<400> 32
atgtctcaga ctcaaccctt agacgtggct atcattggcg gcggtgtgtc tggaacttac 60

agcgcatggc ggctgcagga ggcccaaggt gaccggcagc gtatacagct ttttgaatac 120

tctgatcgga ttggtggtcg cctgttttca atcaatttac cgggtttacc gaacgtcgtt 180

gcagaggtag gggcatgcg ttggatgcca gcaacggatg atggtacagg gggtcacgtc 240

atggttgata aactggtagg tgagttaaag ctgccgacga aagattttcc gatgggctca 300

aacctgcccg aaaaagaccc ggttggtgca aaagacaatt tattttactt acggggtgaa 360

cgtttccgtt tacgtgactt tacagaggcg ccagaaaaga taccgtacaa actggcatgg 420

agcgaacgcg gtttcggacc ggaggattta caggttaaag taatgaattc aatttaccct 480

ggctttgacc aactgtctct ggcagaacaa atgcaagtga aggtattcgg taaggaaatt 540

tggcgttatg gattttggga tctgctttac agagtcttaa gcaacgaagg ataccaattt 600

atgaaagacg ctggtggcta cgaagccaac gtggccaacg catcagcagt gactcagctg 660

cctgcgacag agtactctga caacacaaaa ttcctgacac tgaaaaccgg attccagacc 720

ctgccgctta cgctggcaaa acgtttcgtt gacgtggcag gtggtctggt tccggccgag 780

cagcgcgttc aaatgaaccg tcgccttgtg tcagtgcagt ttagtgacga cacagaatat 840

ccgtatcgtc tgcactttca agcgacggtt acggtagatg ggaataccac cgacgtagcc 900

gggccggagg aaatcatcca cgcacgccag gttattctgg caatgccccg tcgttccttg 960

gaattaatac agagcccgtt atttgatgat ccgtggctga agcaaaatat tgatagtgtg 1020

ctggttcagt cggccttcaa gttattcctt gcctatgagc aaccgtggtg gcgcagccag 1080

ggtcttgtag ccggccgcag cgtgacagat ttgcctattc gccagtgcta ttatatggga 1140

accgaatgtg agcaggatgg aggcgaaaaa acactgaata gccttctgat ggctagttat 1200

aatgatattg gaactgtgcc cttctggaag ggcctggagg gaggtctgcc ttttggcgga 1260

tatcagccaa aatcgctgca aggccgcatt gatgctaatg aagtcgttcc acgcatgcag 1320

tatcagatct ccgatgagat ggtccagatt gcccaaagac aagtcacctc gctgcatgat 1380

caaatcgaat tgcctgctcc ctatagtgct gtgtatcatg cctgggatgc cgatccattt 1440

ggcggcgggt ggcatgaatg gaaagcgaat tatcggttgg atcttattat tcagagaatg 1500

agacatccag ttcaggagca ggaagtctat atcgttgggg aggcgtatag ttatggccag 1560

ggctgggtcg aaggggctct gaccaccgct gaatccacct tgcaggattt ttttgggctt 1620

cctagacctg aatggcttcc tgaagcttat cagttgttgc caacccctgc gccaatcgat 1680

atagaaaatc ccccatcctt ggcgtgtacg gattgcaaaa aaactttggc ggatgtaact 1740

gaatttgcgt atacggggat aaaagcg 1767

<210> 33
<211> 30
<212> DNA
<213> Artificial Sequence

<220>
<223> Artificially Synthesized Primer Sequence D8X-Rv

<400> 33
tccagacaca ccgccgccaa tgatagccac 30

<210> 34
<211> 30
<212> DNA
<213> Artificial Sequence

<220>
<223> Artificially Synthesized Primer Sequence D8Q-Fw

<400> 34
actcaaccct tacaggtggc tatcattggc 30

<210> 35
<211> 30
<212> DNA
<213> Artificial Sequence

<220>
<223> Artificially Synthesized Primer Sequence D8S-Fw

<400> 35
actcaaccct taagcgtggc tatcattggc 30

<210> 36
<211> 30
<212> DNA
<213> Artificial Sequence

<220>
<223> Artificially Synthesized Primer Sequence E39X-Rv

<400> 36
aaacaggcga ccaccaatcc gatcagagta 30

<210> 37
<211> 30
<212> DNA
<213> Artificial Sequence

<220>
<223> Artificially Synthesized Primer Sequence E39N-Fw

<400> 37
atacagcttt ttaactactc tgatcggatt 30

<210> 38
<211> 30
<212> DNA
<213> Artificial Sequence

<220>
<223> Artificially Synthesized Primer Sequence E39S-Fw

<400> 38
atacagcttt ttagctactc tgatcggatt 30

<210> 39
<211> 30
<212> DNA
<213> Artificial Sequence

<220>
<223> Artificially Synthesized Primer Sequence E62X-Rv

<400> 39
cgttgctggc atccaacgca tgccccctac 30

<210> 40
<211> 30
<212> DNA

<213> Artificial Sequence

<220>
<223> Artificially Synthesized Primer Sequence E62Q-Fw

<400> 40
acgtcgttgc acaggtaggg ggcatgcgtt 30

<210> 41
<211> 30
<212> DNA
<213> Artificial Sequence

<220>
<223> Artificially Synthesized Primer Sequence E148X-Rv

<400> 41
aattgaattc attactttaa cctgtaaatc 30

<210> 42
<211> 30
<212> DNA
<213> Artificial Sequence

<220>
<223> Artificially Synthesized Primer Sequence E148H-Fw

<400> 42
ggtttcggac cgcatgattt acaggttaaa 30

<210> 43
<211> 30
<212> DNA
<213> Artificial Sequence

<220>
<223> Artificially Synthesized Primer Sequence E148Q-Fw

<400> 43
ggtttcggac cgcaggattt acaggttaaa 30

<210> 44
<211> 30
<212> DNA
<213> Artificial Sequence

<220>
<223> Artificially Synthesized Primer Sequence E148R-Fw

<400> 44
ggtttcggac cgcgcgattt acaggttaaa 30

<210> 45
<211> 30
<212> DNA
<213> Artificial Sequence

<220>

<223> Artificially Synthesized Primer Sequence E148N-Fw

<400> 45
ggtttcggac cgaacgattt acaggttaaa 30

<210> 46
<211> 30
<212> DNA
<213> Artificial Sequence

<220>
<223> Artificially Synthesized Primer Sequence E148S-Fw

<400> 46
ggtttcggac cgagcgattt acaggttaaa 30

<210> 47
<211> 30
<212> DNA
<213> Artificial Sequence

<220>
<223> Artificially Synthesized Primer Sequence E353X-Rv

<400> 47
tacaagaccc tggctgcgcc accacggttg 30

<210> 48
<211> 30
<212> DNA
<213> Artificial Sequence

<220>
<223> Artificially Synthesized Primer Sequence E353N-Fw

<400> 48
ttccttgcct ataaccaacc gtggtggcgc 30

<210> 49
<211> 30
<212> DNA
<213> Artificial Sequence

<220>
<223> Artificially Synthesized Primer Sequence E353Q-Fw

<400> 49
ttccttgcct atcagcaacc gtggtggcgc 30

<210> 50
<211> 30
<212> DNA
<213> Artificial Sequence

<220>
<223> Artificially Synthesized Primer Sequence E382X-Rv

<400> 50

cagtgttttt tcgcctccat cctgctcaca 30

<210> 51
<211> 30
<212> DNA
<213> Artificial Sequence

<220>
<223> Artificially Synthesized Primer Sequence E382S-Fw

<400> 51
tatatgggaa ccagctgtga gcaggatgga 30

<210> 52
<211> 30
<212> DNA
<213> Artificial Sequence

<220>
<223> Artificially Synthesized Primer Sequence E382H-Fw

<400> 52
tatatgggaa cccattgtga gcaggatgga 30

<210> 53
<211> 30
<212> DNA
<213> Artificial Sequence

<220>
<223> Artificially Synthesized Primer Sequence D402X-Rv

<400> 53
caggcccttc cagaagggca cagttccaat 30

<210> 54
<211> 30
<212> DNA
<213> Artificial Sequence

<220>
<223> Artificially Synthesized Primer Sequence D402R-Fw

<400> 54
gttataatcg cattggaact gtgcccttct 30

<210> 55
<211> 30
<212> DNA
<213> Artificial Sequence

<220>
<223> Artificially Synthesized Primer Sequence D402N-Fw

<400> 55
tataataaca ttggaactgt gcccttctgg 30

<210> 56
<211> 23
<212> DNA
<213> Artificial Sequence

<220>
<223> Artificially Synthesized Primer Sequence D402Q-Fw

<400> 56
gttataatca gattggaact gtg 23

<210> 57
<211> 30
<212> DNA
<213> Artificial Sequence

<220>
<223> Artificially Synthesized Primer Sequence E486X-Rv

<400> 57
aataagatcc aaccgataat tcgctttcca 30

<210> 58
<211> 30
<212> DNA
<213> Artificial Sequence

<220>
<223> Artificially Synthesized Primer Sequence E486Q-Fw

<400> 58
ggcgggtggc atcagtggaa agcgaattat 30

<210> 59
<211> 30
<212> DNA
<213> Artificial Sequence

<220>
<223> Artificially Synthesized Primer Sequence E486H-Fw

<400> 59
ggcgggtggc atcattggaa agcgaattat 30

<210> 60
<211> 590
<212> PRT
<213> Pseudomonas sp.WCHPs060044

<400> 60

Met Ser Gln Thr Gln Pro Leu Asp Val Ala Ile Ile Gly Gly Gly Val
1 5 10 15

Ser Gly Thr Tyr Ser Ala Trp Arg Leu Gln Glu Ala Gln Gly Asp His
20 25 30

```
Gln Arg Ile Gln Leu Phe Glu Tyr Gly Asp Arg Ile Gly Gly Arg Leu
        35                  40                  45

Phe Ser Ile Thr Leu Pro Gly Leu Pro Asn Val Val Ala Glu Val Gly
    50                  55                  60

Gly Met Arg Trp Met Pro Ala Thr Glu Asp Asp Thr Gly Gly His Val
65                  70                  75                  80

Met Val Asp Lys Leu Val Asp Tyr Leu Glu Leu Pro Lys Lys Asp Phe
                85                  90                  95

Pro Met Gly Asn Glu Gln Pro Glu Gly Asp Pro Val Gly Ala Lys Asn
            100                 105                 110

Asn Leu Phe Tyr Leu Arg Gly Gln Arg Phe Arg Phe Arg Asp Phe Thr
        115                 120                 125

Glu Ser Pro Asp Lys Ile Pro Tyr Asn Leu Ala Trp Ser Glu Arg Gly
    130                 135                 140

Phe Gly Pro Glu Asp Leu Gln Val Lys Val Met Asn Ser Ile Tyr Pro
145                 150                 155                 160

Gly Phe Asp Gln Leu Ser Leu Ala Asp Gln Met Gln Val Lys Val Phe
                165                 170                 175

Gly Lys Glu Ile Trp Arg Tyr Gly Phe Trp Asp Leu Leu Tyr Arg Val
            180                 185                 190

Leu Ser Asn Glu Gly Tyr Gln Phe Met Lys Asp Ala Gly Gly Tyr Glu
        195                 200                 205

Ala Asn Val Ala Asn Ala Ser Ala Val Thr Gln Leu Pro Ala Thr Glu
    210                 215                 220

Tyr Ser Asp Asp Thr Lys Phe Leu Thr Leu Lys Thr Gly Phe Gln Thr
225                 230                 235                 240

Leu Pro Leu Thr Leu Asn Glu Arg Phe Lys Ala Val Pro Gly Gly Leu
                245                 250                 255

Ile Pro Ser Asp Gln Arg Val Gln Leu Asn Arg Arg Leu Ala Ser Leu
            260                 265                 270

Gln Tyr Ser Asp Asp Thr Glu Tyr Pro Tyr Arg Leu His Phe Gln Pro
        275                 280                 285
```

```
Thr Gln Thr Ile Asp Gly Thr Thr Thr Asp Ile Val Gly Ala Pro Glu
    290                 295                 300

Val Ile Val His Ala Arg Gln Val Ile Leu Ala Met Pro Arg Arg Ser
305                 310                 315                 320

Leu Glu Leu Ile Glu Ser Pro Leu Phe Gln Asp Pro Trp Leu Lys Glu
                325                 330                 335

Asn Leu Gly Ser Val Leu Val Gln Ser Ala Phe Lys Leu Phe Leu Ala
            340                 345                 350

Tyr Glu Gln Pro Trp Trp Arg Ser Leu Gly Leu Val Ala Gly Arg Ser
        355                 360                 365

Val Thr Asp Leu Pro Ile Arg Gln Cys Tyr Tyr Met Gly Thr Glu Cys
    370                 375                 380

Glu Gln Glu Gly Gly Gln Pro Ser Leu Asn Ser Leu Leu Met Ala Ser
385                 390                 395                 400

Tyr Asn Asp Ile Gly Thr Val Pro Phe Trp Lys Gly Leu Glu Gly Gly
                405                 410                 415

Leu Pro Phe Gln Gly Tyr Gln Pro Lys Ser Leu Leu Gly Glu Val Asp
            420                 425                 430

Ala Asn Glu Ile Val Pro Lys Thr Gln Asn Gln Val Ser Glu Glu Met
        435                 440                 445

Val Arg Ile Ala Gln Arg Gln Val Thr Ser Leu His Asp Gln Val Glu
    450                 455                 460

Leu Pro Ala Pro Tyr Ser Ala Val Tyr His Ala Trp Asp Ala Asp Pro
465                 470                 475                 480

Tyr Gly Gly Gly Trp His Glu Trp Lys Ala Asn Phe Arg Leu Asp Leu
                485                 490                 495

Ile Ile Gln Asn Met Arg His Pro Val Gln Glu Gln Ser Val Tyr Ile
            500                 505                 510

Val Gly Glu Ala Tyr Ser Tyr Gly Gln Gly Trp Val Glu Gly Ala Leu
        515                 520                 525

Thr Thr Ala Glu Ser Thr Leu Gln Asp Phe Phe Gly Leu Ala Arg Pro
    530                 535                 540
```

Ser Trp Leu Pro Ala Ala Tyr Gln Leu Leu Pro Glu Pro Gly Pro Thr
545 550 555 560

Asp Ile Ala Asp Pro Ala Pro Leu Ala Cys Lys Asp Cys Ala Gly Thr
565 570 575

Leu Glu Ala Val Thr Glu Phe Ala Tyr Thr Gly Ile Lys Pro
580 585 590

<210> 61
<211> 1770
<212> DNA
<213> Pseudomonas sp.WCHPs060044

<400> 61
atgtcacaaa ctcagccttt agacgttgcc ataataggtg gtggtgtgag cggtacctac 60

agcgcatggc gtctgcagga ggcccaggga gatcatcagc ggatacagct ttttgaatat 120

ggtgaccgta ttggcggtcg gttgtttagc attaccctgc ccggtctgcc gaacgttgtt 180

gcagaggtgg gtggtatgcg ttggatgcct gcaacagaag atgacacggg cggtcatgtg 240

atggttgata agttagttga ctatcttgag cttccgaaaa aggactttcc tatggggaac 300

gagcagcccg agggggaccc cgtgggtgca aagaataatc tgttttacct gcgtggccaa 360

cgtttccgct tccgtgattt caccgagtca cccgacaaaa ttccttataa cctggcctgg 420

agcgagcgtg gcttcggccc ggaagactta caggtgaagg tcatgaattc tatttacccg 480

ggttttgacc aactgagttt agcagatcaa atgcaagtga aggtttttgg caaagagatt 540

tggcgttatg gattctggga cttattatac cgcgttctgt caaacgaggg ctatcagttt 600

atgaaggatg caggtggtta cgaggcaaac gttgccaatg ccagcgctgt tacccagtta 660

ccggcaacgg agtacagcga cgataccaaa ttcctgactt taaaaactgg attccaaacc 720

cttccgctga ccctgaacga acgtttcaaa gcagtgccgg gtggtctgat tccgagtgat 780

caacgtgtcc aactgaaccg gcgcctggcg tcccttcagt actcggatga cacggaatac 840

ccgtaccgct tacatttcca gcctacccag acaattgatg gaactacaac agatatcgtg 900

ggagcaccgg aagtcattgt tcatgcacgg caggttatac tggcgatgcc gcggagatct 960

ttggaactga ttgagtctcc actgtttcaa gatccttggc tgaaagaaaa tctggggagt 1020

gttttagtgc agagtgcatt taaattattt cttgcctatg agcaaccctg gtggagatca 1080

ctggggttag tggctgggcg cagtgtgacc gatctgccta tacgccaatg ctattatatg 1140

ggtaccgagt gcgaacagga aggtggccag cctagtctga attcgctgct gatggcctcg 1200

tataatgata tcggcacagt cccttttttgg aaaggcctgg aagggggcct tccttttcaa 1260

gggtatcaac ccaaatccct gttaggcgaa gtagatgcca atgaaattgt accaaaaaca 1320

cagaatcaag tatctgaaga aatggtaaga attgcccagc gccaggtaac atctctgcat 1380

gatcaggtag aattgccggc tccgtattca gccgtatatc acgcttggga tgctgatccg 1440

tatggcggcg gctggcacga atggaaagcg aattttcgcc ttgatttgat catccagaat 1500

atgagacacc cggtccagga acagtctgta tatatcgtcg gcgaagccta ttcctatggg 1560

cagggatggg tcgaaggggc gcttacaact gctgaatcaa ctcttcagga tttttttgga 1620

ttggctcgcc catcctggtt gccagctgcg tatcagcttt tgccagaacc aggaccaacg 1680

gatatcgctg atccagcgcc attggcgtgt aaagattgtg cgggaacgtt ggaagcggtc 1740

acggaatttg cgtatacggg aatcaaacca 1770

<210> 62
<211> 30
<212> DNA
<213> Artificial Sequence

<220>
<223> Artificially Synthesized Primer Sequence E39X-Rv

<400> 62
tatggtgacc gtattggcgg tcggttgttt 30

<210> 63
<211> 30
<212> DNA
<213> Artificial Sequence

<220>
<223> Artificially Synthesized Primer Sequence E39Q-Fw

<400> 63
atacagcttt ttcagtatgg tgaccgtatt 30

<210> 64
<211> 30
<212> DNA
<213> Artificial Sequence

<220>
<223> Artificially Synthesized Primer Sequence D74X-Rv

<400> 64
cttatcaacc atcacatgac cgcccgtgtc 30

<210> 65
<211> 30
<212> DNA
<213> Artificial Sequence

<220>
<223> Artificially Synthesized Primer Sequence D74R-Fw

<400> 65

cctgcaacag aacgcgacac gggcggtcat 30

<210> 66
<211> 30
<212> DNA
<213> Artificial Sequence

<220>
<223> Artificially Synthesized Primer Sequence D74N-Fw

<400> 66
cctgcaacag aaaacgacac gggcggtcat 30

<210> 67
<211> 30
<212> DNA
<213> Artificial Sequence

<220>
<223> Artificially Synthesized Primer Sequence E224X-Rv

<400> 67
taaagtcagg aatttggtat cgtcgctgta 30

<210> 68
<211> 30
<212> DNA
<213> Artificial Sequence

<220>
<223> Artificially Synthesized Primer Sequence E224R-Fw

<400> 68
ttaccggcaa cgcgctacag cgacgatacc 30

<210> 69
<211> 30
<212> DNA
<213> Artificial Sequence

<220>
<223> Artificially Synthesized Primer Sequence E354X-Rv

<400> 69
caaccctggt ggagatcact ggggttagtg 30

<210> 70
<211> 30
<212> DNA
<213> Artificial Sequence

<220>
<223> Artificially Synthesized Primer Sequence E354Q-Fw

<400> 70
tttcttgcct atcagcaacc ctggtggaga 30

<210> 71
<211> 30
<212> DNA
<213> Artificial Sequence

<220>
<223> Artificially Synthesized Primer Sequence E383X-Rv

<400> 71
tgcgaacagg aaggtggcca gcctagtctg 30

<210> 72
<211> 30
<212> DNA
<213> Artificial Sequence

<220>
<223> Artificially Synthesized Primer Sequence E383Q-Fw

<400> 72
tatatgggta cccagtgcga acaggaaggt 30

<210> 73
<211> 30
<212> DNA
<213> Artificial Sequence

<220>
<223> Artificially Synthesized Primer Sequence D403X-Rv

<400> 73
caggcctttc caaaaaggga ctgtgccgat 30

<210> 74
<211> 30
<212> DNA
<213> Artificial Sequence

<220>
<223> Artificially Synthesized Primer Sequence D403R-Fw

<400> 74
gcctcgtata atcgcatcgg cacagtccct 30

<210> 75
<211> 30
<212> DNA
<213> Artificial Sequence

<220>
<223> Artificially Synthesized Primer Sequence D403N-Fw

<400> 75
gcctcgtata ataacatcgg cacagtccct 30

<210> 76
<211> 30
<212> DNA

<213> Artificial Sequence

<220>
<223> Artificially Synthesized Primer Sequence E487X-Rv

<400> 76
gatcaaatca aggcgaaaat tcgctttcca 30

<210> 77
<211> 30
<212> DNA
<213> Artificial Sequence

<220>
<223> Artificially Synthesized Primer Sequence E487H-Fw

<400> 77
ggcggctggc accattggaa agcgaatttt 30

<210> 78
<211> 30
<212> DNA
<213> Artificial Sequence

<220>
<223> Artificially Synthesized Primer Sequence E487Q-Fw

<400> 78
ggcggctggc accagtggaa agcgaatttt 30

<210> 79
<211> 30
<212> DNA
<213> Artificial Sequence

<220>
<223> Artificially Synthesized Primer Sequence E532X-Rv

<400> 79
tcaactcttc aggatttttt tggattggct 30

<210> 80
<211> 30
<212> DNA
<213> Artificial Sequence

<220>
<223> Artificially Synthesized Primer Sequence E532Q-Fw

<400> 80
cttacaactg ctcagtcaac tcttcaggat 30

<210> 81
<211> 586
<212> PRT
<213> Artificial Sequence

<220>

<223> AncARODn2 artificially generated from Pseudomonas sp. strain TPU 7192 (S. Nakano et. al., Appl. Environ. Microbiol. 2019 85(12) e00459-19)

<400> 81

Met Pro Thr Glu Leu Asp Ile Ala Ile Ile Gly Gly Gly Val Ser Gly
1               5                   10                  15

Val Tyr Ser Ala Trp Arg Leu Gln Gln His Arg Gly Asp Glu Gln Arg
            20                  25                  30

Ile Ala Leu Phe Glu Tyr Ser Asn Arg Ile Gly Gly Arg Leu Tyr Ser
        35                  40                  45

Arg Lys Leu Pro Gly Leu Pro Asn Val Val Ala Glu Leu Gly Gly Met
    50                  55                  60

Arg Tyr Ile Pro Glu Asp His Leu Met Val Asn Ser Leu Val Asn Glu
65                  70                  75                  80

Leu Lys Leu Pro Thr Lys Asp Phe Pro Met Gly Ser Ser Leu Pro Leu
                85                  90                  95

Asp Pro Lys Ser Lys Asp Pro Ser Pro Lys Asp Val Lys Ala Gly Ser
            100                 105                 110

Glu Asn Asn Leu Phe Tyr Leu Arg Gly Gln Tyr Phe Arg Tyr Arg Asp
        115                 120                 125

Phe Ala Glu Cys Pro Asp Arg Ile Pro Tyr Asn Leu Ser Trp Ser Glu
    130                 135                 140

Arg Gly Tyr Gly Pro Glu Asp Met Gln Val Lys Val Met Asn Leu Ile
145                 150                 155                 160

Cys Pro Gly Phe Ala Asp Met Ser Leu Cys Glu Gln Met Gln Val Lys
                165                 170                 175

Val Phe Gly Lys Glu Ile Trp Arg Tyr Gly Phe Trp Asn Leu Leu Glu
            180                 185                 190

Arg Val Leu Ser Asn Glu Ala Tyr Gln Phe Met Lys Asp Ala Gly Gly
        195                 200                 205

Tyr Glu Ala Asn Val Ala Asn Ala Asn Ala Val Thr Gln Leu Pro Ala
    210                 215                 220

Thr Glu Tyr Ser Asp Asp Thr Glu Phe Leu Thr Leu Lys Asp Gly Phe

225 230 235 240

Gln Ala Leu Pro Leu Thr Leu Cys Glu Gln Phe Glu Glu Leu Pro Gly
245 250 255

Ala Leu His Met Asn Gln Arg Leu Ala Glu Ile Arg Ile Asp Asp Asp
260 265 270

Ser Asp Tyr Arg Tyr Thr Leu Ile Phe Gln Pro Thr Ser Thr Asp Asp
275 280 285

Ser Gly Lys Thr Thr Asp Lys Asp Asp Ala Ala Val Thr Val Arg Ala
290 295 300

Lys Lys Val Ile Leu Ala Met Pro Arg Arg Ser Leu Glu Leu Ile Lys
305 310 315 320

Ser Pro Phe Phe Asp Asp Pro Trp Leu Lys Glu Asn Ile Pro Ser Val
325 330 335

Leu Ile Gln Lys Ala Phe Lys Met Phe Met Ala Tyr Glu Gln Pro Trp
340 345 350

Trp Arg Ser Leu Gly Leu Val Ala Gly Arg Ser Val Thr Asp Leu Pro
355 360 365

Ile Arg Gln Thr Tyr Tyr Met Gly Thr Glu Cys Asp Gln Ser Gly Gly
370 375 380

Glu Pro Thr Thr Asn Ser Leu Leu Met Ala Ser Tyr Asn Asp Ile Gly
385 390 395 400

Thr Val Pro Tyr Trp Lys Gly Leu Glu Ala Gly Glu Pro Phe Gln Gly
405 410 415

Tyr Thr Pro Ala Ser Leu Ser Glu Gly Cys Ala Glu Glu Gln Ile Val
420 425 430

Pro Lys His Gln Phe Gln Ile Thr Glu Asp Met Val Gln Ala Ala His
435 440 445

Arg Gln Val Glu Ala Leu His Asn Gln Lys Gln Leu Pro Gln Pro Tyr
450 455 460

Ser Ala Val Tyr Gln Glu Trp Gly Asp Asp Pro Tyr Gly Gly Gly Trp
465 470 475 480

His Glu Trp Lys Ala Asn Tyr Arg Leu Asp Glu Ile Met Cys Arg Met
485 490 495

Arg His Pro Val Glu Asp Glu Glu Ile Tyr Ile Val Gly Glu Ala Tyr
500 505 510

Ser Tyr Glu Gln Gly Trp Val Glu Gly Ala Leu Asn Thr Ala Glu Ser
515 520 525

Thr Leu Glu Glu Phe Phe Gly Leu Pro Thr Pro Ser Trp Leu Ser Lys
530 535 540

Asp His Asn Phe Leu Pro Val Ala Cys Asn Gly Thr Ser Ser Asp Asn
545 550 555 560

Ser Ala Thr Ser Ala Cys Asn Ala Ser Ser Glu Thr Leu Asn Glu Val
565 570 575

Thr Glu Phe Ala Tyr Glu Gly Ile Asn His
580 585

<210> 82
<211> 1758
<212> DNA
<213> Artificial Sequence

<220>
<223> AncARODn2 artificially generated from Pseudomonas sp. strain TPU 7192 (S. Nakano et. al., Appl. Environ. Microbiol. 2019 85(12) e00459-19)

<400> 82
atgcctacgg agttagacat cgctattatc ggtggggggg tttccggggt ttattccgca 60
tggcgtctgc agcaacatag aggtgacgag caacgtattg cgttatttga gtactcaaat 120
cgcataggtg gccgtctgta ctcacgtaag ctgccgggtc tgccgaacgt tgtggcagaa 180
ctgggtggta tgcggtatat ccccgaggac cacctgatgg ttaactcttt agtgaatgaa 240
ctgaaactgc ctaccaagga ttttccgatg ggctcaagct tgccgttgga ccctaaaagc 300
aaagatccga gcccgaaaga tgttaaagct ggcagcgaga ataatctgtt ttacctgcgt 360
ggtcagtatt ttcgttatcg ggactttgcg gagtgcccgg accgtatacc ttacaactta 420
agttggagtg agcgcggcta tggtcctgag gacatgcaag tgaaagtgat gaacttaatt 480
tgcccgggct tcgcagacat gtcattgtgc gagcaaatgc aggtgaaggt cttcggtaag 540
gaaatatggc gttacggttt ttggaacctg ttagagcggg ttttatcaaa tgaggcttac 600
cagtttatga aggacgcagg tggctacgaa gccaatgttg ctaacgccaa cgcggtaacc 660
caactgcctg caactgaata cagcgatgat accgaatttt taactctgaa agatggcttc 720

caggcattac cgttaaccct gtgtgagcaa ttcgaggagc ttccaggtgc actgcacatg 780

aatcagcgtc tggctgagat ccgtattgat gatgattctg attatcggta tacactgatt 840

ttccaaccca cttcaactga tgatagcggt aagaccaccg acaaagacga tgcagctgta 900

accgttcgcg caaagaaagt gatactggcg atgcctcgcc gcagtctgga actgataaaa 960

agccctttttt ttgacgatcc ttggctgaaa gagaacattc ccagcgtgct gattcagaaa 1020

gcatttaaaa tgttcatggc atacgaacaa ccctggtgga gaagcctggg gctggtagcc 1080

gggcgctcag tcacagattt gcccatccgc caaacctact atatgggtac cgagtgtgat 1140

cagtctggtg gagaaccgac aactaacagt ttattgatgg cgtcctataa tgatatcgga 1200

acagtaccat attggaaagg cttggaagca ggcgaaccgt ttcaggggta tacaccagcc 1260

tctttgagtg aaggctgtgc ggaagaacag attgttccga aacatcaatt ccagattacg 1320

gaagatatgg tgcaggctgc tcatcgccag gttgaagctt tgcacaatca gaaacagctt 1380

ccgcagccat atagtgcggt gtatcaggaa tggggcgatg atccctatgg cggaggctgg 1440

catgaatgga aagcgaacta tagacttgat gaaattatgt gccggatgag acacccagtc 1500

gaagatgaag aaatctatat tgtcggagaa gcgtattctt atgaacaggg atgggtcgaa 1560

ggagccctta acacagcgga aagtactctt gaagaatttt tcggacttcc aacaccatcc 1620

tggctttcta aagatcataa ttttcttcca gtcgcctgta atggaacgtc ttccgataat 1680

tcggccacgt cggcctgtaa tgcctcgtcg gaaacgctta atgaagtaac ggaatttgcc 1740

tatgaaggga ttaatcat 1758